# EUROPEAN PATENT SPECIFICATION

(11) **EP 1 502 102 B1**
(45) Date of publication and mention of the grant of the patent: **14.01.2009**
(21) Application number: 03721355.0
(22) Date of filing: 11.03.2003
(51) Int. Cl.: G01N 33/543, G01N 33/68, C12Q 1/68

(54) **COMPOUNDS AND METHODS FOR ANALYZING THE PROTEOME**
VERBINDUNGEN UND VERFAHREN FÜR DIE ANALYSE DES PROTEOMS
COMPOSES ET PROCEDES POUR ANALYSER LE PROTEOME

(30) Priority: 11.03.2002 US 363433 P
(43) Date of publication of application: 02.02.2005
(73) Proprietor: caprotec bioanalytics GmbH, 12489 Berlin (DE)
(72) Inventor: KÖSTER, Hubert, 22299 Hamburg (DE); SHCHEPINOV, Mikhail, S., Cambridge, MA 02139 (US); LITTLE, Daniel, P., Groton, MA 01450 (US)
(74) Representative: Baldock, Sharon Claire
(86) International application number: PCT/US2003/007479
(87) International publication number: WO 2003/077851

(56) References cited:
- WO-A-01/77668
- WO-A-97/33169
- WO-A-97/42507
- WO-A-98/59360
- WO-A-99/05320
- WO-A-02/063301
- WO-A-03/050544
- WO-A1-97/40385
- US-A- 5 922 617
- US-B1- 6 329 209
- US-B1- 6 391 649
- GYGI S.P. ET AL: 'Quantitative analysis of complex protein mixtures using isotope-coded affinity tags' NATURE BIOTECHNOLOGY vol. 17, October 1999, pages 994 - 999, XP002947934
- CHATTOPADHYAYA, J.B., REESE, C,B.: J.C.S.CHEM.COMM, 1978, pages 639-640,
- FROMAGEOT, H.P.M ET AL.: TETRAHEDRON, vol. 23, 1967, pages 2315-2331,

## Description

### FIELD

Provided herein are compounds and methods using the compounds to specifically and selectively analyze biomolecules. In particular, the compounds and methods are useful for analyzing the proteome.

### BACKGROUND

The Human Genome effort has generated a raw sequence of the 3 billion base pairs of the human genome and revealed about 35,000 genes. Genetic variations amongst different individuals and in between populations are being studied in order to determine the association with the predisposition to disease or the correlation to drug efficacy and/or side effects.

A frequent manifestation of genetic variations are single nucleotide polymorphisms (SNPs). Technologies have been developed to analyze SNPs in an industrial scale (*e.g.*, MassARRAY^{™} and the MassARRAY® system, Sequenom, Inc., San Diego, CA) and in pooled samples to study the frequency of SNPs in populations of various gender, ethnicity, age and health condition. The ultimate goal of these efforts is to understand the etiology of disease on the molecular level (*e.g.*, based on genetic variances (pharmacogenomics)), in order to develop diagnostic assays hand-in-hand with new and more effective and no-side-effect drugs.

Knowledge of the association of an SNP (or SNPs) with a given disease or drug side-effect, however, is not sufficient. Further, it is not sufficient to establish the differential expression profiles of messenger RNAs in comparing a healthy and disease tissue sample as is performed today using expression DNA chips (*e.g.*, GeneChip^{™} technology, Affymetrix, Inc., Santa Clara, CA; LifeArray" technology, Incyte Genomics, Inc., Palo Alto, CA). This is because the metabolic activities in a cell are not being carried out by mRNAs but rather by proteins which are translated from them and subsequently posttranslationally modified (alkylated, glycosylated, phosphorylated, etc.).

The study of proteomics encompasses both the study of individual proteins and how these proteins function within a biochemical pathway. Proteomics also include the study of protein interactions with regard to how they form the architecture which make up living cells. In many human diseases such as cancer, Alzheimer's disease, diabetes as well as host responses to infectious diseases, the elucidation of the complex interactions regulatory proteins, which can cause diseases, is a critical step to finding effective treatment. Often, SNPs and other nucleic acid mutations occur in genes whose products are such proteins as (1) growth related hormones, (2) membrane receptors for growth hormones, (3) components of the trans-membrane signal pathway, (4) DNA binding proteins which act on transcription and the inactivation of suppressor genes (*e.g.* P53) causing the onset of disease. WO9859360 describes arrays for proteomics in which each row displays a different property.

The only technology that is currently being used to analyze complex protein mixtures is 2D gel electrophoresis and subsequent image processing to identify changes in the pattern (structural changes) or intensity of various protein spots. 2D gel electrophoresis is a laborious, error-prone method with low reproducibility and suffers from an inability to be automated. Further, the resolution of 2D gels is insufficient to display all the proteins present in a mixture. Therefore, the analysis of the proteome needs technologies which allow scaling up to industrial levels with the features of an industrial process: high accuracy, reproducibility and flexibility in which the process is of high-throughput, automatable and cost-effective. !t is an object herein to provide such technologies.

### SUMMARY

Gradient arrays for the analysis of biomolecules are provided. In particular, arrays and methods are provided for analyzing complex protein mixtures, such as the proteome. The arrays provide bifunctional reagents that allow for the separation and isolation of complex protein mixtures. Automated instruments for performing the methods are also provided.

Provided herein are methods, arrays of capture compounds (also referred to herein as capture agents) for analysis of the proteome on an industrial level in a high throughput format. The methods and arrays permit sorting of complex mixtures of biomolecules. In addition, they permit identification of protein structures predicative or indicative of specific of phenotypes, such as disease states, thereby eliminating the need for random SNP analysis, expression profiling and protein analytical methods. The arrays and methods sort complex mixtures by providing a variety of different capture agents. In addition, they can be used to identify structural "epitopes" that serve as markers for specific disease states, stratify individual populations relative to specific phenotypes, permit a detailed understanding of the proteins underlying molecular function, and provide targets for drug development. The increased understanding of target proteins permit the design of higher efficiency therapeutics.

Arrays of capture compounds and methods that use the compounds, singly or in collections thereof, to capture, separate and analyze biomolecules, including, but not limited to, mixtures of biomolecules, including biopolymers and macromolecules, such as proteins, individual biomolecules, such as proteins, including individual or membrane proteins, are provided. The arrays contain a plurality, typically at least 10, 50, 100, 1000 or more different capture compounds, where different compounds are located at each locus.

In particular, the arrays are gradient arrays, which are generally a 2-dimensional addressable arrays of capture compounds. In these embodiments the capture compounds, which are defined herein, are those that contain or moieties X and Y linked to a solid support. Hence the arrays are a 2-dimensional array of moieties X and Y on a surface that presents moieties X and Y. The surface is a solid support; the X and Y moieties are arranged in a two dimensional array of continuous gradients of one or more properties of X and Y; the properties of moiety X are in a gradient along the X-axis; the properties of moiety Y are in a gradient along the Y-axis; the X moieties are each independently selected to bind to biomolecules covalently or with sufficiently high affinity so that the resulting complexes of biomolecule/capture compounds are stable under conditions of mass spectrometric analysis; and the Y moieties are each independently selected to increase the selectivity of the binding by moiety X.

Each X moiety in each row (X-axis) of loci can differ in a gradual manner in hydrophilicity, lipophilicity, charge, size, reagent specificity. Each Y moiety in each column (Y-axis) of loci can differ in a gradual manner, in hydrophilicity, lipophilicity, charge, size, reagent specificity.

In some embodiments, the Y moieties can be present at each locus with the X moieties or can be bound to each X moiety.

For example, the X or Y moiety can be an azobenzene group and a gradient of hydrophilicity can be created by increasing (or decreasing) light exposure at each locus in the array. The X moiety can be a charged group and a gradient of charge can created by exposure to an increase in current or voltage. Other properties include increased or decreased specificity of the X or Y groups for NH₂, SH, SS or OH groups.

The resulting arrays present a surface with a plurality of loci (10, 50, 100, 500, 1000, 2000, 3000, 4000, 5000, 10,000 and more) that differ in a particular properties or pairs of properties or a plurality of properties in defined increments. Such a surface permits capture of molecules and biological particules with differing affinities for the X moieties at discrete loci. These gradient arrays can be used in methods for sorting complex mixtures or probing cell and organelle surfaces and in other methods described herein or, for example, in copending U.S. application Serial No. 10/197,954 or International PCT application No. PCT/US02/22821.

Thus the arrays are designed to permit probing of a mixture of biomolecules by virtue of interaction of the capture compounds in the collection with the components of the a mixture under conditions that preserve their three-dimensional configuration. Each locus in the array is designed 1) to bind, either covalently or other chemical interaction with high binding affinity (kₐ) such that the binding is irreversible or stable under conditions of mass spectrometric analysis) to fewer than all, typically about 5 to 20 or more component biomolecules in a mixture, depending upon complexity and diversity of the mixture, under physiological conditions, including hydrophobic conditions, and 2) distinguish among biomolecules based upon topological features.

The arrays are used in a variety of methods, but are particularly designed for assessing biomolecules, such as biopolymer, components in mixtures from biological samples. The arrays are used in top-down unbiased methods that assess structural changes, including post-translational structural changes and, for example, are used to compare patterns, particularly post-translational protein patterns, in diseased versus healthy cells from primarly cells generally from the same individual. The cells that serve as the sources of biomolecules can be frozen into a selected metabolic state or synchronized to permit direct comparison and identification of phenotype-specific, such as disease-specific biomolecules, generally proteins.

A locus in the array includes at a chemical reactivity group X (also referrred to herein as a function or a functionality), which effects the covalent or a high binding affinity (high kₐ) binding, and least one of three other groups (also referred to herein as functions or funtionalities). The loci also includes a selectivity function Y that modulates the interaction of a biomolecule with the reactivity function.

For example, the reactivity group (reactivity function) includes groups that specifically react or interact with functionalities on the surface of a protein such as hydroxyl, amine, amide, sulfide and carboxylic acid groups, or that recognize specific surface areas, such as an antibody, a lectin or a receptor-specific ligand, or interacts with the active site of enzymes. Those skilled in the art can select from a library of functionalities to accomplish this interaction. While this interaction can be highly reaction-specific, these compounds can react multiple times within the same protein molecule depending on the number of surface-accessible functional groups. Modification of the reaction conditions allows the identification of surface accessible functional groups with differing reactivity, thereby permitting identification of one or more highly reactive sites used to separate an individual protein from a mixture. Available technologies do not separate species in the resulting reaction mixture. The collections and compounds provided herein solve that prdoblem through a second functionality, the selectivity group, which alters binding the reactivity groups to the biomolecule.

Selectivity functions include a variety of groups, as well as the geometric spacing of the second functionality, a single stranded unprotected or suitably protected oligonucleotide or oligonucleotide analog. The selective functionality can be separate from the compound and include the solid or semi-solid support. The selective functionality in this embodiment can be porosity, hydrophobicity, charge and other chemical properties of the material. For example, selectivity functions interact noncovalently with target proteins to alter the specificity or binding of the reactivity function. Such functions include chemical groups and biomolecules that can sterically hinder proteins of specific size, hydrophilic compounds or proteins (*e.g.*, PEG and trityls), hydrophobic compounds or proteins (*e.g.*, polar aromatic, lipids, glycolipids, phosphotriester, oligosaccharides), positive or negatively charged groups, groups or biomolecules which create defined secondary or tertiary structure.

In practice in one embodiment, a gradient array is contacted with a biomolecule mixture and the bound molecules are assessed using, for example, mass spectrometry, followed by optional application of tagging, such as fluorescence tagging, after arraying to identify low abundance proteins.

Also provided herein are methods for the discovery and identification of proteins, which are selected based on a defined phenotype. The methods allow proteins to bind to the target molecules under physiological conditions while maintaining the correct secondary and tertiary conformation of the target. The methods can be performed under physiological and other conditions that permit discovery of bioglogically important proteins, including membrane proteins, that are selected based upon a defined phenotype.

The gradient arrays can be composed of moieties selected to capture target proteins or groups related proteins that can mimic biological structures such as nuclear and mitochondrial transmembrane structures, artificial membranes or intact cell walls.

Samples for analysis include any biomolecules, particularly protein-containing samples, such as protein mixtures, including, but not limited to, natural and synthetic sources. Proteins can be prepared by translation from isolated chromosomes, genes, cDNA and genomic libraries. Proteins can be isolated from cells, and other sources. In certain embodiments, the capture compounds provided herein are designed to selectively capture different post-translational modifications of the same protein (*i.e.*, phosphorylation patterns (*e.g.*, oncogenes), glycosylation and other post-translational modifications).

Other methods that employ the collections are also provided. In one method, the arrays are used to distinguish between or among different conformations of a protein and, for example, can be used for phenotypic identification, such as for diagnosis.. For example, diseases of protein aggregation, which are disease involving a conformationally altered protein, such as amyloid diseases, the collections can distingush between the disease-involved form of the protein from the normal protein and thereby diagnose the disease in a sample.

### BRIEF DESCRIPTION OF THE FIGURES

Figure 1 shows the hybridization, separation and mass spectral analysis of a mixture of proteins.
Figure 2 provides a schematic depiction of one embodiment of the apparatus provided herein.

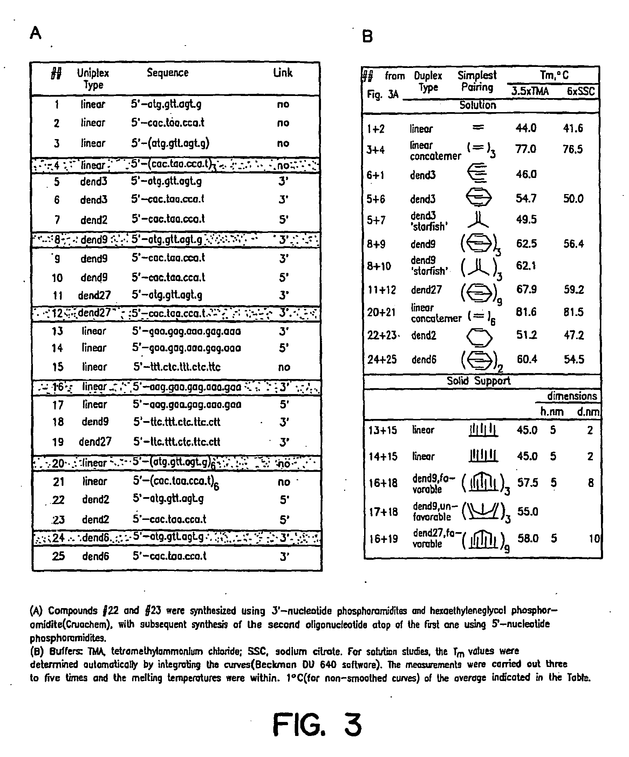

Figure 3 shows exemplary, non-limiting examples of dendrimeric structures upon which the compounds provided herein are based.
Figure 4 illustrates masking synthesis of a chip possessing patches of varying hydrophobicity/hydrophilicity.
Figure 5 shows an exemplary chip possessing a continuous two-dimensional gradient of charge (Y-axis) and hydrophilicity/hydrophobicity (X-axis).
Figure 6 illustrates a light-tunable hydrophobic/hydrophilic diazo switch.
Figure 7 shows a continuous two-dimensional substrate having a gradient of electric charges in one dimension and a gradient of hydrophobic/hydrophilic groups tunable by a light gradient in the other dimension.
Figure 8 illustrates a protein tagged with four compounds provided herein, thereby allowing for specific sorting of the protein.
Figure 9 shows the increased and specific hybridization resulting from use of two or more oligonucleotide tags.
Figure 10 illustrates hybridizations possible with dedrimeric oligonucleotide-tagged proteins.
Figure 11 shows tagging of a single protein with two different oligonucleotides in one reaction.

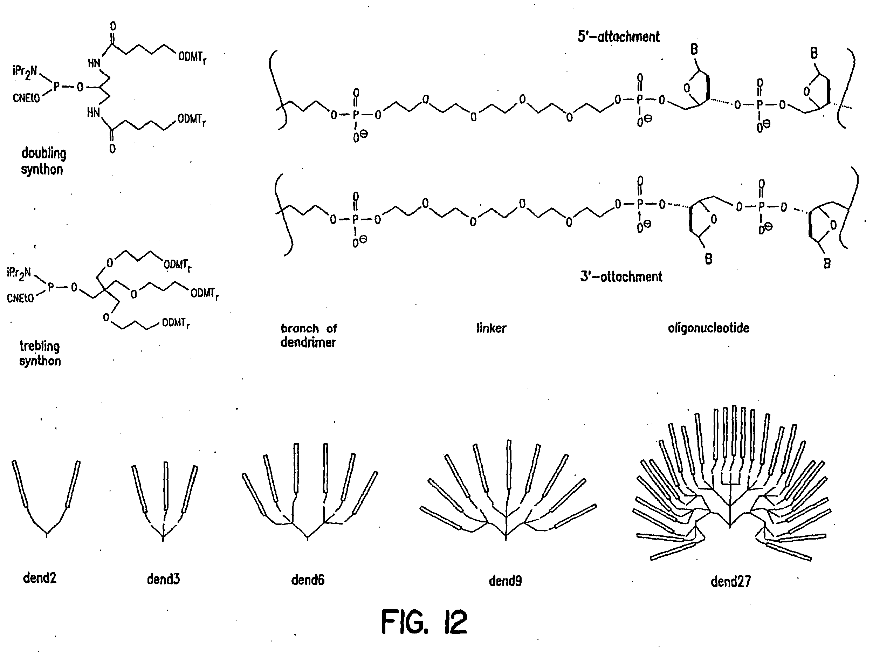

Figure 12 shows various dedrimeric structures for Z.
Figure 13 is a flow diagram of recombinant protein production.
Figure 14 illustrates production of an adapted oligonucleotide dT primed cDNA library.
Figure 15 shows production of an adapted sequence motif specific cDNA library.
Figure 16 shows production of an adapted gene specific cDNA.
Figure 17 illustrates purification of amplification products from a template library.
Figure 18 shows an adapted oligonucleotide dT primed cDNA libraray as a universal template for the amplification of gene subpopulations.
Figure 19 illustrates decrease of complexity during PCR amplification.
Figure 20 shows the attachment of a bifunctional molecule to a solid surface.
Figure 21 shows analysis of purified proteins from compound screening and antibody production.

### DETAILED DESCRIPTION OF THE EMBODIMENTS

### A. Definitions

Unless defined otherwise, all technical and scientific terms used herein have the same meaning as is commonly understood by one of skill in the art to which the invention(s) belong. In the event that there are a plurality of definitions for terms herein, those in this section prevail. Where reference is made to a URL or other such indentifier or address, it understood that such identifiers can change and particular information on the internet can come and go, but equivalent information can be found by searching the internet. Reference thereto evidences the availability and public dissemination of such information.

As used herein, an oligonucleotide means a linear sequence of up to about 20, about 50, or about 100, nucleotides joined by phosphodiester bonds. Above this length the term polynucleotide begins to be used.

As used herein, an oligonucleotide analog means a linear sequence of up to about 20, about 50, or about 100, nucleotide analogs, or linear sequence of up to about 20, about 50, or about 100 nucleotides linked by a "backbone" bond other than a phosphodiester bond, for example, a phosphotriester bond, a phosphoramidate bond, a phophorothioate bond, a methylphosphonate diester bond, a thioester bond, or a peptide bond (peptide nucleic acid).

As used herein, peptide nucleic acid (PNA) refers to nucleic acid analogs in that the ribose-phosphate backbone is replaced by a backbone held together by amide bonds.

As used herein, proteome means all the proteins present within a cell.

As used herein, a biomolecule is any compound found in nature, or derivatives thereof. Biomolecules include, but are not limited to oligonucleotides, oligonucleosides, proteins, peptides, amino acids, lipids, steroids, peptide nucleic acids (PNAs), oligosaccharides and monosaccharides.

As used herein, MALDI-TOF refers to matrix assisted laser desorption ionization-time of flight mass spectrometry.

As used herein, the term "conditioned" or "conditioning," when used in reference to a protein thereof, means that the polypeptide is modified to decrease the laser energy required to volatilize the protein, to minimize the likelihood of fragmentation of the protein, or to increase the resolution of a mass spectrum of the protein or of the component amino acids. Resolution of a mass spectrum of a protein can be increased by conditioning the protein prior to performing mass spectrometry. Conditioning can be performed at any stage prior to mass spectrometry and can be performed while the protein is immobilized. A protein can bye conditioned, for example, by treating it with a cation exchange material or an anion exchange material, which can reduce the charge heterogeneity of the protein, thereby for eliminating peak broadening due to heterogeneity in the number of cations (or anions) bound to the various proteins a population. In one embodiment, removal of all cations by ion exchange, except for H⁺ and ammonium ions, is performed. Contacting a polypeptide with an alkylating agent such as alkyliodide, iodoacetamide, iodoethanol, or 2,3-epoxy-1-propanol, the formation of disulfide bonds, for example, in a proteins can be prevented. Likewise, charged amino acid side chains can be converted to uncharged derivatives employing trialkylsilyl chlorides.

Since the capture compounds contain protein and nucleic acid portions, conditioning suitable for one or both portions is also contemplated. Hence, a prepurification to enrich the biomolecules to be analyzed and the removal of all cations, such as by ion exchange, except for H + and ammonium, or other conditioning treatment to improve resolution is advantageous for analysis of the nucleic acid portion as well as the protein portion.

Conditioning of proteins is generally unnecessary because proteins are relatively stable under acidic, high energy conditions so that proteins do not require conditioning for mass spectrometric analyses. There are means of improving resolution, however, in one embodiment for shorter peptides, such as by incorporating modified amino acids that are more basic than the corresponding unmodified residues. Such modification in general increases the stability of the polypeptide during mass spectrometric analysis. Also, cation exchange chromatography, as well as general washing and purification procedures that remove proteins and other reaction mixture components away from the protein can be used to increase the resolution of the spectrum resulting from mass spectrometric analysis of the protein.

As used herein, "matrix" refers to the material with which the capture compound biomolecule conjugates are combined for MALDI mass spectrometric analysis. Any matrix material, such as solid acids, including 3-hydroxypicolinic acid, liquid matrices, such as glycerol, known to those of skill in the art for nucleic acid and/or protein analyses is contemplated. Since the compound biomolecule conjugates contain nucleic acid and protein a mixture (optimal for nucleic acids and proteins) of matrix molecules can be used.

As used herein, macromolecule refers to any molecule having a molecular weight from the hundreds up to the millions. Macromolecules include, but are not limited to, peptides, proteins, nucleotides, nucleic acids, carbohydrates, and other such molecules that are generally synthesized by biological organisms, but can be prepared synthetically or using recombinant molecular biology methods.

As used herein, the term "biopolymer" is refers to a biological molecule, including macromolecules, composed of two or more monomeric subunits, or derivatives thereof, which are linked by a bond or a macromolecule. A biopolymer can be, for example, a polynucleotide, a polypeptide, a carbohydrate, or a lipid, or derivatives or combinations thereof, for example, a nucleic acid molecule containing a peptide nucleic acid portion or a glycoprotein. The methods and collections herein, though described with reference to biopolymers, can be adapted for use with other synthetic schemes and assays, such as organic syntheses of pharmaceuticals, or inorganics and any other reaction or assay performed on a solid support or in a well in nanoliter or smaller volumes.

As used herein, biomolecule includes biopolymers and macromolecules and all molecules that can be isolated from living organisms and viruses, including, but are not limited to, cells, tissues, prions, animals, plants, viruses, bacteria and other organsims.

As used herein, a biological particle refers to a virus, such as a viral vector or viral capsid with or without packaged nucleic acid, phage, including a phage vector or phage capsid, with or without encapsulated nucleotide acid, a single cell, including eukaryotic and prokaryotic cells or fragments thereof, a liposome or micellar agent or other packaging particle, and other such biological materials. For purposes herein, biological particles include molecules that are not typically considered macromolecules because they are not generally synthesized, but are derived from cells and viruses.

As used herein, a drug refers to any compound that is a candidate for use as a therapeutic or as lead compound for designing a therapeutic or that is a known pharmaceutical. Such compounds can be small molecules, including small organic molecules, peptides, peptide mimetics, antisense molecules, antibodies, fragments of antibodies, recombinant antibodies. Of particular interest are "drugs" that have specific binding properties so that they can be used as selectivity groups or can be used as for sorting of the capture compounds, either a sorting functionality that binds to a target on a support, or linked to a solid support, where the sorting functionality is the drug target.

As used herein, the term "nucleic acid" refers to single-stranded and/or double-stranded polynucleotides such as deoxyribonucleic acid (DNA), and ribonucleic acid (RNA) as well as analogs or derivatives of either RNA or DNA. A nucleic acid molecules are linear polymers of nucleotides, linked by 3',5' phosphodiester linkages. In DNA, deoxyribonucleic acid, the sugar group is deoxyribose and the bases of the nucleotides are adenine, guanine, thymine and cytosine. RNA, ribonucleic acid, has ribose as the sugar and uracil replaces thymine. Also included in the term "nucleic acid" are analogs of nucleic acids such as peptide nucleic acid (PNA), phosphorothioate DNA, and other such analogs and derivatives or combinations thereof.

As used herein, the term "polynucleotide" refers to an oligomer or polymer containing at least two linked nucleotides or nucleotide derivatives, including a deoxyribonucleic acid (DNA), a ribonucleic acid (RNA), and a DNA or RNA derivative containing, for example, a nucleotide analog or a "backbone" bond other than a phosphodiester bond, for example, a phosphotriester bond, a phosphoramidate bond, a methylphosphonate diester bond, a phophorothioate bond, a thioester bond, or a peptide bond (peptide nucleic acid). The term "oligonucleotide" also is used herein essentially synonymously with "polynucleotide," although those in the art recognize that oligonucleotides, for example, PCR primers, generally are less than about fifty to one hundred nucleotides in length.

Nucleotide analogs contained in a polynucleotide can be, for example, mass modified nucleotides, which allows for mass differentiation of polynucleotides; nucleotides containing a detectable label such as a fluorescent, radioactive, colorometric, luminescent or chemiluminescent label, which allows for detection of a polynucleotide; or nucleotides containing a reactive group such as biotin or a thiol group, which facilitates immobilization of a polynucleotide to a solid support. A polynucleotide also can contain one or more backbone bonds that are selectively cleavable, for example, chemically, enzymatically or photolytically. For example, a polynucleotide can include one or more deoxyribonucleotides, followed by one or more ribonucleotides, which can be followed by one or more deoxyribonucleotides, such a sequence being cleavable at the ribonucleotide sequence by base hydrolysis. A polynucleotide also can contain one or more bonds that are relatively resistant to cleavage, for example, a chimeric oligonucleotide primer, which can include nucleotides linked by peptide nucleic acid bonds and at least one nucleotide at the 3' end, which is linked by a phosphodiester bond, or the like, and is capable of being extended by a polymerase. Peptide nucleic acid sequences can be prepared using well known methods (see, for example, Weiler et al. (1997) Nuclei acids Res. 25:2792-2799) .

A polynucleotide can be a portion of a larger nucleic acid molecule, for example, a portion of a gene, which can contain a polymorphic region, or a portion of an extragenic region of a chromosome, for example, a portion of a region of nucleotide repeats such as a short tandem repeat (STR) locus, a variable number of tandem repeats (VNTR) locus, a microsatellite locus or a minisatellite locus. A polynucleotide also can be single stranded or double stranded, including, for example, a DNA-RNA hybrid, or can be triple stranded or four stranded. Where the polynucleotide is double stranded DNA, it can be in an A, B, L or Z configuration, and a single polynucleotide can contain combinations of such configurations.

As used herein, a "mass modification" with respect to a biomolecule to be analyzed for mass spectrometry, refers to the inclusion of changes in consituent atoms or groups that change the molecule weight of the resulting molecule in defined increments detectable by mass spectrometic analysis. Mass modifications do not radiolabels, such as isotope labels or or fluroescent gropus or other such tags normally used for detection by means other than mass spectrometry.

As used herein, the term "polypeptide," means at least two amino acids, or amino acid derivatives, including mass modified amino acids and amino acid analogs, which are linked by a peptide bond and which can be a modified peptide bond. A polypeptide can be translated from a polynucleotide, which can include at least a portion of a coding sequence, or a portion of a nucleotide sequence that is not naturally translated due, for example, to it being located in a reading frame other than a coding frame, or it being an intron sequence, a 3' or 5' untranslated sequence, a regulatory sequence such as a promoter. A polypeptide also can be chemically synthesized and can be modified by chemical or enzymatic methods following translation or chemical synthesis. The terms "polypeptide," "peptide" and "protein" are used essentially synonymously herein, although the skilled artisan recognizes that peptides generally contain fewer than about fifty to one hundred amino acid residues, and that proteins often are obtained from a natural source and can contain, for example, post-translational modifications. A polypeptide can be post-translationally modified by, for example, phosphorylation (phosphoproteins), glycosylation (glycoproteins, proteoglycans), which can be performed in a cell or in a reaction *in vitro.*

As used herein, the term "conjugated" refers stable attachment, typically by virtue of a chemical interaction, including ionic and/or covalent attachment. Among the conjugation means are streptavidin- or avidin- to biotin interaction; hydrophobic interaction; magnetic interaction (*e.g.*, using functionalized magnetic beads, such as DYNABEADS, which are streptavidin-coated magnetic beads sold by Dynal, Inc. Great Neck, NY and Oslo Norway); polar interactions, such as "wetting" associations between two polar surfaces or between oligo/polyethylene glycol; formation of a covalent bond, such as an amide bond, disulfide bond, thioether bond, or via crosslinking agents; and via an acid-labile or photocleavable linker.

As used herein, "sample" refers to a composition containing a material to be detected. For purposes sample refers to anything which can contain an biomolecule. The sample can be a biological sample, such as a biological fluid or a biological tissue obtained from any organism or a cell of or from an organism or a viral particle or portions thereof. Examples of biological fluids include urine, blood, plasma, serum, saliva, semen, stool, sputum, cerebral spinal fluid, tears, mucus, sperm, amniotic fluid or the like. Biological tissues are aggregate of cells, usually of a particular kind together with their intercellular substance that form one of the structural materials of a human, animal, plant, bacterial, fungal or viral structure, including connective, epithelium, muscle and nerve tissues. Examples of biological tissues also include organs, tumors, lymph nodes, arteries and individual cell(s).

Thus, samples include biological samples (*e.g.*, any material obtained from a source originating from a living being (*e.g.*, human, animal, plant, bacteria, fungi, protist, virus). The biological sample can be in any form, including solid materials (*e.g.*, tissue, cell pellets and biopsies, tissues from cadavers) and biological fluids (*e.g.*, urine, blood, saliva, amniotic fluid and mouth wash (containing buccal cells)). In certain embodiments, solid materials are mixed with a fluid. In embodiments herein, the a sample for mass spectrometric analysis includes samples that contain a mixture of matrix used for mass spectrometric analyses and the capture compound/biomolecule complexss.

As used herein, the term "solid support" means a non-gaseous, non-liquid material having a surface. Thus, a solid support can be a flat surface constructed, for example, of glass, silicon, metal, plastic or a composite; or can be in the form of a bead such as a silica gel, a controlled pore glass, a magnetic or cellulose bead; or can be a pin, including an array of pins suitable for combinatorial synthesis or analysis.

As used herein, a collection refers to combination of generally 10, 50, 100, 500, 1000 or more members. In particular a collection refers to such combination of the capture compounds as provided herein.

As used herein, an array refers to a collection of elements, such as the capture compounds. An addressable array is one in that the members of the array are identifiable, typically by position on a solid phase support but also by virtue of an identifier or detectable label. Hence, in general the members of an array are be immobilized to discrete identifiable loci on the surface of a solid phase. A plurality of of the compounds are attached to a support, such as an array on the surface of a support, such as a silicon chip or other surface, generally through binding of the sorting functionality with a group or compound on the surface of the support. Addressing can be achieved by labeling each each member electronically, such as with an radio-frequency (RF) tag, through the use of color coded beads or other such identifiable and color coded labels and through molecular weight. Hence, in general the members of the array are immobilized to discrete identifiable loci on the surface of a solid phase or directly or indirectly linked to or otherwise associated with the identifiable label, such as affixed to a microsphere or other particulate support (herein referred to as beads) and suspended in solution or spread out on a surface.

As used herein, a gradient array refers to an array of moieties, such as X and Y, such that properties of members of the array, gradually change along each axis. X is a reactivity group or function as defined herein and Y is a selectivity function or group as defined herein. Selected properties of each X and Y are modified in a predetermined manner.

As used herein, "substrate" refers to an insoluble support onto that a sample and/or matrix is deposited. Support can be fabricated from virtually any insoluble or solid material. For example, silica gel, glass (*e.g.*, controlled-pore glass (CPG)), nylon, Wang resin, Merrifield resin, dextran cross-linked with epichlorohydrin (*e.g.*, Sephadex^{R}), agarose (e.g., Sepharose^{R}), cellulose, magnetic beads, Dynabeads, a metal surface (*e.g.*, steel, gold, silver, aluminum, silicon and copper), a plastic material (*e.g.*, polyethylene, polypropylene, polyamide, polyester, polyvinylidenedifluoride (PVDF)) Exemplary substrate include, but are not limited to, beads (*e.g.*, silica gel, controlled pore glass, magnetic, dextran cross-linked with epichlorohydrin (e.g., Sephadex^{R}), agarose (e.g., Sepharose^{R}), cellulose), capillaries, flat supports such as glass fiber filters, glass surfaces, metal surfaces (steel, gold, silver, aluminum, copper and silicon), plastic materials including multiwell plates or membranes (*e.g.*, of polyethylene, polypropylene, polyamide, polyvinylidenedifluoride), pins (*e.g.*, arrays of pins suitable for combinatorial synthesis or analysis or beads in pits of flat surfaces such as wafers (*e.g.*, silicon wafers) with or without filter plates. The solid support is in any desired form, including, but not limited to, a bead, capillary, plate, membrane, wafer, comb, pin, a wafer with pits, an array of pits or nanoliter wells and other geometries and forms known to those of skill in the art. Supports include flat surfaces designed to receive or link samples at discrete loci. In one embodiment, flat surfaces include those with hydrophobic regions surrounding hydrophilic loci for receiving, containing or binding a sample.

The supports can be particulate or can be in the form of a continuous surface, such as a microtiter dish or well, a glass slide, a silicon chip, a nitrocellulose sheet, nylon mesh, or other such materials. When particulate, typically the particles have at least one dimension in the 5-10 mm range or smaller. Such particles, referred collectively herein as "beads", are often, but not necessarily, spherical. Reference to "bead," however, does not constrain the geometry of the matrix, which can be any shape, including random shapes, needles, fibers, and elongated. "Beads", particularly microspheres that are sufficiently small to be used in the liquid phase, are also contemplated. The "beads" can include additional components, such as magnetic or paramagnetic particles (see, *e.g.*" Dyna beads (Dynal, Oslo, Norway)) for separation using magnets, as long as the additional components do not interfere with the methods and analyses herein.

As used herein, "polymorphism" refers to the coexistence of more than one form of a gene or portion thereof. A portion of a gene of that there are at least two different forms, *e.g.*, two different nucleotide sequences, is referred to as a "polymorphic region of a gene". A polymorphic region can be a single nucleotide, *e.g.*, a single nucleotide polymorphism (SNP), the identity of that differs in different alleles. A polymorphic region also can be several nucleotides in length.

As used herein, "polymorphic gene" refers to a gene having at least one polymorphic region.

As used herein, "allele", which is used interchangeably herein witch "allelic variant" refers to alternative forms of a gene or portions thereof. Alleles occupy the same locus or position on homologous chromosomes. When a subject has two identical alleles of a gene, the subject is said to be homozygous for the gene or allele. When a subject has two different alleles of a gene, the subject is said to be heterozygous for the gene. Alleles of a specific gene can differ from each other in a single nucleotide, or several nucleotides, and can include substitutions, deletions, and insertions of nucleotides. An allele of a gene also can be a form of a gene containing a mutation.

As used herein, "predominant allele" refers to an allele that is represented in the greatest frequency for a given population. The allele or alleles that are present in lesser frequency are referred to as allelic variants.

As used herein, "associated" refers to coincidence with the development or manifestation of a disease, condition or phenotype. Association can be due to, but is not limited to, genes responsible for housekeeping functions whose alteration can provide the foundation for a variety of diseases and conditions, those that are part of a pathway that is involved in a specific disease, condition or phenotype and those that indirectly contribute to the manifestation of a disease, condition or phenotype.

As used herein, the term "subject" refers to a living organism, such as a mammal, a plant, a fungi, an invertebrate, a fish, an insect, a pathogenic organism, such as a virus or a bacterium, and, includes humans and other mammals.

As used herein, the term "gene" or "recombinant gene" refers to a nucleic acid molecule containing an open reading frame and including at least one exon and (optionally) an intron sequence. A gene can be either RNA or DNA. Genes can include regions preceding and following the coding region.

As used herein, "intron" refers to a DNA fragment present in a given gene that is spliced out during mRNA maturation.

As used herein, "nucleotide sequence complementary to the nucleotide sequence set forth in SEQ ID NO: x" refers to the nucleotide sequence of the complementary strand of a nucleic acid strand having SEQ ID NO: x. The term "complementary strand" is used herein interchangeably with the term "complement". The complement of a nucleic acid strand can be the complement of a coding strand or the complement of a non-coding strand. When referring to double stranded nucleic acids, the complement of a nucleic acid having SEQ ID NO: x refers to the complementary strand of the strand having SEQ ID NO: x or to any nucleic acid having the nucleotide sequence of the complementary strand of SEQ ID NO: x. When referring to a single stranded nucleic acid having the nucleotide sequence SEQ ID NO: x, the complement of this nucleic acid is a nucleic acid having a nucleotide sequence that is complementary to that of SEQ ID NO: x.

As used herein, the term "coding sequence" refers to that portion of a gene that encodes a amino acids that constitute a polypeptide or protein.

As used herein, the term "sense strand" refers to that strand of a double-stranded nucleic acid molecule that has the sequence of the mRNA that encodes the amino acid sequence encoded by the double-stranded nucleic acid molecule.

As used herein, the term "antisense strand" refers to that strand of a double-stranded nucleic acid molecule that is the complement of the sequence of the mRNA that encodes the amino acid sequence encoded by the double-stranded nucleic acid molecule.

As used herein, the amino acids, which occur in the various amino acid sequences appearing herein, are identified according to their well-known, three-letter or one-letter abbreviations. The nucleotides, which occur in the various DNA fragments, are designated with the standard single-letter designations used routinely in the art (see, Table 1).

As used herein, amino acid residue refers to an amino acid formed upon chemical digestion (hydrolysis) of a polypeptide at its peptide linkages. The amino acid residues described herein are, in certain embodiments, in the "L" isomeric form. Residues in the "D" isomeric form can be substituted for any L-amino acid residue, as long as the a desired functional property is retained by the polypeptide. NH₂ refers to the free amino group present at the amino terminus of a polypeptide. COOH refers to the free carboxy group present at the carboxyl terminus of a polypeptide. In keeping with standard polypeptide nomenclature described in J. Biol. Chem., 243:3552-59 (1969) and adopted at 37 C.F.R. § § 1.821 - 1.822, abbreviations for amino acid residues are shown in the following Table:

**Table 1**

| Table of Correspondence | | |
|---|---|---|
| SYMBOL | | |
| **1-Letter** | **3-Letter** | **AMINO ACID** |
| Y | Tyr | tyrosine |
| G | Gly | glycine |
| F | Phe | phenylalanine |
| M | Met | methionine |
| A | Ala | alanine |
| S | Ser | serine |
| I | Ile | isoleucine |
| L | Leu | leucine |
| T | Thr | threonine |
| V | Val | valine |
| P | Pro | proline |
| K | Lys | lysine |
| H | His | histidine |
| Q | Gln | glutamine |
| E | Glu | glutamic acid |
| Z | Glx | Glu and/or Gln |
| W | Trp | tryptophan |
| R | Arg | arginine |
| D | Asp | aspartic acid |
| N | Asn | asparagine |
| B | Asx | Asn and/or Asp |
| C | Cys | cysteine |
| X | Xaa | Unknown or other |

It should be noted that all amino acid residue sequences represented herein by formulae have a left to right orientation in the conventional direction of amino-terminus to carboxyl-terminus. In addition, the phrase "amino acid residue" is broadly defined to include the amino acids listed in the Table of Correspondence and modified and unusual amino acids, such as those referred to in 37 C.F.R. § § 1.821-1.822. Furthermore, it should be noted that a dash at the beginning or end of an amino acid residue sequence indicates a peptide bond to a further sequence of one or more amino acid residues or to an amino-terminal group such as NH₂ or to a carboxyl-terminal group such as COOH.

In a peptide or protein, suitable conservative substitutions of amino acids are known to those of skill in this art and can be made generally without altering the biological activity of the resulting molecule. Those of skill in this art recognize that, in general, single amino acid substitutions in non-essential regions of a polypeptide do not substantially alter biological activity (see, *e.g.*, Watson et al. Molecular Biology of the Gene, 4th Edition, 1987, The Benjamin/Cummings Pub. co., p.224).

Such substitutions can be made in accordance with those set forth in TABLE 2 as follows:

**TABLE 2**

| | |
|---|---|
| Ala (A) | Gly; Ser |
| Arg (R) | Lys |
| Asn (N) | Gln; His |
| Asp (D) | Glu |
| Cys (C) | Ser |
| Gln (Q) | Asn |
| Glu (E) | Asp |
| Gly (G) | Ala; Pro |
| His (H) | Asn; Gln |
| Ile (I) | Leu; Val |
| Leu (L) | Ile; Val |
| Lys (K) | Arg; Gln |
| Met (M) | Leu; Tyr; Ile |
| Phe (F) | Met; Leu; Tyr |
| Ser (S) | Thr |
| Thr (T) | Ser |
| Trp (W) | Tyr |
| Tyr (Y) | Trp; Phe |
| Val (V) | Ile; Leu |

Other substitutions are also permissible and can be determined empirically or in accord with known conservative substitutions.

As used herein, a DNA or nucleic acid homolog refers to a nucleic acid that includes a preselected conserved nucleotide sequence, such as a sequence encoding a therapeutic polypeptide. By the term "substantially homologous" is meant having at least 80%, at least 90%, at least 95% homology therewith or a less percentage of homology or identity and conserved biological activity or function.

The terms "homology" and "identity" are often used interchangeably. In this regard, percent homology or identity can be determined, for example, by comparing sequence information using a GAP computer program. The GAP program uses the alignment method of Needleman and Wunsch (*J. Mol. Bio*/*.* 48:443 (1970), as revised by Smith and Waterman (*Adv. Appl. Math.* 2:482 (1981). Briefly, the GAP program defines similarity as the number of aligned symbols (*e.g.*, nucleotides or amino acids) that are similar, divided by the total number of symbols in the shorter of the two sequences. The default parameters for the GAP program can include: (1) a unary comparison matrix (containing a value of 1 for identities and 0 for non-identities) and the weighted comparison matrix of Gribskov and Burgess, Nucl. Acids Res. 14:6745 (1986), as described by Schwartz and Dayhoff, eds., ATLAS OF PROTEIN SEQUENCE AND STRUCTURE, National Biomedical Research Foundation, pp. 353-358 (1979); (2) a penalty of 3.0 for each gap and an additional 0.10 penalty for each symbol in each gap; and (3) no penalty for end gaps.

Whether any two nucleic acid molecules have nucleotide sequences that are at least 80%, 85%, 90%, 95%, 96%, 97%, 98% or 99% "identical" can be determined using known computer algorithms such as the "FAST A" program, using for example, the default parameters as in Pearson and Lipman, Proc. Natl. Acad. Sci. USA 85:2444 (1988). Alternatively the BLAST function of the National Center for Biotechnology Information database can be used to determine identity

In general, sequences are aligned so that the highest order match is obtained. "Identity" *per se* has an art-recognized meaning and can be calculated using published techniques. (See, *e.g.:* Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H. & Lipton, D., SIAM J Applied Math 48:1073 (1988)). Methods commonly employed to determine identity or similarity between two sequences include, but are not limited to, those disclosed in Guide to Huge Computers, Martin J. Bishop, ed., Academic Press, San Diego, 1994, and Carillo, H. & Lipton, D., SIAM J Applied Math 48:1073 (1988). Methods to determine identity and similarity are codified in computer programs. Computer program methods to determine identity and similarity between two sequences include, but are not limited to, GCG program package (Devereux, J., et al., Nucleic Acids Research 12(I):387 (1984)), BLASTP, BLASTN, FASTA (Atschul, S.F., et al., J Molec Biol 215:403 (1990)).

Therefore, as used herein, the term "identity" represents a comparison between a test and a reference polypeptide or polynucleotide. For example, a test polypeptide can be defined as any polypeptide that is 90% or more identical to a reference polypeptide.

As used herein, the term at least "90% identical to" refers to percent identities from 90 to 99.99 relative to the reference polypeptides. Identity at a level of 90% or more is indicative of the fact that, assuming for exemplification purposes a test and reference polypeptide length of 100 amino acids are compared. No more than 10% (*e.g.*, 10 out of 100) amino acids in the test polypeptide differs from that of the reference polypeptides. Similar comparisons can be made between a test and reference polynucleotides. Such differences can be represented as point mutations randomly distributed over the entire length of an amino acid sequence or they can be clustered in one or more locations of varying length up to the maximum allowable, *e.g.*, 10/100 amino acid difference (approximately 90% identity). Differences are defined as nucleic acid or amino acid substitutions, or deletions.

As used herein: stringency of hybridization in determining percentage mismatch is as follows:
1) high stringency: 0.1 x SSPE, 0.1 % SDS, 65°C
2) medium stringency: 0.2 x SSPE, 0.1 % SDS, 50°C
3) low stringency: 1.0 x SSPE, 0.1 % SDS, 50°C

Those of skill in this art know that the washing step selects for stable hybrids and also know the ingredients of SSPE (see, *e.g.,* Sambrook, E.F. Fritsch, T. Maniatis, in: Molecular Cloning, A Laboratory Manual, Cold Spring Harbor Laboratory Press (1989), vol. 3, p. B.13, see, also, numerous catalogs that describe commonly used laboratory solutions). SSPE is pH 7.4 phosphate- buffered 0.18 NaCl. Further, those of skill in the art recognize that the stability of hybrids is determined by Tₘ, which is a function of the sodium ion concentration and temperature (Tₘ = 81.5° C-16.6(log₁₀[Na+]) + 0.41 (%G+C)-600/l)), so that the only parameters in the wash conditions critical to hybrid stability are sodium ion concentration in the SSPE (or SSC) and temperature.

It is understood that equivalent stringencies can be achieved using alternative buffers, salts and temperatures. By way of example and not limitation, procedures using conditions of low stringency are as follows (see also Shilo and Weinberg, Proc. Natl. Acad. Sci. USA 78:6789-6792 (1981)): Filters containing DNA are pretreated for 6 hours at 40°C in a solution containing 35% formamide; 5X SSC, 50 mM Tris-HCl (pH 7.5), 5 mM EDTA, 0.1 % PVP, 0.1% Ficoll, 1 % BSA, and 500 µg/ml denatured salmon sperm DNA (10X SSC is 1.5 M sodium chloride, and 0.15 M sodium citrate, adjusted to a pH of 7).

Hybridizations are carried out in the same solution with the following modifications: 0.02% PVP, 0.02% Ficoll, 0.2% BSA, 100 µg/ml salmon sperm DNA, 10% (wt/vol) dextran sulfate, and 5-20 X 10⁶ cpm ³²P-labeled probe is used. Filters are incubated in hybridization mixture for 18-20 hours at 40°C, and then washed for 1.5 hours at 55 °C in a solution containing 2X SSC, 25 mM Tris-HCl (pH 7.4), 5 mM EDTA, and 0.1 % SDS. The wash solution is replaced with fresh solution and incubated an additional 1.5 hours at 60°C. Filters are blotted dry and exposed for autoradiography. If necessary, filters are washed for a third time at 65-68°C and reexposed to film. Other conditions of low stringency which can be used are well known in the art (*e.g.*, as employed for cross-species hybridizations).

By way of example and not way of limitation, procedures using conditions of moderate stringency include, for example, but are not limited to, procedures using such conditions of moderate stringency are as follows: Filters containing DNA are pretreated for 6 hours at 55°C in a solution containing 6X SSC, 5X Denhart's solution, 0.5% SDS and 100 µg/ml denatured salmon sperm DNA. Hybridizations are carried out in the same solution and 5-20 X 10⁶ cpm ³²P-labeled probe is used. Filters are incubated in hybridization mixture for 18-20 hours at 55°C, and then washed twice for 30 minutes at 60°C in a solution containing 1X SSC and 0.1 % SDS. Filters are blotted dry and exposed for autoradiography. Other conditions of moderate stringency which can be used are well-known in the art. Washing of filters is done at 37°C for 1 hour in a solution containing 2X SSC, 0.1 % SDS.

By way of example and not way of limitation, procedures using conditions of high stringency are as follows: Prehybridization of filters containing DNA is carried out for 8 hours to overnight at 65°C in buffer composed of 6X SSC, 50 mM Tris-HCl (pH 7.5), 1 mM EDTA, 0.02% PVP, 0.02% Ficoll, 0.02% BSA, and 500 µg/ml denatured salmon sperm DNA. Filters are hybridized for 48 hours at 65°C in prehybridization mixture containing 100 µg/ml denatured salmon sperm DNA and 5-20 X 10⁶ cpm of ³²P-labeled probe. Washing of filters is done at 37°C for 1 hour in a solution containing 2X SSC, 0.01 % PVP, 0.01 % Ficoll, and 0.01 % BSA. This is followed by a wash in 0.1X SSC at 50°C for 45 minutes before autoradiography. Other conditions of high stringency which can be used are well known in the art.

The term substantially identical or substantially homologous or similar varies with the context as understood by those skilled in the relevant art and generally means at least 60% or 70%, preferably means at least 80%, 85% or more preferably at least 90%, and most preferably at least 95% identity.

It is to be understood that the compounds provided herein can contain chiral centers. Such chiral centers can be of either the (R) or (S) configuration, or can be a mixture thereof. Thus, the compounds provided herein can be enantiomerically pure, or be stereoisomeric or diastereomeric mixtures. In the case of amino acid residues, such residues can be of either the L- or D-form. In one embodiment, the configuration for naturally occurring amino acid residues is L.

As used herein, substantially pure means sufficiently homogeneous to appear free of readily detectable impurities as determined by standard methods of analysis, such as thin layer chromatography (TLC), gel electrophoresis, high performance liquid chromatography (HPLC) and mass spectrometry (MS), used by those of skill in the art to assess such purity, or sufficiently pure such that further purification would not detectably alter the physical and chemical properties, such as enzymatic and biological activities, of the substance. Methods for purification of the compounds to produce substantially chemically pure compounds are known to those of skill in the art. A substantially chemically pure compound can, however, be a mixture of stereoisomers. In such instances, further purification might increase the specific activity of the compound.

As used herein, a cleavable bond or moiety refers to a bond or moiety that is cleaved or cleavable under the specific conditions, such as chemically, enzymatically or photolytically. Where not specified herein, such bond is cleavable under conditions of MALDI-MS analysis, such as by a UV or IR laser.

As used herein, a "selectively cleavable" moiety is a moiety that can be selectively cleaved without affecting or altering the composition of the other portions of the compound of interest. For example, a cleavable moiety L of the compounds provided herein is one that can be cleaved by chemical, enzymatic, photolytic, or other means without affecting or altering composition (*e.g.*, the chemical composition) of the conjugated biomolecule, including a protein. "Non-cleavable" moieties are those that cannot be selectively cleaved without affecting or altering the composition of the other portions of the compound of interest.

As used herein, binding with high affinity refers to a binding that as an association constant kₐ of at least 10⁹ and generally 10¹⁰, 10¹¹ liters/mole or greater) or a K_{eq} of 10⁹, 10¹⁰, 10¹¹, 10¹² or greater. For purposes herein, high affinity bonds formed by the reactivity groups are those that are stable to the laser (UV and IR) used in MALDI-MS analyses.

As used herein, "alkyl", "alkenyl" and "alkynyl", if not specified, contain from 1 to 20 carbons, or 1 to 16 carbons, and are straight or branched carbon chains. Alkenyl carbon chains are from 2 to 20 carbons, and, in certain embodiments, contain 1 to 8 double bonds. Alkenyl carbon chains of 1 to 16 carbons, in certain embodiments, contain 1 to 5 double bonds. Alkynyl carbon chains are from 2 to 20 carbons, and, in one embodiment, contain 1 to 8 triple bonds. Alkynyl carbon chains of 2 to 16 carbons, in certain embodiments, contain 1 to 5 triple bonds. Exemplary alkyl, alkenyl and alkynyl groups include, but are not limited to, methyl, ethyl, propyl, isopropyl, isobutyl, n-butyl, sec-butyl, tert-butyl, isopentyl, neopentyl, tert-penytyl and isohexyl. The alkyl, alkenyl and alkynyl groups, unless otherwise specified, can be optionally substituted, with one or more groups, including alkyl group substituents that can be the same or different.

As used herein, "lower alkyl", "lower alkenyl", and "lower alkynyl" refer to carbon chains having less than about 6 carbons.

As used herein, "alk(en)(yn)yl" refers to an alkyl group containing at least one double bond and at least one triple bond.

As used herein, an "alkyl group substituent" includes, but is not limited to, halo, haloalkyl, including halo lower alkyl, aryl, hydroxy, alkoxy, aryloxy, alkyloxy, alkylthio, arylthio, aralkyloxy, aralkylthio, carboxy alkoxycarbonyl, oxo and cycloalkyl.

As used herein, "aryl" refers to aromatic groups containing from 5 to 20 carbon atoms and can be a mono-, multicyclic or fused ring system. Aryl groups include, but are not limited to, phenyl, naphthyl, biphenyl, fluorenyl and others that can be unsubstituted or are substituted with one or more substituents.

As used herein, "aryl" also refers to aryl-containing groups, including, but not limited to, aryloxy, arylthio, arylcarbonyl and arylamino groups.

As used herein, an "aryl group substituent" includes, but is not limited to, alkyl, alkenyl, alkynyl, cycloalkyl, cycloalkylalkyl, aryl, heteroaryl optionally substituted with 1 or more, including 1 to 3, substituents selected from halo, halo alkyl and alkyl, aralkyl, heteroaralkyl, alkenyl containing 1 to 2 double bonds, alkynyl containing 1 to 2 triple bonds, alk(en)(yn)yl groups, halo, pseudohalo, cyano, hydroxy, haloalkyl and polyhaloalkyl, including halo lower alkyl, especially trifluoromethyl, formyl, alkylcarbonyl, arylcarbonyl that is optionally substituted with 1 or more, including 1 to 3, substituents selected from halo, halo alkyl and alkyl, heteroarylcarbonyl, carboxy, alkoxycarbonyl, aryloxycarbonyl, aminocarbonyl, alkylaminocarbonyl, dialkylaminocarbonyl, arylaminocarbonyl, diarylaminocarbonyl, aralkylaminocarbonyl, alkoxy, aryloxy, perfluoroalkoxy, alkenyloxy, alkynyloxy, arylalkoxy, aminoalkyl, alkylaminoalkyl, dialkylaminoalkyl, arylaminoalkyl, amino, alkylamino, dialkylamino, arylamino, alkylarylamino, alkylcarbonylamino, arylcarbonylamino, azido, nitro, mercapto, alkylthio, arylthio, perfluoroalkylthio, thiocyano, isothiocyano, alkylsulfinyl, alkylsulfonyl, arylsulfinyl, arylsulfonyl, aminosulfonyl, alkylaminosulfonyl, dialkylaminosulfonyl and arylaminosulfonyl.

As used herein, "aralkyl" refers to an alkyl group in that one of the hydrogen atoms of the alkyl is replaced by an aryl group.

As used herein, "heteroaralkyl" refers to an alkyl group in that one of the hydrogen atoms of the alkyl is replaced by a heteroaryl group.

As used herein, "cycloalkyl" refers to a saturated mono- or multicyclic ring system, in one embodiment, of 3 to 10 carbon atoms, or 3 to 6 carbon atoms; cycloalkenyl and cycloalkynyl refer to mono- or multicyclic ring systems that respectively include at least one double bond and at least one triple bond. Cycloalkenyl and cycloalkynyl groups can contain, in one embodiment, 3 to 10 carbon atoms, with cycloalkenyl groups, in other embodiments, containing 4 to 7 carbon atoms and cycloalkynyl groups, in other embodiments, containing 8 to 10 carbon atoms. The ring systems of the cycloalkyl, cycloalkenyl and cycloalkynyl groups can be composed of one ring or two or more rings that can be joined together in a fused, bridged or spiro-connected fashion, and can be optionally substituted with one or more alkyl group substituents. "Cycloalk(en)(yn)yl" refers to a cycloalkyl group containing at least one double bond and at least one triple bond.

As used herein, "heteroaryl" refers to a monocyclic or multicyclic ring system, in one embodiment of about 5 to about 15 members where one or more, or 1 to 3, of the atoms in the ring system is a heteroatom, which is, an element other than carbon, for example, nitrogen, oxygen and sulfur atoms. The heteroaryl can be optionally substituted with one or more, including 1 to 3, aryl group substituents. The heteroaryl group can be optionally fused to a benzene ring. Exemplary heteroaryl groups include, but are not limited to, pyrroles, porphyrines, furans, thiophenes, selenophenes, pyrazoles, imidazoles, triazoles, tetrazoles, oxazoles, oxadiazoles, thiazoles, thiadiazoles, indoles, carbazoles, benzofurans, benzothiophenes, indazoles, benzimidazoles, benzotriazoles, benzoxatriazoles, benzothiazoles, benzoselenozoles, benzothiadiazoles, benzoselenadiazoles, purines, pyridines, pyridazines, pyrimidines, pyrazines, pyrazines, triazines, quinolines, acridines, isoquinolines, cinnolines, phthalazines, quinazolines, quinoxalines, phenazines, phenanthrolines, imidazinyl, pyrrolidinyl, pyrimidinyl, tetrazolyl, thienyl, pyridyl, pyrrolyl, N-methylpyrrolyl, quinolinyl and isoquinolinyl.

As used herein, "heteroaryl" also refers to heteroaryl-containing groups, including, but not limited to, heteroaryloxy, heteroarylthio, heteroarylcarbonyl and heteroarylamino.

As used herein, "heterocyclic" refers to a monocyclic or multicyclic ring system, in one embodiment of 3 to 10 members, in another embodiment 4 to 7 members, including 5 to 6 members, where one or more, including 1 to 3 of the atoms in the ring system is a heteroatom, which is, an element other than carbon, for example, nitrogen, oxygen and sulfur atoms. The heterocycle can be optionally substituted with one or more, or 1 to 3 aryl group substituents. In certain embodiments, substituents of the heterocyclic group include hydroxy, amino, alkoxy containing 1 to 4 carbon atoms, halo lower alkyl, including trihalomethyl, such as trifluoromethyl, and halogen. As used herein, the term heterocycle can include reference to heteroaryl.

As used herein, the nomenclature alkyl, alkoxy, carbonyl, etc., are used as is generally understood by those of skill in this art. For example, as used herein alkyl refers to saturated carbon chains that contain one or more carbons; the chains can be straight or branched or include cyclic portions or be cyclic.

Where the number of any given substituent is not specified (*e.g.*, "haloalkyl"), there can be one or more substituents present. For example, "haloalkyl" can include one or more of the same or different halogens. As another example, "C₁₋₃alkoxyphenyl" can include one or more of the same or different alkoxy groups containing one, two or three carbons.

Where named substituents such as carboxy or substituents represented by variables such as W are separately enclosed in parentheses, yet possess no subscript outside the parentheses indicating numerical value and that follow substituents not in parentheses, *e.g.*, "C₁₋₄ alkyl(W)(carboxy)", "W" and "carboxy" are each directly attached to C₁₋₄ alkyl.

As used herein, "halogen" or "halide" refers to F, Cl, Br or I.

As used herein, pseudohalides are compounds that behave substantially similar to halides. Such compounds can be used in the same manner and treated in the same manner as halides (X⁻, in that X is a halogen, such as Cl or Br). Pseudohalides include, but are not limited to, cyanide, cyanate, isocyanate, thiocyanate, isothiocyanate, selenocyanate, trifluoromethoxy, and azide.

As used herein, "haloalkyl" refers to a lower alkyl radical in that one or more of the hydrogen atoms are replaced by halogen including, but not limited to, chloromethyl, trifluoromethyl, 1-chloro-2-fluoroethyl and the like.

As used herein, "haloalkoxy" refers to RO- in that R is a haloalkyl group.

As used herein, "sulfinyl" or "thionyl" refers to -S(O)-. As used herein, "sulfonyl" or "sulfuryl" refers to -S(O)₂-. As used herein, "sulfo" refers to -S(O)₂O-.

As used herein, "carboxy" refers to a divalent radical, -C(O)O-.

As used herein, "aminocarbonyl" refers to -C(O)NH₂.

As used herein, "alkylaminocarbonyl" refers to -C(O)NHR in that R is hydrogen or alkyl, including lower alkyl.

As used herein "dialkylaminocarbonyl" as used herein refers to -C(O)NR'R in that R' and R are independently selected from hydrogen or alkyl, including lower alkyl.

As used herein, "carboxamide" refers to groups of formula -NR'COR.

As used herein, "diarylaminocarbonyl" refers to -C(O)NRR' in that R and R' are independently selected from aryl, including lower aryl, such as phenyl.

As used herein, "aralkylaminocarbonyl" refers to -C(O)NRR' in that one of R and R' is aryl, including lower aryl, such as phenyl, and the other of R and R' is alkyl, including lower alkyl.

As used herein, "arylaminocarbonyl" refers to -C(O)NHR in that R is aryl, including lower aryl, such as phenyl.

As used herein, "alkoxycarbonyl" refers to -C(O)OR in that R is alkyl, including lower alkyl.

As used herein, "aryloxycarbonyl" refers to -C(O)OR in that R is aryl, including lower aryl, such as phenyl.

As used herein, "alkoxy" and "alkylthio" refer to RO- and RS-, in that R is alkyl, including lower alkyl.

As used herein, "aryloxy" and "arylthio" refer to RO- and RS-, in that R is aryl, including lower aryl, such as phenyl.

As used herein, "alkylene" refers to a straight, branched or cyclic, in one embodiment straight or branched, divalent aliphatic hydrocarbon group, in certain embodiments having from 1 to about 20 carbon atoms, in other embodiments 1 to 1 2 carbons, including lower alkylene. The alkylene group is optionally substituted with one or more "alkyl group substituents." There can be optionally inserted along the alkylene group one or more oxygen, sulphur or substituted or unsubstituted nitrogen atoms, where the nitrogen substituent is alkyl as previously described. Exemplary alkylene groups include methylene (-CH₂-), ethylene (-CH₂CH₂-), propylene (-(CH₂)₃-), cyclohexylene (-C₆H₁₀-), methylenedioxy (-O-CH₂-O-) and ethylenedioxy (-O-(CH₂)₂-O-). The term "lower alkylene" refers to alkylene groups having 1 to 6 carbons. In certain embodiments, alkylene groups are lower alkylene, including alkylene of 1 to 3 carbon atoms.

As used herein, "alkenylene" refers to a straight, branched or cyclic, in one embodiment straight or branched, divalent aliphatic hydrocarbon group, in certain embodiments having from 2 to about 20 carbon atoms and at least one double bond, in other embodiments 1 to 12 carbons, including lower alkenylene. The alkenylene group is optionally substituted with one or more "alkyl group substituents." There can be optionally inserted along the alkenylene group one or more oxygen, sulphur or substituted or unsubstituted nitrogen atoms, where the nitrogen substituent is alkyl as previously described. Exemplary alkenylene groups include -CH=CH-CH=CH- and -CH=CH-CH₂-. The term "lower alkenylene" refers to alkenylene groups having 2 to 6 carbons. In certain embodiments, alkenylene groups are lower alkenylene, including alkenylene of 3 to 4 carbon atoms.

As used herein, "alkynylene" refers to a straight, branched or cyclic, in one embodiment straight or branched, divalent aliphatic hydrocarbon group, in certain embodiments having from 2 to about 20 carbon atoms and at least one triple bond, in other embodiments 1 to 12 carbons, including lower alkynylene. The alkynylene group is optionally substituted with one or more "alkyl group substituents." There can be optionally inserted along the alkynylene group one or more oxygen, sulphur or substituted or unsubstituted nitrogen atoms, where the nitrogen substituent is alkyl as previously described. Exemplary alkynylene groups include -C≡C-C≡C-, -C≡C- and -C≡C-CH₂-. The term "lower alkynylene" refers to alkynylene groups having 2 to 6 carbons. In certain embodiments, alkynylene groups are lower alkynylene, including alkynylene of 3 to 4 carbon atoms.

As used herein, "alk(en)(yn)ylene" refers to a straight, branched or cyclic, in one embodiment straight or branched, divalent aliphatic hydrocarbon group, in certain embodiments having from 2 to about 20 carbon atoms and at least one triple bond, and at least one double bond; in other embodiments 1 to 12 carbons, including lower alk(en)(yn)ylene. The alk(en)(yn)ylene group is optionally substituted with one or more "alkyl group substituents." There can be optionally inserted along the alkynylene group one or more oxygen, sulphur or substituted or unsubstituted nitrogen atoms, where the nitrogen substituent is alkyl as previously described. Exemplary alk(en)(yn)ylene groups include -C=C-(CH₂ₙ-C≡C-, where n is 1 or 2. The term "lower alk(en)(yn)ylene" refers to alk(en)(yn)ylene groups having up to 6 carbons. In certain embodiments, alk(en)(yn)ylene groups are lower alk(en)(yn)ylene, including alk(en)(yn)ylene of 4 carbon atoms.

As used herein, "arylene" refers to a monocyclic or polycyclic, in one embodiment monocyclic, divalent aromatic group, in certain embodiments having from 5 to about 20 carbon atoms and at least one aromatic ring, in other embodiments 5 to 12 carbons, including lower arylene. The arylene group is optionally substituted with one or more "alkyl group substituents." There can be optionally inserted around the arylene group one or more oxygen, sulphur or substituted or unsubstituted nitrogen atoms, where the nitrogen substituent is alkyl as previously described. Exemplary arylene groups include 1,2-, 1,3- and 1,4-phenylene. The term "lower arylene" refers to arylene groups having 5 or 6 carbons. In certain embodiments, arylene groups are lower arylene.

As used herein, "heteroarylene" refers to a divalent monocyclic or multicyclic ring system, in one embodiment of about 5 to about 15 members where one or more, or 1 to 3 of the atoms in the ring system is a heteroatom, which is, an element other than carbon, for example, nitrogen, oxygen and sulfur atoms. The heteroarylene group can be optionally substituted with one or more, or 1 to 3, aryl group substituents.

As used herein, "alkylidene" refers to a divalent group, such as =CR'R", which is attached to one atom of another group, forming a double bond. Exemplary alkylidene groups are methylidene (=CH₂) and ethylidene (=CHCH₃). As used herein, "aralkylidene" refers to an alkylidene group in that either R' or R" is and aryl group.

As used herein, "amido" refers to the divalent group -C(O)NH-. "Thioamido" refers to the divalent group -C(S)NH-. "Oxyamido" refers to the divalent group -OC(O)NH-. "Thiaamido" refers to the divalent group -SC(O)NH-. "Dithiaamido" refers to the divalent group -SC(S)NH-. "Ureido" refers to the divalent group -HNC(O)NH-. "Thioureido" refers to the divalent group -HNC(S)NH-.

As used herein, "semicarbazide" refers to -NHC(O)NHNH-. "Carbazate" refers to the divalent group -OC(O)NHNH-. "Isothiocarbazate" refers to the divalent group -SC(O)NHNH-. "Thiocarbazate" refers to the divalent group -OC(S)NHNH-. "Sulfonylhydrazide" refers to the group -SO₂NHNH-. "Hydrazide" refers to the divalent groups -C(O)NHNH-. "Azo" refers to the divalent group -N = N-. "Hydrazinyl" refers to the divalent group -NH-NH-.

As used herein, the term "amino acid" refers to α-amino acids that are racemic, or of either the D- or L-configuration. The designation "d" preceding an amino acid designation (*e.g.*, dAla, dSer, dVal, etc.) refers to the D-isomer of the amino acid. The designation "dl" preceding an amino acid designation (*e.g.*, dlAla) refers to a mixture of the L- and D-isomers of the amino acid.

As used herein, when any particular group, such as phenyl or pyridyl, is specified, this means that the group is unsubstituted or is substituted. Substituents where not specified are halo, halo lower alkyl, and lower alkyl.

As used herein, conformationally altered protein disease (or a disease of protein aggregation) refers to diseases associated with a protein or polypeptide that has a disease-associated conformation. The methods and collections provided herein permit detection of a conformer associated with a disease to be detected. Diseases and associated proteins that exhibit two or more different conformations in which at least one conformation is a conformationally altered protein, include, but are not limited to amyloid diseases and other neurodegenerative diseases known to those of skill in the art and set forth below.

As used herein, cell sorting refers to an assay in which cells are separated and recovered from suspension based upon properties measured in flow cytometry analysis. Most assays used for analysis can serve as the basis for sorting experiments, as long as gates and regions defining the subpopulation(s) to be sorted do not logically overlap. Maximum throughput rates are typically 5000 cells/second (18 x 106 cells/hour). The rate of collection of the separated population(s) depends primarily upon the condition of the cells and the percentage of reactivity.

As used herein, the abbreviations for any protective groups, amino acids and other compounds, are, unless indicated otherwise, in accord with their common usage, recognized abbreviations, or the IUPAC-IUB Commission on Biochemical Nomenclature (see, Biochem. 1972, 11:942). For example, DMF = *N*,*N*-dimethylformamide, DMAc =*N*,*N*-dimethylacetamide; THF = tetrahydrofuran; TRIS = tris(hydroxymethyl)aminomethane; SSPE = saline-sodium phosphate-EDTA buffer; EDTA = ethylenediaminetetraacetic acid; SDS = sodium dodecyl sulfate.

### B. Arrays of compounds

Gradient arrays of capture compounds that selectively bind to biomolecules in samples, such as biomolecules, particurlarly, although not exclusively, a cell lysate or *in vitro* translated polypeptides from a cell lysate are provided. Each locus in the array can bind to specific groups or classes of biolopolymers, and is designed to covalently or tightly (sufficient to sustain mass spectrmetric analysis, for example) to a subset of all of the biomolecules in the sample. For example, a sample can contain 1000's of members, for example a cell lysate. The arrays of compounds permit sufficient selectivity so that, for example, about 10-20 of the components of the sample bind to each member of the collection. The exact number is a small enough number for routine analyses to identify them, generally in one step, such as by mass spectrometry.

The arrays permit a top down holistic approach to analysis of the proteome, including post-translationally modified proteins, and other biomolecules. Protein and other biomolecule patterns are the starting point for analyses that use these arrayss; rather than nucleic acids and the genome (bottom up). The arrays can be used to assess the biomolecule components of a sample, such as a biological sample, to identify components specific to a particular phenotype, such as a disease state, to identify structural function, biochemical pathways and mechanisms of action. The arrays and methods of use permit an unbiased analysis of biomolecules, since the methods do not necessarily assess specific classes of targets, instead, changes in samples are detected or identified. The arrays permit the components of a complex mixture of biomolecules (*i.e.*, a mixture of 50, 100, 500, 1000, 2000 and more) to be sorted into discrete loci containing reduced numbers, typically by 10%, 50% or greater reduction in complexity, or to about 1 to 50 different biomolecules per locus in an array, so that the components at each spot can be analyzed, such as by mass spectrometric analysis alone or in combination with other analyses. In some embodiments, such as for phenotypic analyses, homogeneity of the starting sample, such as cells, can be important. To provide homogeneity, cells, with different phenotypes, such as diseased versus healthy, from the same individual are compared. Methods for doing so are provided herein.

By virtue of the structure of moieties at the loci in the arrays, the arrays can be used to detect structural changes, such as those from the post-translational processing of proteins, and can be used to detect changes in membrane proteins, which are involved in the most fundamental processes, such as, signal transduction, ion channels, receptors for ligand interaction and cell-to-cell interactions. When cells become diseased, changes associated with disease, such as transformation, often occur in membrane proteins.

The arrays contain sets of moieties at each locus. The moieties at each locus include an X and a Y group. In general members of each set differs differ in at least one property group, and generally in two or three or more from each other locus. As provided herein, the differences comprise a gradient, typically a two-dimensional gradient based upon gradual changes in properties of each X and Y at each locus and accross the X and Y axes in the array.

In practicing methods, the arrays are contacted with a sample or partially purified or purified components thereof to effect binding of biomolecules to capture compounds in the array. The resulting array is optionally treated with a reagent that specifically cleaves the bound polymers, such as a protease, and is subjected to analysis, particularly mass spectrometric analysis to identify components of the bound biomolecules at each locus. Once a molecular weight of a biomolecule, such as a protein or portion thereof of interest is determined, the biomolecule can be identified. Methods for identification include comparison of the molecular weights with databases, for example protein databases that iclude protease fragments and their molecular weights.

The X and Y groups are functional groups that confer reactivity, selectivity and separative properties, depending on the specificity of separation and analysis required (which depends on the complexity of the mixture to be analyzed). In general, the loci in the arrays include at least two functional groups (functions) selected from: a reactivity function (X), which binds to biolopolymers either covalently or with a high kₐ (generally greater than about 10⁹, 10¹⁰, 10¹² liters/mole and/or such that the binding is substantially irreversible or stable under conditions of mass spectrometric analyses, such as MALDI-MS conditions); and a selectivity function (Y), which by virtue of non-covalent interactions alters, generally increases, the specificity of the reactivity function.

In general, the reactivity function that is reative group that specifically interacts, tyically covalently or with high binding affinity (kₐ), with particular biomolecules, such as proteinsm, or portions thereof,; and the other functionality, the selectivity functions, alters, typically increasing, the specificity of the reactivity function. In general, the reactive function covalently interacts with groups on a particular biomolecule, such amine groups on the surface of a protein. The reactivity function interacts with biomolecules to form a covalent bond or a non-covalent bond that is stable under conditions of analyis, generally with a kₐ of greater than 10⁹ liters/mole or greater than 10¹⁰ liters/mole. Conditions of analysis include, but are not limited to, mass spectrophotometric analysis, such as matrix assisted laser desorption ionization-time of flight (MALDI-TOF) mass spectrometry. The selectivity function influences the types of biomolecules that can interact with the reactivity function through a non-covalent interaction. The selectivity function alters the specificity for the particular groups, generally reducing the number of such groups with which the reactivity functions reacts. A goal is to reduce the the number of proteins or biomolecules bound at a locus, so that the proteins which can then be separated, such as by mass spectrometry.

Included among the moieties as provided herein are those that can be classified in at least two sets: one for reactions in aqueous solution (*e.g.,* for reaction with hydrophilic biomolecules), and the other for reaction in organic solvents (*e.g.*, chloroform) (*e.g.*, for reaction with hydrophobic biomolecules). Thus, in certain embodiments, the arrays herein discriminate between hydrophilic and hydrophobic biomolecules, including, but not limited to, proteins, and allow for analysis of both classes of biomolecules.

### C. Components of the arrays

Arrays with loci containing bound moieties (also referred to as capture agents) are provided. The arrays include a core "Z", the solid surface, that presents one or more reactivity functions "X" and optionally one or more of a selectivity function "Y", which are arrayed at the loci. The particular manner in which the functions are presented on the solid surface (Z) is a matter of design choice, but are selected such that the resulting loci captures biomolecules, particularly proteins, with sufficient specificity and either covalently or with bonds of sufficient stablity or affinity to permit analysis, such as by mass spectrometry, including MALDI mass spectrometric analysis, so that at least a portion of bound biomolecules remain bound (generally a binding affinity of 10⁹, 10¹⁰, 10¹¹ liters/mole or greater, or a K_{eq} of 10⁹, 10¹⁰, 10¹¹, 10¹² or greater).

X, the reactivity functionality is selected to be anything that forms such a covalent bond or a bond of high affinity that is stable under conditions of mass spectrometric analysis, particularly MALDI analysis. The selectivity functionality Y, is a group that "looks" at the topology of the protein around reactivity binding sites and functions to select particular groups on biolmolecules from among those with which a reactivity group can form a covalent bond (or high affinity bond). For example a selectivity group can cause steric hindrance, or permit specific binding to an epitope, or anything in between. It can be a substrate for a drug, lipid, peptide. It selects the environment of the groups with which the reactivity function interacts. The selectivity functionality Y, can be one whereby a capture compound forms a covalent bond with a biomolecule in a mixture or interacts with high stabilty such that the affinity of binding of the capture compound to the biomolecule through the reactive functionality in the presence of the selectivity functionality is at least ten-fold or 100-fold greater than in the absence of the selectivity functionality.

Reactivity functions ("X") confer the ability on the compounds the ability to bind either covalently or with a high affinity (greater than 10⁹, generally greater than 10¹⁰ or 10¹¹ liters/mole, typically greater than a monoclonal antibody, and typically stable to mass spectrometric analysis, such as MALDI-MS) to a biomolecule, particularly proteins, including functional groups thereon, which include post-translationally added groups. Generally the binding is covalent or is of such affinity that it is stable under conditions of analysis, such as mass spectral, including MALDI-TOF, analysis. Exemplary groups are set forth herein.

In the compounds provided herein, X is a moiety that binds to an amino acid side chain

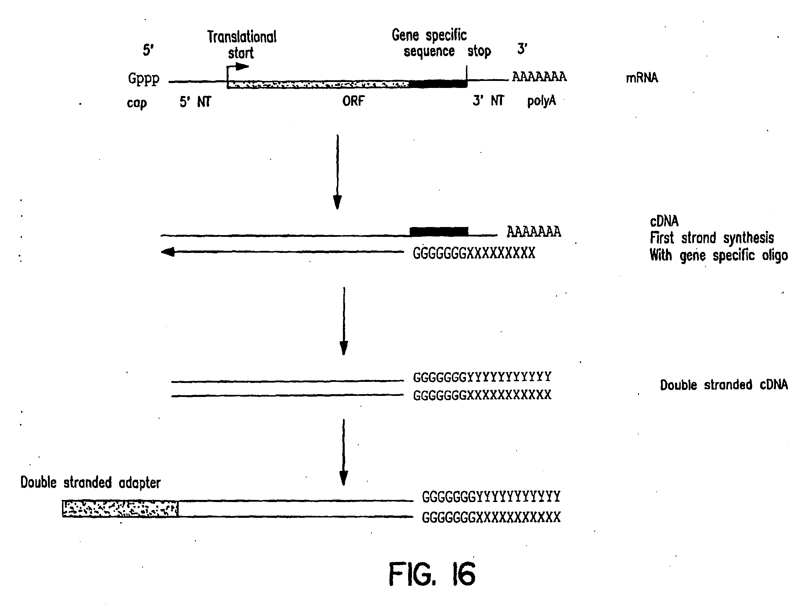

X is a group that reacts or interacts with functionalities on the surface of a protein to form covalent or non-covalent bonds with high affinity. A wide selection of different functional groups are available for X to interact with a protein. X can act either as a nucleophile or an electrophile to form covalent bonds upon reaction with the amino acid residues on the surface of a protein. Exemplary reagents that bind covalently to amino acid side chains include, but are not limited to, protecting groups for hydroxyl, carboxyl, amino, amide, and thiol moieties, including, for example, those disclosed in T.W. Greene and P.G.M. Wuts, "Protective Groups in Organic Synthesis," 3rd ed. (1999, Wiley Interscience); photoreactive groups, Diels Alder couples (*i.e.,* a diene on one side and a sngle double bond on the other side). Other groups for X include those described in co-pending U.S. application Serial No. 10/197,954 (see Figure 16).

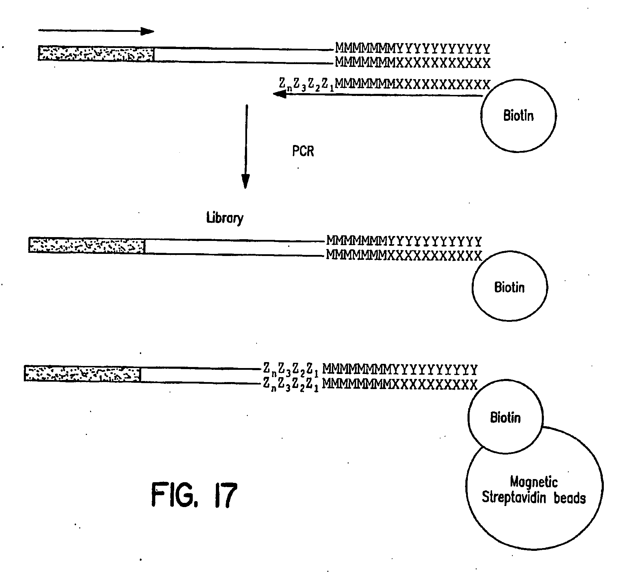

The selectivity functions ("Y") serves to modulate the reactivity function by reducing the number of groups to which the reactivity functions binds, such as by steric hindrance and other interactions. It is a group that modifies the steric and/or electronic (*e.g.*, mesomeric, inductive effects) properties as well as the resulting affinities properties of the capture compound. Selectivity functions include any functional groups that increase the selectivity of the reactivity group so that it binds to fewer different biomolecules than in the absence of the selectivity function or binds with greater affinity to biolmolecules than in its absence. In the capture compounds provided herein, Y is allowed to be extensively varied depending on the goal to be achieved regarding steric hindrance and electronic factors as they relate to modulating the reactivity of the cleavable bond L, if present, and the reactive functionality X. For example, a reactivity function X can be selected to bind to amine groups on proteins; the selectivity function can be selected to ensure that only groups exposed on the surface can be accessed. The selectivity function is such that the compounds bind to or react with (via the reactivity function) fewer different biomolecules when it is part of the molecule than when it is absent and/or the compounds bind with greater specificity and higher affinity The selectivity function can be attached directly to a compounds or can be attached via a linker, such as CH₂CO₂ or CH₂-O-(CH₂)ₙ-O, where n is an integer from 1 to 12, or 1 to 6, or 2 to 4. Other groups for Y include those described in co-pending U.S. application Serial No. 10/197,954 (see Figure 17).

In certain embodiments, each Y is independently a group that modifies the affinity properites and/or steric and/or electronic (*e.g.*, mesomeric, inductive effects) properties of the resulting capture compound. For example, Y, in certain embodiments, is selected from ATP analogs and inhibitors; peptides and peptide analogs; polyethyleneglycol (PEG); activated esters of amino acids, isolated or within a peptide; cytochrome C; and hydrophilic trityl groups.

In another embodiment, Y is a small molecule moiety, a natural product, a protein agonist or antagonist, a peptide or an antibody. In another embodiment, Y is a hydrophilic compound or protein (*e.g.*, PEG or trityl ether), a hydrophobic compound or protein (*e.g.,* polar aromatics, lipids, glycolipids, phosphotriesters, oligosaccharides), a positive or negatively charged group, a small molecule, a pharmaceutical compound or a biomolecule that creates defined secondary or tertiary structures.

More detailed description and discussion of each functionality in the description that follows.

### Exemplary compounds and descriptions of X and Y

It is understood that for the gradient arrays provided here, "Z" is a solid support to which X, and Y (and any other groups) are bound or otherwise linked, directly or indirectly.

In one embodiment, the compounds for use in the methods provided herein have formulae:

X-Q-Y

in which Q is a single stranded unprotected or suitably protected oligonucleotide or oligonucleotide analog (*e.g.,* PNA) of up to 50 building blocks, which is capable of hybridizing with a base-complementary single stranded nucleic acid molecule;
X is a functional group which interacts with and/or reacts with functionalities on the surface of a biomolecule, including, but not limited to, a protein, to form covalent bonds or bonds stable under conditions of mass spectrometric analysis as defined herein; and
Y is a functional group which interacts with and/or reacts by imposing unique selectivity by introducing functionalities which interact noncovalently with target proteins.

In another embodiment, the compounds for use in the methods provided herein have formula: 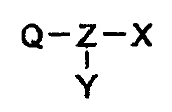 in which Q is a single stranded unprotected or suitably protected oligonucleotide or oligonucleotide analog (*e.g.*, peptide nucleic acid (PNA)) of up to 50 building blocks, which is capable of hybridizing with a base-complementary single stranded nucleic acid molecule;
Z is a solid support;
X is a functional group which interacts with and/or reacts with functionalities on the surface of a biomolecule, including, but not limited to, a protein, to form covalent bonds or bonds stable under conditions of mass spectrometric analysis; and
Y is a functional group which interacts with and/or reacts by imposing unique selectivity by introducing functionalities which interact noncovalently with target proteins.

In another embodiment, Y is selected from ATP analogs and inhibitors; peptides and peptide analogs; polyethyleneglycol (PEG); activated esters of amino acids, isolated or within a peptide; cytochrome C; and hydrophilic trityl groups.

In another embodiment, the compounds for use in the methods provided herein have the formulae: 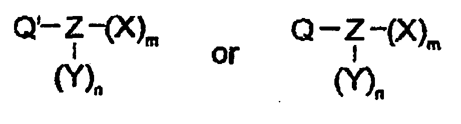 where Q, Q', Z, X and Y are as defined above; m is an integer from 1 to 100, in one embodiment 1 to 10, in another embodiment 1 to 3, 4 or 5; and n is an integer from 1 to 100, in one embodiment 1 to 10, in another embodiment 1 to 3, 4 or 5.

In another embodiment, X is a pharmaceutical drug. Such compounds may be used as drug transport molecules. The compounds of these embodiments may be used in drug screening by capturing biomolecules, including but not limited to proteins, which bind to the pharmaceutical drug. Mutations in the biomolecules interfering with binding to the pharmaceutical drug are identified, thereby determining possible mechanisms of drug resistance. See, *e.g.,* Hessler et al. (November 9-11, 2001) Ninth Foresight Conference on Molecular Nanotechnology(Abstract) (http://www.foresight.org/Conferences/MNT9/A bstracts/Hessler/).

In further embodiments, the compounds for use in the methods provided herein are those of the above formulae, where Z is an insoluble support or a substrate, including a bead, including, but not limited to, polymeric, magnetic, colored, R_{f}-tagged, etc. beads, that, in certain embodiments, is linked to X through a first optional spacer, and a cleavable linkage; and is linked to Q through a second optional spacer. In these embodiments, the density of the biopolymer to be analyzed, and thus signal intensity of the subsequent analysis, is increased relative to embodiments where Z is a divalent or multivalent group. In these embodiments, an appropriate array of single stranded oligonucleotides or oligonucleotide analogs that are complementary to Q will be employed in the methods provided herein.

In further embodiments herein, Z is as defined above, and is a non-cleavable linker. The compounds of these embodiments are generally useful in embodiments where cleavage of the biopolymer from the Q moiety is not required prior to or during analysis of the biopolymer, including analysis of biopolymer-biopolymer interactions, such as protein-protein interactions, or biopolymer small molecule (*e.g.,* drug or drug candidate) interactions, including protein-small molecule (*e.g.*, drug or drug candidate) interactions.

In all embodiments herein, the compounds for use in the methods herein may be classified in at least two sets: one for reactions in aqueous solution (*e.g.*, for reaction with hydrophilic biomolecules), and the other for reaction in organic solvents (*e.g.*, chloroform)(*e.g.*, for reaction with hydrophobic biomolecules). Thus, in certain embodiments, the compounds provided herein discriminate between hydrophilic and hydrophobic biomolecules, including, but not limited to, proteins, and allow for analysis of both classes of biomolecules.

The variables Y, X and Z, with reference to linkers and spacers, are described in further detail below. The below descriptions apply to all of the above formulae.

### 2. The moiety Z

For purposes herein, Z is a solid support. The following includes a description of linkers and spacers for linking X and Y moieties to Z.

### a. Z is a cleavable moiety

In certain embodiments herein, in the compounds for use in the methods provided herein, Z is a moiety that is cleavable prior to or during analysis of the biomolecule, including mass spectral analysis, without altering the chemical structure of the biomolecule, including, but not limited to, a protein. In one embodiment, the methods provided herein include methods of mass spectral analysis of biomolecules, including proteins, that are displayed in an addressable format. In certain embodiments, the format is an array of single stranded oligonucleotides that are complementary to the oligonucleotide portions, or oligonucleotide analog portions, (Q) of the compounds. In these embodiments, Z is a group that is (i) stable to the reaction conditions required for reaction of the compounds provided herein with the biomolecule, such as a protein, (ii) stable to the conditions required for hybridization of the Q moiety with the single stranded oligonucleotides, and (iii) cleavable prior to or during analysis of the biomolecule.

In one embodiment, Z is a photocleavable group that is cleaved by a laser used in MALDI-TOF mass spectrometry. In another embodiment, Z is an acid labile group that is cleaved upon application of a matrix to the hybridized compound-biomolecule conjugates, or by exposure to acids (*e.g.*, trifluoroacetic or hydrochloric acids) in a vapor or liquid form, prior to analysis. In this embodiment, the matrix maintains the spacial integrity of the array, allowing for addressable analysis of the array.

### b. Z is a non-cleavable moiety

In other embodiments herein, the compounds for use in the methods provided herein have a Z moiety that is not cleavable under conditions used for analysis of biomolecules, including, but not limited to, mass spectrometry, such as matrix assisted laser desorption ionization-time of flight (MALDI-TOF) mass spectrometry. The compounds of these embodiments are useful, *e.g.,* in methods provided herein for determining biomolecule-biomolecule, including protein-protein, interactions, and for determining biomolecule-small molecule, including protein-drug or protein-drug candidate, interactions. In these embodiments, it is not necessary for the Z group to be cleaved for the analysis.

### c. Divalent or multivalent Z moieties

In one embodiment, Z is a cleavable or non-cleavable divalent or multivalent group that contains less than 50, or less than 20 members, and is selected from straight or branched chain alkylene, straight or branched chain alkenylene, straight or branched chain alkynylene, straight or branched chain alkylenoxy, straight or branched chain alkylenthio, straight or branched chain alkylencarbonyl, straight or branched chain alkylenamino, cycloalkylene, cycloalkenylene, cycloalkynylene, cycloalkylenoxy, cycloalkylenthio, cycloalkylencarbonyl, cycloalkylenamino, heterocyclylene, arylene, arylenoxy, arylenthio, arylencarbonyl, arylenamino, heteroarylene, heteroarylenoxy, heteroarylenthio, heteroarylencarbonyl, heteroarylenamino, oxy, thio, carbonyl, carbonyloxy, ester, amino, amido, phosphino, phosphineoxido, phosphoramidato, phosphinamidato, sulfonamido, sulfonyl, sulfoxido, carbamato, ureido, and combinations thereof, and is optionally substituted with one or more, including one to four, substituents each independently selected from R¹⁵;
each R¹⁵ is independently a group that modifies the steric and/or electronic (*e.g.*, mesomeric, inductive effects) properties of Z; in one embodiment, R¹⁵ is a group that is a component of a luminescent, including fluorescent, phosphorescent, chemiluminescent and bioluminescent, system, or is a group that may be detected in a colorimetric assay.

Fluorescent, colorimetric and phosphorescent groups are well known to those of skill in the art (see, *e.g.,* U.S. Patent No. 6,274,337; Sapan et al. (1999) Biotechnol. Appl. Biochem. 29 (Pt. 2):99-108; Sittampalam et al. (1997) Curr. Opin. Chem. Biol. 1(3):384-91; Lakowicz, J. R., Principles of Fluorescence Spectroscopy, New York: Plenum Press (1983); Herman, B., Resonance Energy Transfer Microscopy, in: Fluorescence Microscopy of Living Cells in Culture, Part B, Methods in Cell Biology, vol. 30, ed. Taylor, D. L. & Wang, Y. -L., San Diego: Academic Press (1989), pp. 219-243; Turro, N. J., Modern Molecular Photochemistry, Menlo Park: Benjamin/Cummings Publishing Col, Inc. (1978), pp. 296-361 and the Molecular Probes Catalog (1997), OR, USA). Fluorescent moieties include, but are not limited to, 1- and 2-aminonaphthalene; p,p'-diaminostilbenes, pyrenes, quaternary phenanthridine salts, 9-aminoacridines, p,p'-diaminobenzophenone imines, anthracenes, oxacarbocyanine, merocyanine, 3-aminoequilenin, perylene, bis-benzoxazole, bis-p-oxazolyl benzene, 1,2-benzophenazin, retinol, bis-3-aminopyridinium salts, hellebrigenin, tetracycline, sterophenol, benzimidazolylphenylamine, 2-oxo-3-chromen, indole, xanthen, 7-hydroxycoumarin, phenoxazine, calicylate, strophanthidin, porphyrins, triarylmethanes and flavin. Fluorescent compounds which have functionalities for linking to a compound provided herein, or which can be modified to incorporate such functionalities include, *e.g.*, dansyl chloride; fluoresceins such as 3,6-dihydroxy-9-phenylxanthhydrol; rhodamineisothiocyanate; N-phenyl 1-amino-8-sulfonatonaphthalene; N-phenyl 2-amino-6-sulfonatonaphthalene; 4-acetamido-4-isothiocyanato-stilbene-2,2'-disulfonic acid; pyrene-3-sulfonic acid; 2-toluidinonaphthalene-6-sulfonate; N-phenyl-N-methyl-2-aminoaphthalene-6-sulfonate; ethidium bromide; stebrine; auromine-0,2-(9'-anthroyl)palmitate; dansyl phosphatidylethanolamine; N,N'-dioctadecyl oxacarbocyanine: N,N'-dihexyl oxacarbocyanine; merocyanine, 4-(3'pyrenyl)stearate; d-3-aminodesoxy-equilenin; 12-(9'-anthroyl)stearate; 2-methylanthracene; 9-vinylanthracene; 2,2'(vinylene-p-phenylene)bisbenzoxazole; p-bis(2-(4-methyl-5-phenyl-oxazolyl))benzene; 6-dimethylamino-1,2-benzophenazin; retinol; bis(3'-aminopyridinium) 1,10-decandiyl diiodide; sulfonaphthylhydrazone of hellibrienin; chlorotetracycline; N-(7-dimethylamino4-methyl-2-oxo-3-chromenyl)maleimide; N-(p-(2-benzimidazolyl)-phenyl)maleimide; N-(4-fluoranthyl)maleimide; bis(homovanillic acid); resazarin; 4-chloro-7-nitro-2,1,3-benzooxadiazole; merocyanine 540; resorufin; rose bengal; and 2,4-diphenyl-3(2H)-furanone. Many fluorescent tags are commercially available from SIGMA chemical company (Saint Louis, Mo.), Molecular Probes, R&D systems (Minneapolis, Minn.), Pharmacia LKB Biotechnology. (Piscataway, N.J.), CLONTECH Laboratories, Inc. (Palo Alto, Calif.), Chem Genes Corp., Aldrich Chemical Company (Milwaukee, Wis.), Glen Research, Inc., GIBCO BRL Life Technologies, Inc. (Gaithersberg, Md.), Fluka Chemica-Biochemika Analytika (Fluka Chemie AG, Buchs, Switzerland), and Applied Biosystems (Foster City, Calif.) as well as other commercial sources known to one of skill in the art.

Chemiluminescent groups intended for use herein include any components of light generating systems that are catalyzed by a peroxidase and require superoxide anion (O_{2̅}) (and/or hydrogen peroxide (H₂O₂))(see, *e.g.,* Musiani et al. (1998) Histol. Histopathol. 13(1):243-8). Light-generating systems include, but are not limited to, luminol, isoluminol, peroxyoxalate-fluorophore, acridinium ester, lucigenin, dioxetanes, oxalate esters, acridan, hemin, indoxyl esters including 3-O-indoxyl esters, naphthalene derivatives, such as 7-dimethylaminonaphthatene-1,2-dicarbonic acid hydrazide and cypridina luciferin analogs, including 2-methyl-6-[p-methoxyphenyl]-3,7-dihyroimidazo[1,2-*α*]pyrazin-3-one, 2-methyl-6-phenyl-3,7-dihyroimidazo[1,2-α]pyrazin-3-one and 2-methyl-6-[p-[2-[sodium 3-carboxylato-4-(6-hydroxy-3-xanthenon-9-yl]phenylthioureylene]ethyleneoxy]phenyl]-3,7-dihyroimidazo[1,2-α]pyrazin-3-one. In other embodiments, the chemiluminescent moieties intended for use herein include, but are not limited to, luminol, isoluminol, N-(4-aminobutyl)-N-ethyl isoluminol (ABEI), N-(4-aminobutyl)-N-methyl isoluminol (ABMI), which have the following structures and participate in the following reactions: 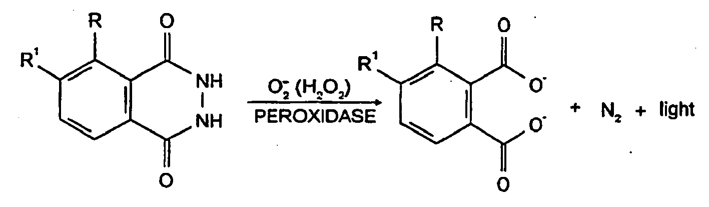 in which luminol is represented, when R is NH₂ and R¹ is H; isoluminol, when R is H and R¹ is NH₂; for ABEI ((6-[N-(4-aminobutyl)-N-ethylamino]-2,3-dihyrophthalazine-1-4-dione), when R is H and R¹ is C₂H₅-N-(CH₂)₄NH₂; and for ABMI ((6-[N-(4-aminobutyl)-N-methylamino]-2,3-dihyrophthalazine-1-4-dione), when R is H and R¹ is CH₃-N-(CH₂)₄NH₂.

Bioluminescent groups for use herein include luciferase/luciferin couples, including firefly [*Photinus pyralis*] luciferase, the *Aequorin* system (*i.e.,* the purified jellyfish photoprotein, aequorin). Many luciferases and substrates have been studied and well-characterized and are commercially available (*e.g.*, firefly luciferase is available from Sigma, St. Louis, MO, and Boehringer Mannheim Biochemicals, Indianapolis, IN; recombinantly produced firefly luciferase and other reagents based on this gene or for use with this protein are available from Promega Corporation, Madison, WI; the aequorin photoprotein luciferase from jellyfish and luciferase from *Renilla* are commercially available from Sealite Sciences, Bogart, GA; coelenterazine, the naturally-occurring substrate for these luciferases, is available from Molecular Probes, Eugene, OR]. Other bioluminescent systems include crustacean, particularly *Cyrpidina* (*Vargula*), systems; insect bioluminescence generating systems including fireflies, click beetles, and other insect systems; bacterial systems; dinoflagellate bioluminescence generating systems; systems from molluscs, such as *Latia* and *Pholas;* earthworms and other annelids; glow worms; marine polycheate worm systems; South American railway beetle; fish (*i.e.,* those found in species of *Aristostomias,* such as *A. scintillans* (see, *e.g.,* O'Day et al. (1974) Vision Res. 14:545-550), *Pachystomias,* and *Malacosteus,* such as *M. niger;* blue/green emmitters include cyclthone, myctophids, hatchet fish (agyropelecus), vinciguerria, howella, florenciella, and Chauliodus); and fluorescent proteins, including green (*i.e.*, GFPs, including those from *Renilla* and from *Ptilosarcus*), red and blue (*i.e.,* BFPs, including those from *Vibrio fischeri, Vibrio harveyi* or *Photobacterium phosphoreum*) fluorescent proteins (including *Renilla mulleri* luciferase, *Gaussia* species luciferase and *Pleuromamma* species luciferase) and phycobiliproteins.

### i. Cleavable divalent or multivalent Z moieties

In one embodiment, Z is a cleavable divalent or multivalent moiety and has the formula:

-(S¹)ₜ-M(R¹⁵)ₐ-(S²)_{b}-L-

in which S¹ and S² are spacer moieties; t and b are each independently 0 or 1; M is a central moiety possessing two or more points of attachment (*i.e.,* divalent or higher valency); in certain embodiments, two to six points of attachment (*i.e.,* divalent to hexavalent), in other embodiments, 2, 3, 4 or 5 points of attachment (*i.e.,* divalent, trivalent, tetravalent or pentavalent); R¹⁵ is a functional group that modifies the steric and/or electronic (*e.g.*, mesomeric, inductive effects) properties of M; a is O to 4, in certain embodiments, 0, 1 or 2; and L is a bond that is cleavable prior to or during analysis, including mass spectral analysis, of a biomolecule without altering the chemical structure of the biomolecule, such as a protein.

A spacer region S¹ and/or S² can be present on either or both sides of the central moiety M of the compounds, thereby reducing steric hindrance in reactions with the surface of large protein molecules (S²) or facilitating hybridization with complementary sequences (S¹). For embodiments wherein the protein and the complementary sequence possess low steric hinderance, a spacer may not be required. In certain embodiments, steric hindrance can enhance selectivity. This enhanced selectivity can be achieved either by the presence of one or more bulky substituents R¹⁵ which is attached to M or by the selection of the appropriate spacer molecules for S¹ and/or S².

In certain embodiments, S¹ and S² are each independently selected from -(CH₂)ᵣ-, -(CH₂O)-, -(CH₂CH₂-O)ᵣ-,-(NH-(CH₂)ᵣ-C(=O))ₛ-, -(NH-CH(R⁵²)-C(=O))ₛ-, -(O-(CH)ᵣ-C(=O))ₛ-, 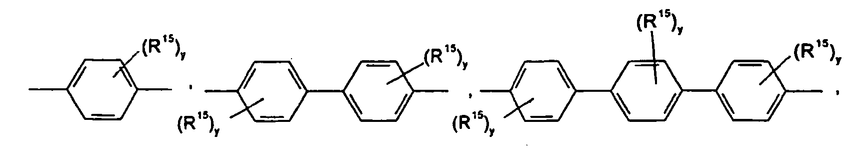 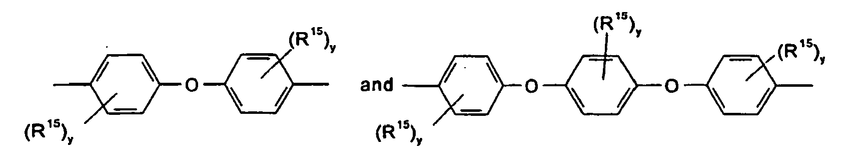 where R¹⁵ is as above; r and s are each independently an integer from 1 to 1 O; R⁵² is the side chain of a natural α-amino acid; and y is an integer from 0 to 4. In one embodiment, y is 0 or 1.

In certain embodiments, R¹⁵ is -H, -OH, -OR⁵¹, -SH, -SR⁵¹, -NH₂, -NHR⁵¹, -NR⁵¹₂, -F, -Cl, -Br, -I, -SO₃H, -PO⁻²₄, -CH₃, -CH₂CH₃, -CH(CH₃)₂ or -C(CH₃)₃; where R⁵¹ is straight or branched chain alkyl, straight or branched chain alkenyl, straight or branched chain alkynyl, aryl, heteroaryl, cycloalkyl, heterocyclyl, straight or branched chain aralkyl, straight or branched chain aralkenyl, straight or branched chain aralkynyl, straight or branched chain heteroaralkyl, straight or branched chain heteroaralkenyl, straight or branched chain heteroaralkynyl, straight or branched chain cycloalkylalkyl, straight or branched chain cycloalkylalkenyl, straight or branched chain cycloalkylalkynyl, straight or branched chain heterocyclylalkyl, straight or branched chain heterocyclylalkenyl or straight or branched chain heterocyclylalkynyl.

In certain embodiments, the cleavable group L is cleaved either prior to or during analysis of the biomolecule, such as a protein. The analysis may include mass spectral analysis, for example MALDI-TOF mass spectral analysis. The cleavable group L is selected so that the group is stable during conjugation to a biomolecule, hybridization of the Q moiety to a complementary sequence, and washing of the hybrid; but is susceptable to cleavage under conditions of analysis of the biomolecule, including, but not limited to, mass spectral analysis, for example MALDI-TOF mass spectral analysis. In certain embodiments, the cleavable group L can be a disulfide moiety, created by reaction of the compounds where X = -SH, with the thiol side chain of cysteine residues on the surface of biomolecules, including, but not limited to, proteins. The resulting disulfide bond can be cleaved under various reducing conditions including, but not limited to, treatment with dithiothreitol and 2-mercaptoethanol.

In another embodiment, L is a photocleavable group, which can be cleaved by a short treatment with UV light of the appropriate wave length either prior to or during mass spectrometry. Photocleavable groups, including those bonds which can be cleaved during MALDI-TOF mass spectrometry by the action of a laser beam, may be used. For example, a trityl ether or an ortho nitro substituted aralkyl, including benzyl, group are susceptible to laser induced bond cleavage during MALDI-TOF mass spectrometry. Other useful photocleavable groups include, but are not limited to, o-nitrobenzyl, phenacyl, and nitrophenylsulfenyl groups.

Other photocleavable groups for use herein include those disclosed in International Patent Application Publication No. WO 98/20166. In one embodiment, the photocleavable groups have formula I: 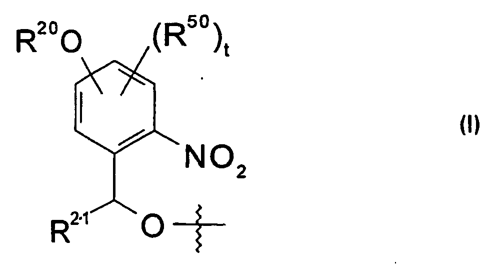 where R²⁰ is ω-O-alkylene-; R²¹ is selected from hydrogen, alkyl, aryl, alkoxycarbonyl, aryloxycarbonyl and carboxy; t is 0-3; and R⁵⁰ is alkyl, alkoxy, aryl or aryloxy. In one embodiment, Q is attached to R²⁰ through (S¹)ₜ-M(R¹⁵)ₐ-(S²)_{b}; and the biomolecule of interest is captured onto the R²¹ CH-O- moiety via a reactive derivative of the oxygen (*e.g.* , X).

In another embodiment, the photocleavable groups have formula II: 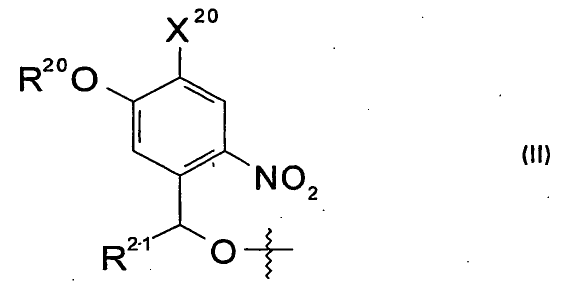 where R²⁰ is ω-O-alkylene- or alkylene; R²¹ is selected from hydrogen, alkyl, aryl, alkoxycarbonyl, aryloxycarbonyl and carboxy; and X²⁰ is hydrogen, alkyl or OR²¹. In one embodiment, Q is attached to R²⁰ through (S¹)ₜ-M(R¹⁵)ₐ-(S²)_{b}; and the biomolecule of interest is captured onto the R²¹CH-O- moiety via a reactive derivative of the oxygen (*e.g.*, X).

In further embodiments, R²⁰ is -O-(CH₂)₃- or methylene; R²¹ is selected from hydrogen, methyl and carboxy; and X²⁰ is hydrogen, methyl or OR²¹. In another embodiment, R²¹ is methyl; and X²⁰ is hydrogen. In certain embodiments, R²⁰ is methylene; R²¹ is methyl; and X²⁰ is 3-(4,4'-dimethoxytrityloxy)propoxy.

In another embodiment, the photocleavable groups have formula III: 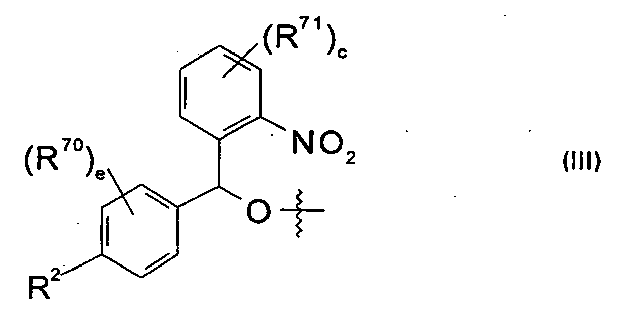 where R² is selected from ω-O-alkylene-O and ω-O-alkylene-, and is unsubstituted or substituted on the alkylene chain with one or more alkyl groups; c and e are each independently 0-4; and R⁷⁰ and R⁷¹ are each independently alkyl, alkoxy, aryl or aryloxy. In certain embodiments, R² is ω-O-alkylene-, and is substituted on the alkylene chain with a methyl group. In one embodiment, Q is attached to R² through (S¹)ₜ-M(R¹⁵)ₐ-(S²)_{b}; and the biomolecule of interest is captured onto the Ar₂CH-O- moiety via a reactive derivative of the oxygen (*e.g.*, X).

In further embodiments, R² is selected from 3-O-(CH₂)₃-O-, 4-O-(CH₂)₄-, 3-O-(CH₂)₃-, 2-O-CH₂CH₂-, -OCH₂-,

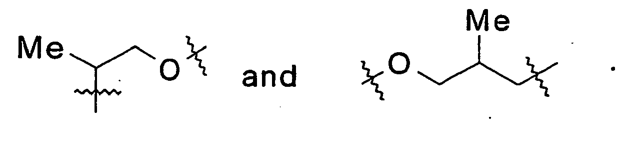

In other embodiments, c and e are 0.

Other cleavable groups L include acid sensitive groups, in which bond cleavage is promoted by formation of a cation upon exposure to mild to strong acids. For these acid-labile groups, cleavage of the group L can be effected either prior to or during analysis, including mass spectrometric analysis, by the acidity of the matrix molecules, which are deposited as a thin layer on top of the hybrids or by applying a short treatment of the array with an acid, such as the vapor of trifluoroacetic acid. Exposure of a trityl group to acetic or trifluoroacetic acid produces cleavage of the ether bond either before or during MALDI-TOF mass spectrometry.

The compound-biomolecule array can be treated by either chemical, including, but not limited to, cyanogen bromide, or enzymatic, including, but not limited to, trypsin, chymotrypsin, an exopeptidase (*e.g.*, aminopeptidases and carboxypeptidases) reagents to effect cleavage. For the latter, all but one peptide fragment will remain hybridized when digestion is quantitative. Partial digestion may also be of advantage to identify and characterize proteins following desorption from the array. The cleaved protein/peptide fragments are desorbed, analyzed, and characterized by their respective molecular weights.

In certain embodiments herein, L is selected from -S-S-, -O-P(=O)(OR⁵¹)-NH-, -O-C(=O)-, 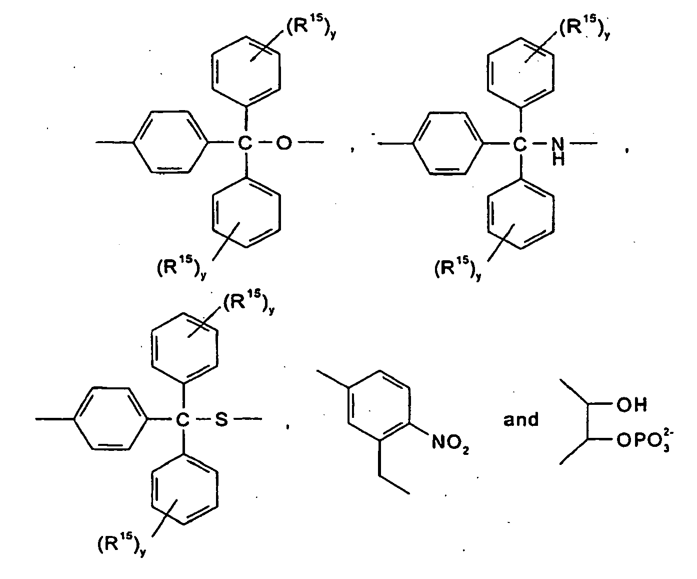 where R¹⁵, R⁵¹ and y are as defined above. In certain embodiments, R¹⁵ is -H, -OH, -OR⁵¹, -SH, -SR⁵¹, -NH₂, -NHR⁵¹, -N(R⁵¹)₂, -F, -Cl, -Br, -I, -SO₃H, -PO⁻²₄, -CH₃, -CH₂CH₃, -CH(CH₃)₂ or -C(CH₃)₃; where R⁵¹ is straight or branched chain alkyl, straight or branched chain alkenyl, straight or branched chain alkynyl, aryl, heteroaryl, cycloalkyl, heterocyclyl, straight or branched chain aralkyl, straight or branched chain aralkenyl, straight or branched chain aralkynyl, straight or branched chain heteroaralkyl, straight or branched chain heteroaralkenyl, straight or branched chain heteroaralkynyl, straight or branched chain cycloalkylalkyl, straight or branched chain cycloalkylalkenyl, straight or branched chain cycloalkylalkynyl, straight or branched chain heterocyclylalkyl, straight or branched chain heterocyclylalkenyl or straight or branched chain heterocyclylalkynyl.

### ii. Non-cleavable divalent Z moieties

In another embodiment, Z is a non-cleavable divalent moiety and has the formula:

-(S¹)ₜ-M(R¹⁵)ₐ-(S²)_{b}-

in which S¹, M, R¹⁵, S², t, a and b are as defined above.

### e. Z is an insoluble support or a substrate

For the gradient arrays provided herein, Z may be the insoluble support or substrate. In these embodiments, Z is linked to a plurality of X moieties or a plurality of Y and X moieties or mixtures of X, Q and/or Y and other moieties. Z, in certain embodiments, has tens up to hundreds, thousands, millions, or more points of attachment. In these embodiments, the insoluble support or substrate moiety Z is based on a flat surface constructed, for example, of glass, silicon, metal, plastic or a composite; or can be in the form of a bead such as a silica gel, a controlled pore glass, a magnetic or cellulose bead; or can be a pin, including an array of pins suitable for combinatorial synthesis or analysis. Substrates can be fabricated from virtually any insoluble or solid material. For example, silica gel, glass (*e.g.*, controlled-pore glass (CPG)), nylon, Wang resin, Merrifield resin, dextran cross-linked with epichlorohydrin (e.g., Sephadex^{R}), agarose (e.g., Sepharose^{R}), cellulose, magnetic beads, Dynabeads, a metal surface (*e.g.*, steel, gold, silver, aluminum, silicon and copper), a plastic material (*e.g.*, polyethylene, polypropylene, polyamide, polyester, polyvinylidenedifluoride (PVDF)) Exemplary substrates include, but are not limited to, beads (*e.g.*, silica gel, controlled pore glass, magnetic, dextran cross-linked with epichlorohydrin (e.g., Sephadex^{R}), agarose (e.g., Sepharose^{R}), cellulose), capillaries, flat supports such as glass fiber filters, glass surfaces, metal surfaces (steel, gold, silver, aluminum, copper and silicon), plastic materials including multiwell plates or membranes (*e.g.*, of polyethylene, polypropylene, polyamide, polyvinylidenedifluoride), pins (*e.g.*, arrays of pins suitable for combinatorial synthesis or analysis or beads in pits of flat surfaces such as wafers (*e.g.*, silicon wafers) with or without plates. The solid support is in any desired form, including, but not limited to, a bead, capillary, plate, membrane, wafer, comb, pin, a wafer with pits, an array of pits or nanoliter wells and other geometries and forms known to those of skill in the art. Supports include flat surfaces designed to receive or link samples at discrete loci. In one embodiment, flat surfaces include those with hydrophobic regions surrounding hydrophilic loci for receiving, containing or binding a sample.

In one embodiment, the solid supports or substrates Z are beads, including, but not limited to, polymeric, magnetic, colored, R_{f}-tagged, etc. beads. The beads may be made from hydrophobic materials, including, but not limited to, polystyrene, polyethylene, polypropylene or teflon, or hydrophilic materials, including, but not limited to, cellulose, dextran cross-linked with epichlorohydrin (e.g., Sephadex^{R}), agarose (e.g., Sepharose^{R}), polyacrylamide, silica gel and controlled pore glass.

In further embodiments, the insoluble support or substrate Z moieties may optionally possess spacer groups S¹ and/or S², or for embodiments where Z is a cleavable linkage, L. The S¹, S² and/or L moieties are attached to the surface of the insoluble support or substrate.

In these embodiments, the density of the biopolymer to be analyzed, and thus signal intensity of the subsequent analysis, is increased relative to embodiments where Z is a divalent or multivalent group. In these embodiments, an appropriate array of single stranded oligonucleotides or oligonucleotide analogs that are complementary to Q will be employed in the methods provided herein.

In another embodiment, Z is a support that, when functionalized with Q, X and Y, mimics the function of an artificial membrane by capturing biomolecules, including but not limited to, proteins. In one embodiment, Z is a support that, when functionalized with Q, X and Y, mimics the specificity of the inside of a cell membrane. In this embodiment, the support is able to capture proteins and other biomolecules that the compounds provided herein would otherwise not be able to capture, including proteins within cell membranes. In one embodiment, the supports function under physiological conditions. Thus, in the above embodiments, choice of Z, Q, X and Y allows for design of surfaces and supports that mimic cell membranes and other biological membranes. When the compounds provided herein act as an artificial membrane, dendrimer polymer chemistry may be employed for controlled synthesis of membranes having consistent pore dimensions and membrane thicknesses, through synthesis of amphiphilic dedndrimeric or hyperbranched block copolymers that can be self-assembled to form ultrathin organic film membranes on porous supports.

### Gradient arrays

Provided herein are two dimensional gradient arrays. In these embodiments, Z is a solid support and X, and Y moieties (separately or linked to Z) are arrayed thereon such that the collection of loci presents a gradient of selected properties. At each locus in the array X and Y moieties are presented, such as side-by-side or bound, as along each row (*i.e.,* X axis), each Y moiety is the same and a selected property of X, such as hydrophobicity, lipophilisity, charge, size, specificity or other property of each X is altered in a predetermined manner along the X-axis to provide a gradient of such property. Similarly, along each column of loci in the array, a property of Y, such as hydrophobicity, charge, size, specificity or other property of each Y is altered in a predetermined manner along the Y-axis to provide a gradient of such property. For each array, the property of X moieties that is altered is not the same as the property of the Y moieties that is altered. The resulting array thereby presents a two dimensional gradient of two (or more) selected properties and presents loci with different affinities. The number of loci in the array can be any number, such as 10, 50, 140, 500, 1000, 10⁴ and more or any predetermined number.

Such solid supports can be any solid support, such as those provided herein, and include beads, flat squares and rectangles and other geometries, and can be any size desired, such a 1 µm, 10 µm, 50 µm, 100 µm, 1 mm, 1 cm, 10 cm and larger, where the dimension refers to the largest dimension, and for purposes herein is of a size convenient to perform mass spectrometric analyses, particularly MALDI analyses. In such arrays, the X and Y moieties can be provided such that they present a gradient of selected properties.

In practice, the resulting arrays are analyzed as described herein. For example, the resulting arrays are contacted with a sample, such as a sample containing proteins, to be analyzed and, matrix is added and the resulting array analyzed by mass spectrometry. Exemplary gradient arrays are set forth below.

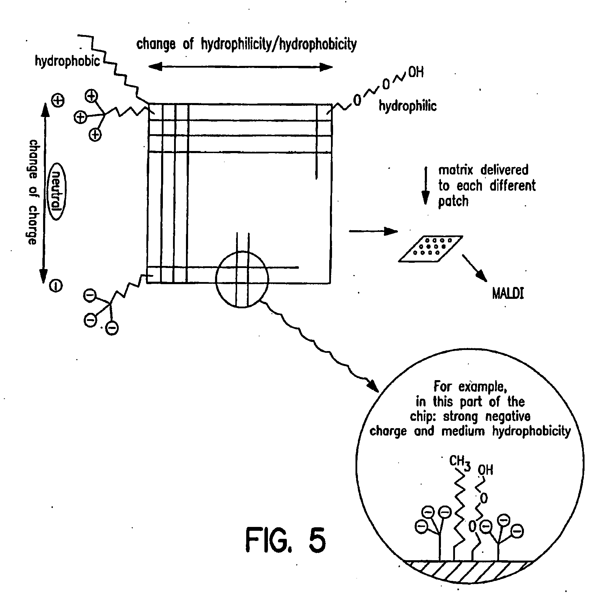

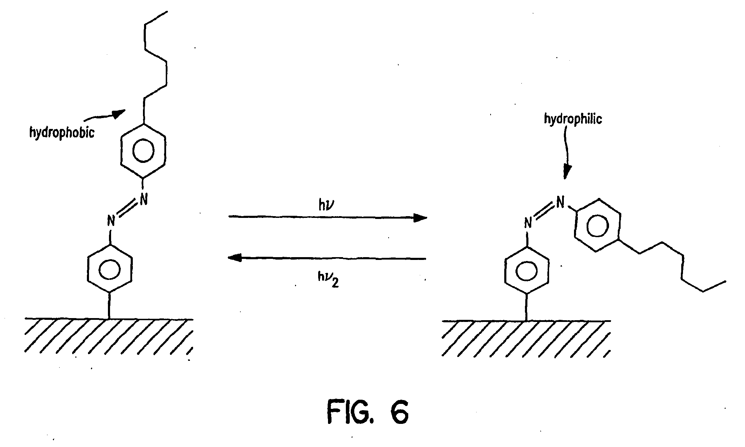

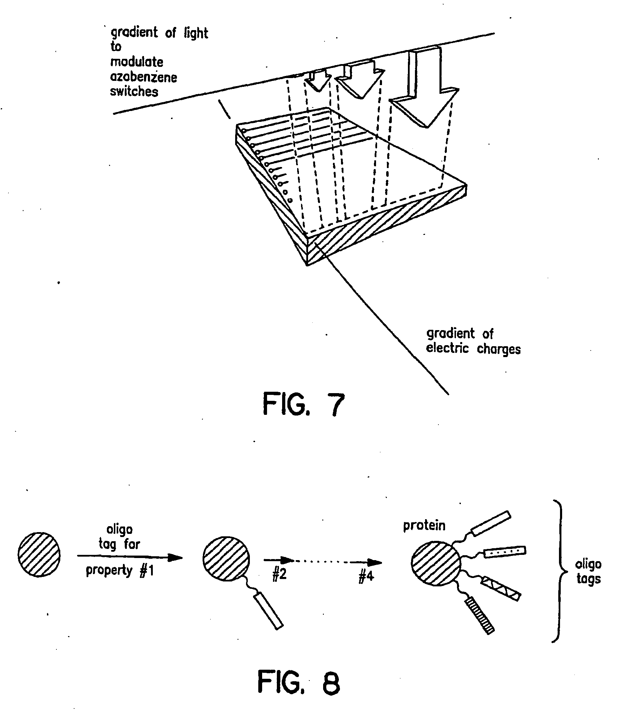

Figure 5 shows a continuous support with gradients or lyotropic monolayers where the molecules are increasingly hydrophobic/hydrophilic, charged (negative or positive), increasing/decreasing in size. Other properties well known to those of skill in the art may also be used, such as chemical properties, including reagents specificity for NH₂, SH, SS, OH, etc. groups. In one embodiment, the gradient along the X axis differs from the Y axis, *e.g.,* X = increasing hydrophobicity and Y = increasing positive charge. In one embodiment, azobenzene switches (see, *e.g.*, Figure 6) are used wherein hydrophilicity is introduced as the azobenzene group is exposed to light. A gradient of hydrophobicity/hydrophilicity is formed as the light exposure increases. Charge may be introduced using electrodes which generate a gradient of electronic charges. See, *e.g.,* Figure 7. Alternatively, Along each direction (X and Y), there will be many patches with different affinities. The mixture of proteins is applied to the surface of the substrate, whereby the proteins arrange according to their unique affinities. The matrix is then spotted atop of each area and analyzed by, *e.g.*, MALDI-TOF.

### f. Mass modified Z moieties

In further embodiments, including embodiments where Z includes a cleavable moiety, Z can include a mass modifying tag. In certain embodiments, the mass modifying tag is attached to the cleavable linker L. In one embodiment, the mass modified Z moiety has the formula:

-(S¹)ₜ-M(R¹⁵)ₐ-(S²)_{b}-L-T-

in which S¹, t, M, R¹⁵, a, S², b and L are selected as above; and T is a mass modifying tag. Mass modifying tags for use herein include, but are not limited to, groups of formula -X¹R¹⁰-, where X¹ is a divalent group such as -O-, -O-C(O)-(CH₂)_{y}-C(O)O-, -NH-C(O)-, -C(O)-NH-, -NH-C(O)-(CH₂)_{y}-C(O)O-, -NH-C(S)-NH-, -O-P(O-alkyl)-O-, -O-SO₂-O-, -O-C(O)-CH₂-S-, -S-, -NH- and 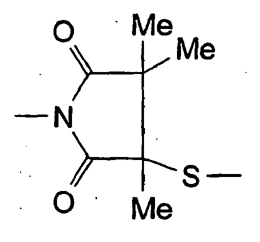 and R¹⁰ is a divalent group including -(CH₂CH₂O)_{z}-CH₂CH₂O-, -(CH₂CH₂O)_{z}-CH₂CH₂O-alkylene, alkylene, alkenylene, alkynylene, arylene, heteroarylene, -(CH₂)_{z}-CH₂-O-, -(CH₂)_{z}-CH₂-O-alkylene, -(CH₂CH₂NH)_{z}-CH₂CH₂NH-, -CH₂-CH(OH)-CH₂O-, -Si(R¹²)(R¹³)-, -CHF- and -CF₂-; where y is an integer from 1 to 20; z is an integer from 0 to 200; R¹¹ is the side chain of an α-amino acid; and R¹² and R¹² are each independently selected from alkyl, aryl and aralkyl.

In other embodiments, -X¹R¹⁰- is selected from -S-S-, -S-,
-(NH-(CH₂)_{y}-NH-C(O)-(CH₂)_{y}-C(O))_{z}-NH-(CH₂)_{y}-NH-C(O)-(CH₂)_{y}-C(O)O-,
-(NH-(CH₂)_{y}-C(O))_{z}-NH-(CH₂)_{y}-C(O)O-,
-(NH-CH(R¹¹)-C(O))_{z}-NH-CH(R¹¹)-C(O)O-, and
-(O-(CH₂)_{y}-C(O))_{z}-NH-(CH₂)_{y}-C(O)O-.

In the above embodiments, where R¹⁰ is an oligo-/polyethylene glycol derivative, the mass-modifying increment is 44, *i.e.,* five different mass-modified species can be generated by changing z from O to 4, thus adding mass units of 45 (z = O), 89 (z = 1), 133 (z = 2), 177 (z = 3) and 221 (z = 4) to the compounds. The oligo/polyethylene glycols can also be monoalkylated by a lower alkyl such as methyl, ethyl, propyl, isopropyl, t-butyl and the like.

Other mass modifying tags include, but are not limited to -CHF-, -CF₂-, -Si(CH₃)₂-, -Si(CH₃)(C₂H₅)- and -Si(C₂H₅)₂. In other embodiments, the mass modifying tags include homo- or heteropeptides. A non-limiting example that generates mass-modified species with a mass increment of 57 is an oligoglycine, which produce mass modifications of, *e.g.,* 74 (y = 1, z = O), 131 (y = 1, z = 2), 188 (y = 1, z = 3) or 245 (y = 1, z = 4). Oligoamides also can be used, *e.g.,* mass-modifications of 74 (y = 1, z = 0), 88 (y = 2, z = 0), 102 (y = 3, z = 0), 116 (y = 4, z = 0), etc., are obtainable. Those skilled in the art will appreciate that there are numerous possibilities in addition to those exemplefied herein for introducing, in a predetermined manner, many different mass modifying tags to the compounds provided herein.

In other embodiments, R¹⁵ and/or S² may be functionalized with -X¹R¹⁰H or -X¹R¹⁰-alkyl, where X¹ and R¹⁰ are defined as above, to serve as mass modifying tags.

### 3. The moiety X

In the compounds provided herein, X is a moiety that binds an amino acid side chain of a protein. Thus, in certain embodiments X is a group which can react or interact with functionalities on the surface of a protein to form covalent or non-covalent bonds. A wide selection of different functional groups are available for X to interact with a protein. For example, X can act either as a nucleophile or an electrophile to form covalent bonds upon reaction with the amino acid residues on the surface of a protein. Reagents that bind to amino acid side chains include, but are not limited to, protecting groups for hydroxyl, carboxyl, amino, amide, and thiol moieties, including those disclosed in T.W. Greene and P.G.M. Wuts, "Protective Groups in Organic Synthesis," 3rd ed. (1999, Wiley Interscience). These protecting groups can react with amino acid side chains such as hydroxyl (serine, threonine, tyrosine); amino (lysine, arginine, histadine, proline); amide (glutamine, asparagine); carboxylic acid (aspartic acid, glutamic acid); and sulfur derivatives (cysteine, methionine), and are readily adaptable for use in these compounds as the reactive moiety X.

It is noteworthy that in addition to the wide range of group-specific reagents which are known to persons of skill in the art, reagents which are known in natural product chemistry can also serve as a basis for X in forming covalent linkages.

Alternatively described herein, X is a protein purification dye, such as acridine or methylene blue, that has a strong affinity for certain proteins.

X can act as an electron donor or an electron acceptor to form non-covalent bonds or a complex, such as a charge-transfer complex, with a biomolecule, including, but not limited to, a protein. These reagents include those which interact strongly and with high specificity with biomolecules, including, but not limited to, proteins, without forming covalent bonds through the interaction of complementary affinity surfaces. For example, well known complexes such as biotinstreptavidin, antibody-antigen, receptor-ligand, lectin-carbohydrate and other similar types of reagents, are described.

Further, these complexes have strong interactions which are stable enough to allow for a suitable washing of the unbound biomolecules, including, but not limited to, proteins, out of the complexed biological mixtures.

If S² is not present, the reactivity of X can be influenced by one or more substituted functionalities, for example, R¹⁵ on M. Electronic (*e.g.*, mesomeric, inductive) and/or steric effects can be used to modulate the reactivity of X and the stability of the resulting X-biomolecule linkage. In these embodiments, subsets of biomolecular mixtures, including, but not limited to, protein mixtures, can react and be analyzed due to the modulation by R¹⁵, which changes the electronic or steric properties of X and, therefore, increases the selectivity of the reaction of X with the biomolecule.

In these embodiments, X is an active ester, such as -C(= O)O-Ph-pNO₂, -C(= O)O-C₆F₅ or -C(= O)-O-(N-succinimidyl); an active halo moiety, such as an α-halo ether or an α-halo carbonyl group, including, but not limited to, -OCH₂-I, -OCH₂-Br, -OCH₂-Cl, -C(O)CH₂I, -C(O)CH₂Br and -C(O)CH₂Cl; amino acid side chain-specific functional groups, such as maleimido (for cysteine), a metal complex, including gold or mercury complexes (for cysteine or methionine), an expoxide or isothiocyanate (for arginine or lysine); reagents which bind to active sites of enzymes, including, but not limited to, transition state analogs: ligands which bind to receptors, such as insulin; specific peptides which bind to biomolecule surfaces, including glue peptides; lectins (*e.g.*, mannose type, lactose type); antibodies, *e.g.*, against phosphorylated peptides; antigens, such as a phage display library; haptens; biotin; avidin; or streptavidin. Other embodiments of X are well known to those of skill in the art and include those disclosed in Techniques in Protein Chemistry, Vol. 1 (1989) T. Hugli ed. (Academic Press); Techniques in Protein Chemistry, Vol. 5 (1994) J.W. Crabb ed. (Academic Press); Lundblad Techniques in Protein Modification (1995) (CRC Press, Boca Raton, FL); Glazer et al. (1976) Chemical Modification of Proteins (North Holland (Amsterdam))(American Elsevier, New York); and Hermanson (1996) Bioconjugate Techniques (Academic Press, San Diego, CA).

### 4. Further embodiments

In certain embodiments, the compounds provided herein have the formula:

N¹ₘ-Bᵢ-N²ₙ-(S¹)ₜ-M(R¹⁵)ₐ-(S²)_{b}-L-X

in which N¹, B, N², S¹, M, S², L, X, m, i, n, t, a and b are as defined above. In further embodiments, the compounds for use in the methods provided herein include a mass modifying tag and have the formula:

N¹ₘ-Bᵢ-N²ₙ-(S¹)ₜ-M(R¹⁵)ₐ-(S²)_{b}-L-T-X

in which N¹, B, N², S¹, M, S², L, T, X, m, i, n, t, a and b are as defined above.

In other embodiments, including those where Z is not a cleavable linker, the compounds provided herein have the formula:

N¹ₘ-Bᵢ-N²ₙ-(S¹)ₜ-M(R¹⁵)ₐ-(S²)_{b}-X

in which N¹, B, N², S¹, M, S², X, m, i, n, t, a and b are as defined above.

### D. Preparation of the Compounds

The preparation of the compounds is described below. Any compound or similar compound may be synthesized according to a method discussed in general below or by only minor modification of the methods by selecting appropriate starting materials.

On general, the compounds may be prepared starting with the central moiety Z. In certain embodiments, Z is (S¹)ₜ-M(R¹⁵)ₐ-(S²)_{b}-L-. In these embodiments, the compounds may be prepared starting with an appropriately substituted (*e.g.*, with one or more R¹⁵ groups) M group. M(R¹⁵)ₐ is optionally linked with S¹ and/or S², followed by linkage to the cleavable linker L. Alternatively, the L group is optionally linked to S², followed by reaction with M(R¹⁵)ₐ, and optionally S¹. This Z group is then derivatized on its S¹ (or M(R¹⁵)ₐ) terminus to have a functionality for coupling with an oligonucleotide or oligonucleotide analog Q (*e.g.*, a phosphoramidite, H-phosphonate, or phosphoric triester group). The Q group will generally be N-protected on the bases to avoid competing reactions upon introduction of the X moiety. In one embodiment, the Z group is reacted with a mixture of all possible permutations of an oligonucleotide or oligonucleotide Q (*e.g.*, 4ⁱ permutations where i is the number of nucleotides or nucleotide analogs in B). The resulting Q-Z compound or compounds is(are) then derivatized through the L terminus to possess an X group for reaction with a biomolecule, such as a protein. If desired, the N-protecting groups on the Q moiety are then removed. Alternatively, the N-protecting groups may be removed following reaction of the compound with a biomolecule, including a protein. In other embodiments, Q can be synthesized on Z, including embodiments an insoluble support or substrate, such as a bead. In a further embodiment, Q is presynthesized by standard solid state techniques, then linked to M. Alternatively, Q may be synthesized stepwise on the M moiety.

Provided below are examples of syntheses of the compounds provided herein containing alkaline-labile and photocleavable linkers. One of ordinary skill in the art would be able to prepare other compounds within the scope of this disclosure by routine modification of the methods presented below, or by other methods well known to those of skill in the art.

For synthesis of a compound provided herein containing an alkaline-labile linker, 1,4-di(hydroxymethyl)benzene (*i.e.,* M) is mono-protected, *e.g.,* as the corresponding mono-tert-butyldimethylsilyl ether. The remaining free alcohol is derivatized as the corresponding 2-cyano-ethyl-*N,N*-diisopropylphosphoramidite by reaction with 2-cyanoethyl-*N,N-*diisopropylchlorophosphoramidite. Reaction of this amidite with an oligonucleotide, (*i.e.*, Q), is followed by removal of the protecting group to provide the corresponding alcohol. Reaction with, *e.g.*, trichloromethyl chloroformate affords the illustrated chloroformate *(i.e.,* X).

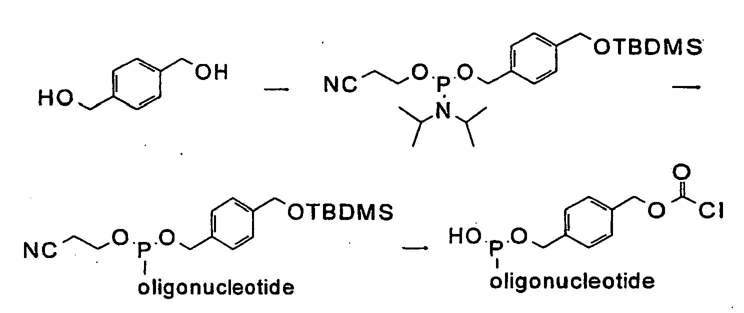

For the synthesis of a compound provided herein containing a photocleavable linker, 2-nitro-5-hydroxybenzaldehyde *(i.e.,* a precursor of L) is reacted with, *e.g.*, 3-bromo-1-propanol to give the corresponding ether-alcohol. The alcohol is then protected, *e.g.*, as the corresponding *tert*-butyldimethylsilyl ether. Reaction of this compound with trimethylaluminum gives the corresponding benzyl alcohol, which is derivatized as its phosphoramidite using the procedure described above. The amidite is reacted with an oligonucleotide *(i.e.,* Q), followed by removal of the protecting group and derivatization of the resulting alcohol as the corresponding chloroformate (*i.e.,* X).

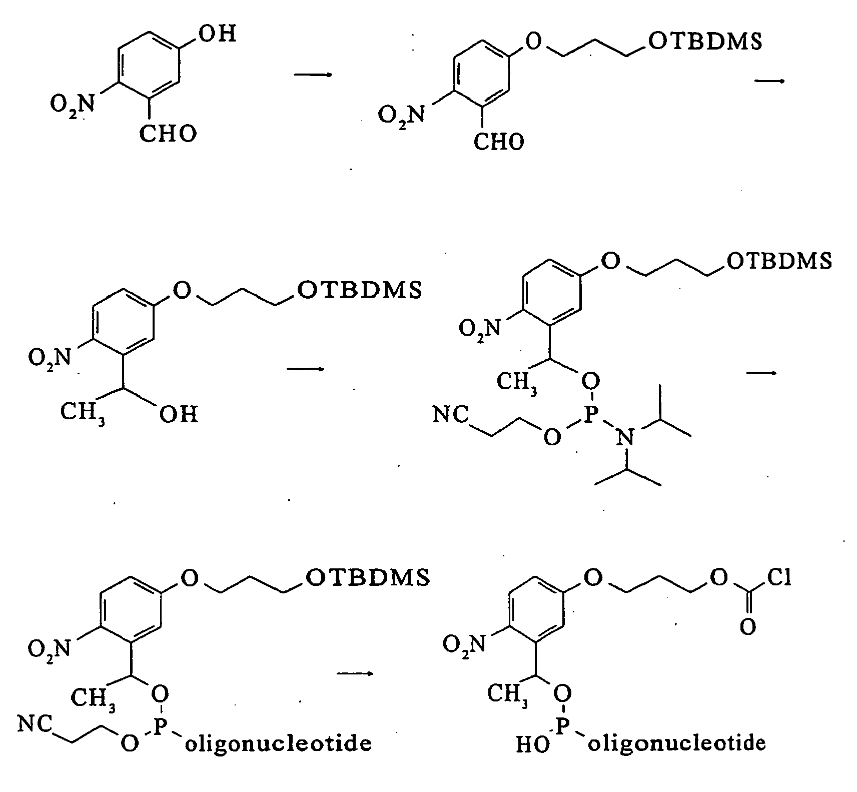

For the synthesis of the compounds provided herein containing an acid labile linker, *e.g.*, a heterobifunctional trityl ether, the requisite phosphoramidite trityl ether is reacted with the oligonucleotide or oligonucleotide analog Q, followed by deprotection of the trityl ether and capture of a biomolecule, *e.g.*, a protein, on the alcohol via a reactive derivative of the alcohol (X), as described above.

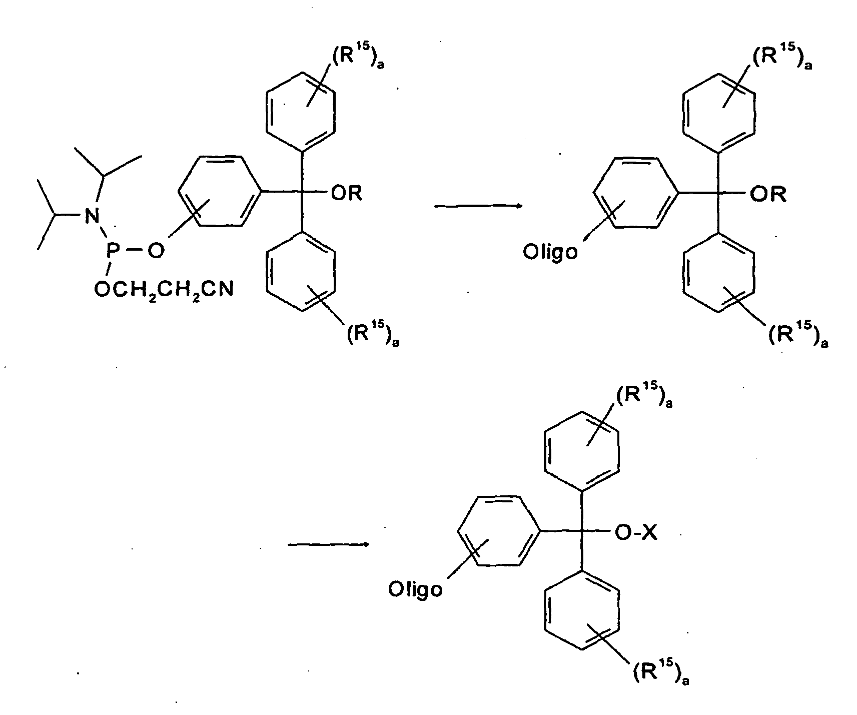

Those of skill in the art will appreciate that the above syntheses of the compounds provided herein are exemplary only. Other syntheses of the compounds provided herein can be envisioned. Also, one of skill in the art will be able to modify the above syntheses in a routine manner to synthesize other compounds within the scope of the instant disclosure.

### E. Methods of Use of the Arrays

### 1. General methods

The arrays provided herein may be used for the analysis, quantification, purification and/or identification of the components of biomolecule mixtures, including, but not limited to, protein mixtures. To initiate the analytical process, these mixtures are pre-purified according to standard procedures. In one embodiment, proteins are isolated from biological fluids by cell lysis followed by either precipitation methods (*e.g.*, ammonium sulfate) or enzymatic degradation of the nucleic acids and carbohydrates (if necessary) and the low molecular weight material is removed by molecular sieving. Proteins can also be obtained from expression libraries. Aliquots of the protein mixture are reacted with the compounds provided herein, having different functionalities X to segregate the mixture into separate protein families according to the selected reactivity of X. The diversity of B is selected depending on the complexity of the mixture of proteins. Hence, there are sets of compounds differing in X and B which can be selected for the analysis. In certain embodiments, the analysis is conducted using the smallest possible number of reactions necessary to completely analyze the mixture. Thus, in these embodiments, selection of the diversity of B and of the number of X groups of different reactivity will be a function of the complexity of the biomolecular mixture to be analyzed. Minimization of the diversity of B and the number of X groups allows for complete analysis of the mixture with minimal complexity.

The separation of proteins from a complex mixture is achieved by virtue of the compound-protein products being bound to an array of complementary sequence. The supernatant, which contains the compound-protein products, is contacted with and allowed to hybridize to an array of complementary sequences. In one embodiment, a flat solid support which carries at spatially distinct locations, an array of oligonucleotides or oligonucleotide analogs that is complementary to the selected N¹ₘ-Bᵢ-N²ₙ oligonucleotide or oligonucleotide analog, is hybridized to the compound-protein products.

In embodiments where Z is an insoluble support or substrate, such as a bead, separation of the compound-protein products into an addressable array may be achieved by sorting into an array of microwell or microtiter plates, or other microcontainer arrays. In certain embodiments, the microwell or microtiter plates, or microontainers, include single stranded oligonucleotides or oligonucleotide analogs that are complementary to the oligonucleotide or oligonucleotide analog Q.

After reaction or complexation of the compounds with the proteins, any excess compounds can be removed by adding a reagent designed to act as a "capturing agent." For example, a biotinylated small molecule, which has a functionality identical or similar to that which reacted with the selected X, is allowed to react with any excess compound. Exposure of this mixture to streptavidin bound to a magnetic bead, allows for removal of the excess of the compound.

Hybridization of the compound-protein products to a complementary sequence is effected according to standard conditions (*e.g.*, in the present of chaotropic salts to balance Tₘ values of the various hybrids). Any non-hybridized material can be washed off and the hybridized material analyzed.

In other embodiments, selective pooling of the products of different X moiety-containing reagents (*e.g.*, amino- and thiol-reactive X groups; antibody and amino-reactive X groups; antibody and lectin X groups, etc.) may be performed for combined analysis on a single assay (*e.g.*, on a single chip).

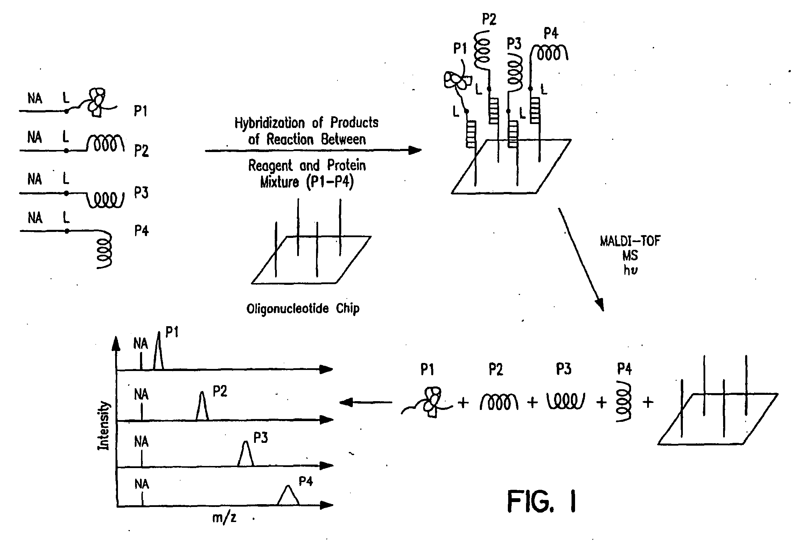

Figure 1 shows a method to separate and analyze a complex mixture of proteins by use of MALDI-TOF mass spectrometry. Exposure of a compound as described herein, to a mixture of biomolecules, including, but not limited to, proteins (P1 to P4), affords a compound-protein array (NA = oligonucleotide moiety or oligonucleotide analog moiety, L = cleavable linker, P = protein). Separation of the array is effected by hybridization of the Q portion of the array to a complementary sequence attached to a support, such as an oligonucleotide chip. The proteins (P1 to P4) are then analyzed by MALDI-TOF mass spectrometry.

When the complexity of a mixture of biomolecules, including, but not limited to, proteins, is low, affinity chromatographic or affinity filtration methods can be applied to separate the compound-protein products from the protein mixture. If the proteins to be analyzed were fluorescently labeled prior to (or after) reaction with the compound but prior to hybridization, these labeled proteins could also be detected on the array. In this way the positions which carry a hybrid can be detected prior to scanning over the array with MALDI-TOF mass spectrometry and the time to analyze the array minimized. Mass spectrometers of various kinds can be applied to analyze the proteins (*e.g.*, linear or with reflection, with or without delayed extraction, with TOF, Q-TOFs or Fourier Transform analyzer with lasers of different wavelengths and xy sample stages).

Mass spectrometer formats for use herein are matrix assisted laser desorption ionization (MALDI), continuous or pulsed electrospray (ES) ionization, ionspray, thermospray, or massive cluster impact mass spectrometry and a detection format such as linear time-of-flight (TOF), reflectron time-of-flight, single quadruple, multiple quadruple, single magnetic sector, multiple magnetic sector, Fourier transform, ion cyclotron resonance (ICR), ion trap, and combinations thereof such as MALDI-TOF spectrometry. For example, for ES, the samples, dissolved in water or in a volatile buffer, are injected either continuously or discontinuously into an atmospheric pressure ionization interface (API) and then mass analyzed by a quadrupole. The generation of multiple ion peaks which can be obtained using ES mass spectrometry can increase the accuracy of the mass determination. Even more detailed information on the specific structure can be obtained using an MS/MS quadrupole configuration.

Methods for performing MALDI are well known to those of skill in the art. Numerous methods for improving resolution are also known. For example, resolution in MALDI TOF mass spectrometry can be improved by reducing the number of high energy collisions during ion extraction (see, *e.g.,* Juhasz et al. (1996) Analysis, Anal. Chem. 68:941-946, see also, *e.g.,* U.S. Patent No. 5,777,325, U.S. Patent No. 5,742,049, U.S. Patent No. 5,654,545, U.S. Patent No. 5,641,959, U.S. Patent No. 5,654,545, U.S. Patent No. 5,760,393 and U.S. Patent No. 5,760,393 for descriptions of MALDI and delayed extraction protocols).

In MALDI mass spectrometry, various mass analyzers can be used, *e.g.*, magnetic sector/magnetic deflection instruments in single or triple quadrupole mode (MS/MS), Fourier transform and time-of-flight (TOF), including orthogonal time-of-flight (O-TOF), configurations as is known in the art of mass spectrometry. For the desorption/ionization process, numerous matrix/laser combinations can be used. Ion-trap and reflectron configurations can also be employed.

MALDI-MS requires the biomolecule to be incorporated into a matrix. It has been performed on polypeptides and on nucleic acids mixed in a solid *(i.e.,* crystalline) matrix. The matrix is selected so that it absorbs the laser radiation. In these methods, a laser, such as a UV or IR laser, is used to strike the biopolymer/matrix mixture, which is crystallized on a probe tip or other suitable support, thereby effecting desorption and ionization of the biopolymer. In addition, MALDI-MS has been performed on polypeptides, glycerol, and other liquids as a matrix.

A complex protein mixture can be selectively dissected, and in taking all data together, completely analyzed through the use of compounds with different functionalities X. The proteins present in a mixture of biological origin can be detected because all proteins have reactive functionalities present on their surfaces. If at each position on the compound-protein array, there is the same protein cleavable under the same conditions as L or is added without covalent attachment to the solid support and serving as an internal molecular weight standard, the relative amount of each protein (or peptide if the protein array was enzymatically digested) can be determined. This process allows for the detection of changes in expressed proteins when comparing tissues from healthy and disease individuals, or when comparing the same tissue under different physiological conditions (*e.g.*, time dependent studies). The process also allows for the detection of changes in expressed proteins when comparing different sections of tissues (*e.g.*, tumors), which may be obtained, *e.g.*, by laser bioposy.

Protein-protein interactions and protein-small molecule (*e.g.*, drug) interactions can be studied by contacting the compound-protein array with a mixture of the molecules of interest. In this case, a compound will be used which has no cleavable linkage L, or which has a linkage L that is stable under MALDI-TOF MS conditions. Subsequent scanning of the array with the mass spectrometer demonstrates which hybridized proteins of the protein array have effectively interacted with the protein or small molecule mixtures of interest.

Analysis using the well known 2-hybrid methodology is also possible and can be detected via mass spectrometry. See, *e.g.*, U.S. Patent Nos. 5,512,473, 5,580,721, 5,580,736, 5,955,280, 5,695,941. See also, Brent et al. (1996) Nuclei Acids Res. 24(17):3341-3347.

In the above embodiments, including those where Z contains a cleavable linkage, the compounds may contain a mass modifying tag. In these embodiments, the mass modifying tag is used to analyze the differences in structure (*e.g.*, side chain modification such as phosphoylation or dephosphorylation) and/or expression levels of biopolymers, including proteins. In one embodiment, two compounds (or two sets of compounds having identical permuted B moieties) are used that only differ in the presence or absence of a mass modifying tag (or have two mass tags with appropriate mass differences). One compound (or one set of compounds) is (are) reacted with "healthy" tissue and the mass modified compound(s) are reacted with the "disease" tissue under otherwise identical conditions. The two reactions are pooled and analyzed in a duplex mode. The mass differences will elucidate those proteins that are altered structurally or expressed in different quantity in the disease tissue. Three or more mass modifying tags can be used in separate reactions and pooled for multiplex analysis to follow the differences during different stages of disease development *(i.e.,* mass modifying tag 1 at time point 1, mass modifying tag 2 at time point 2 etc.), or, alternatively, to analyze different tissue sections of a disease tissue such as a tumor sample.

In further embodiments, selectivity in the reaction of the compounds provided herein with a biopolymer, including a protein, mixture can also be achieved by performing the reactions under kinetic control and by withdrawing aliquots at different time intervals. Alternatively, different parallel reactions can be performed (all differing in the B moiety of the Q group) and either performed with different stochiometric ratios or stopped at different time intervals and analyzed separately.

In embodiments where the compounds provided herein possess a luminescent or colorimetric group, the immobilized compound-biomolecule conjugate may be viewed on the insoluble support prior to analysis. Viewing the conjugate provides information about where the conjugate has hybridized (such as for subsequent MALDI-TOF mass spectrometric analysis). In certain embodiments, with selected reagents the quantity of a given protein from separate experiments (*e.g.,* healthy vs. disease, time point 1 vs. time point 2, etc.) may be determined by using dyes which can be spectrophotometrically differentiated.

In another embodiment, the methods are performed by tagging the biopolymers to be analyzed, including but not limited to proteins, with more than one, in one embodiment three to five, of the compounds provided herein. Such compounds would possess functionality designed to target smaller chemical features of the biomolecules rather than a macromolecular feature. See, *e.g.*, Figure 8. Such smaller chemical features include, but are not limted to, NH₂, SH, SS (after capping SH, SS may be targeted by, *e.g.*, gold), and OH. In one non-limiting example, the phenolic OH of tyrosine is selectively captured using a diazo compound, such as an aryldiazonium salt. In this embodiment, the reaction may be performed in water. For example, a functionalized diazonium salt could be used where the functionality allows for subsequent capture of a compound provided herein, thereby providing a oligonucleotide-labelled biomolecule. One such functionalized diazonium salt is:

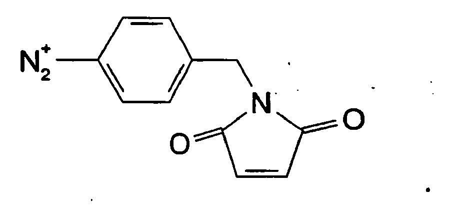

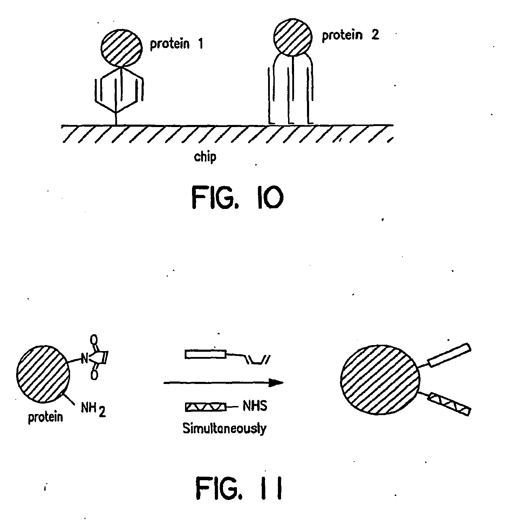

A biomolecule modified with this reagent is then labelled with an oligonucleotide possessing a diene residue. It is appreciated by those of skill in the art that many reagent couples other that dienophile/diene may be used in these embodiments. In the case of dienophile/diene, the reaction of the dienophile with the diene may be performed in the presence of many other functional groups, including N-hydroxysuccinimido-activated oligonucleotides reacting with an NH₂ group. Thus, these two labelling specific reactions may be performed simultaneously (*i.e.*, in one reaction mixture). See, *e.g.,* Figure 11.

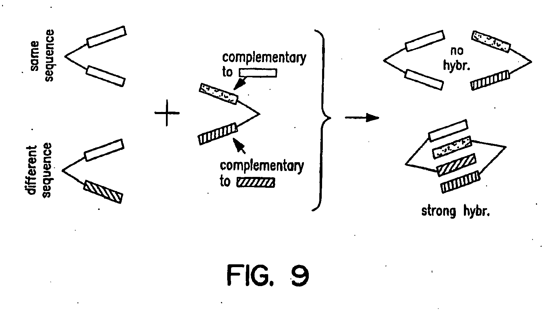

Subsequently, the multiply-tagged biomolecules are hybridized on an array of antisense oligonucleotides, in one embodiment a chip containing an array of antisense oligonucleotides. In general, the specificity of hybridization increases using compounds provided herein where Z is a dendrimer. See, *e.g.*, Figure 9. In one embodiment, Z is a dendritic structure containing up to about 6 branches. In this embodiment, the methods provided herein allow for separation between biomolecules labelled with, *e.g.,* fibe oligotags, where four are similar and one is different. See, *e.g.*, Figure 10.

In embodiments where the compounds for use in the methods provided herein are insoluble or poorly soluble in water or aqueous buffers, organic solvents are added to the buffers to improve solubility. In one embodiment, the ratio of buffer:organic solvent is such that denaturation of the biomolecule does not occur. In another embodiment, the organic solvents used include, but are not limited to, acetonitrile, formamide and pyridine. In another embodiment, the ratio of buffer:organic solvent is about 4:1. To determine if an organic co-solvent is needed, the rate of reaction of the compounds provided herein with a water-souble amine, such as 5'-aminothymidine, is measured. For example, the following reaction is performed is a variety of solvent mixtures well known to those of skill in the art to determine optimal conditions for subsequent biomolecular tagging and analysis:

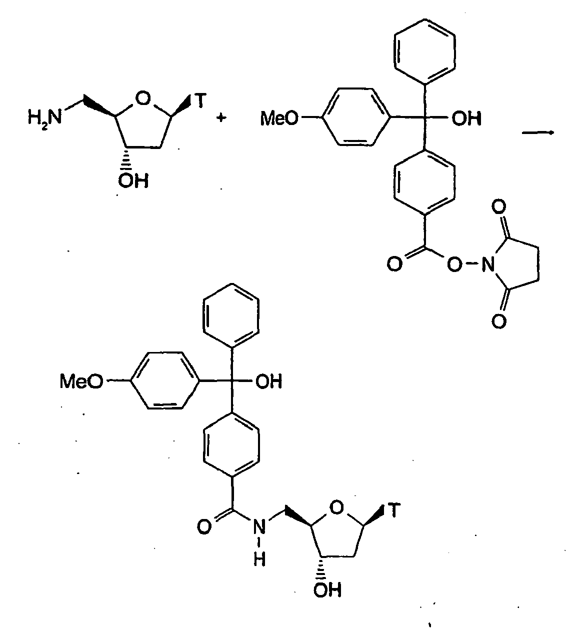

### 2. Phenotype analyses

The arrays permit a top down holistic approach to analysis of the proteome and other biomolecules. As noted, the arrays and methods of use provide an unbiased way to analyze biomolecules, since the methods do not necessarily assess specific classes of targets, but rather detect or identify changes in the samples. The changes identified include structural changes that are related to the primary sequences and modifications, including post-translational modifications. In addition, since the capture compounds can include a solubility function they can be designed for reaction in hydrophobic conditions, thereby permitting analysis of membrane-bound and membrane-associated molecules, particularly proteins.

Problems with proteome analysis arise from genetic variation that is not related to a target phenotype, proteome variation due to differences, such as gender, age, metabolic state, the complex mixtures of cells in target tissues and variations from cell cycle stage. Thus, to identify or detect changes, such as disease-related changes, among the biomolecule components of tissues and cells, homogeneity of the sample can be important. To provide homogeneity, cells, with different phenotypes, such as diseased versus healthy, from the same individual are compared. As a result, differences in patterns of biomolecules can be attributed to the differences in the phenotype rather than from differences among individuals. Hence, samples can be obtained from a single individual and cells with different phenotypes, such as healthy versus diseased and responders versus non-responders, are separated. In addition, the cells can be synchronized or frozen into a metabolic state to further reduce background differences.

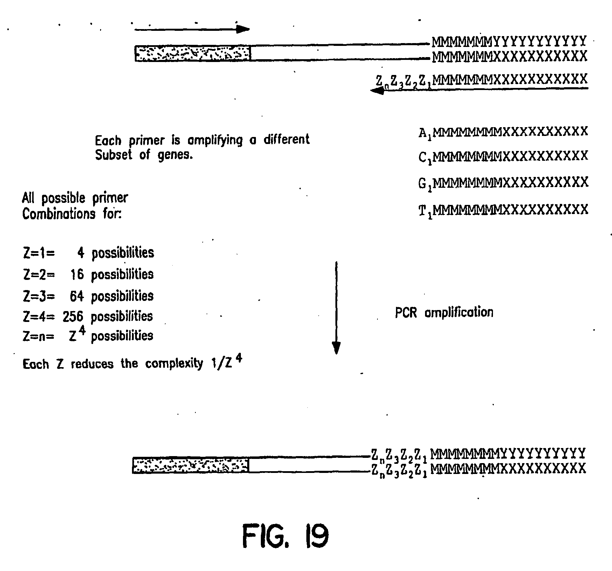

Thus, the arrays can be used to identify phenotype-specific proteins or modifications thereof or other phenotype-specific biomolecules and patterns thereof. This can be achieved by comparing biomolecule samples from cells or tissues with one phenotype to the equivalent cells to biomolecule samples form cells or tissues with another phenotype. Phenotypes in cells from the same individual and cell type are compared. In particular, primary cells, primary cell culture and/or synchronized cells are compared. The patterns of binding of biomolecules from the cells to capture compound members of the collection can be identified and used as a signature or profile of a disease or healthy state or other phenotypes. The particular bound biomolecule, such as protein, proteins also can be identified and new disease-associated markers, such as particular proteins or structures thereof can be identified. Example 6 provides an exemplary embodiment in which cells are separated. See also Figure 19.

Phenotypes for comparison include, but are not limited to:
1) samples from diseased versus healthy cells or tissues to identify proteins or other biomolecules associated with disease or that are markers for disease;
2) samples from drug responders and non responders (*i.e.* on 20-30% of malignant melanoma patients respond to alpha interferon and others to do not) to identify biomolecules indicative of response;
3) samples from cells or tissues with a toxicity profile to drugs or environmental conditions to identify biomolecules associated with the response or a marker of the response; and
4) samples from cells or tissues exposed to any condition or exhibiting any phenotype in order to identify biomolecules, such as proteins, associated with the response or phenotype or that are a marker therefor.

Generally the samples for each phenotype are obtained from the same organism, such as from the same mammal so that the cells are essentially matched and any variation should reflect variation due to the phenotype not the source of the cells. Samples can be obtained from primary cells (or tissues). In all instances, the samples can be obtained from the same individual either before exposure or treatment or from healthy non-diseased tissue in order to permit identification of phenotype-associated biomolecules.

Cells can be separated by any suitable method that permits identification of a particular phenotype and then separation of the cells based thereon. Any separation method, such as, for example, panning, negative panning-where unwanted cells are captured and the wanted cells remain in the supernatant) where the live cells are recovered can be used. These methods include, but are not limited to:
1) flow cytometry;
2) specific capture;
3) negative panning in which unwanted cells are captured and the targeted cells remain in the supernatant and live cells are recovered for analysis; and
4) Laser Capture Microdissection (LCM) (Arcturus, Inc Mountain View, CA).

Thus sorting criteria include, but are not limited to, membrane potential, ion flux, enzymatic activity, cell surface markers, disease markers, and other such criteria that permit separation of cells from an individual based on phenotype.

### a) Exemplary separation methods

### 1) Laser Capture Microdissection

Laser Capture Microdissection (LCM) (Arcturus, Inc Mountain View, CA) uses a microscope platform combined with a low-energy IR laser to activate a plastic capture film onto selected cells of interest. The cells are then gently lifted from the surrounding tissue. This approach precludes any absorption of laser radiation by microdissected cells or surrounding tissue, thus ensuring the integrity of RNA, DNA, and protein prepared from the microdissected samples for downstream analysis.

### 2) Flow cytometry for separation

Flow cytometry is a method, somewhat analogous to fluorescent microscopy, in which measurements are performed on particles (cells) in liquid suspension, which flow one at a time through a focused laser beam at rates up to several thousand particles per second. Light scattered and fluorescence emitted by the particles (cells) is collected, filtered, digitized and sent to a computer for analysis. Typically flow cytometry measures the binding of a fluorochrome-labeled probe to cells and the comparison of the resultant fluorescence to the background fluorescence of unstained cells. Cells can be separated using a version of flow cytometry, flow sorting, in which the particles (cells) are separated and recovered from suspension based upon properties measured in flow. Cells that are recovered via flow sorting are viable and can be collected under sterile conditions. Typically recovered subpopulations that are in excess of 99.5% pure (see Figures 19a and 19b).

Flow cytometry allows cells to be distingused using various parameters including physical and/or chemical characteristics associated with cells or properties of cell-associated reagents or probes, any of which are measured by instrument sensors. Separation: Live v. Dead Forward and side scatter are used for preliminary identification and gating of cell populations. Scatter parameters are used to exclude debris, dead cells, and unwanted aggregates. In a peripheral blood or bone marrow sample, lymphocyte, monocyte and granulocyte populations can be defined, and separately gated and analyzed, on the basis of forward and side scatter. Cells that are recovered via flow sorting are viable and can be collected under sterile conditions. Typically recovered subpopulations are in excess of 99.5% pure.

Common cell sorting experiments usually involve immunofluorescence assays, *i.e.,* staining of cells with antibodies conjugated to fluorescent dyes in order to detect antigens. In addition, sorting can be performed using GFP-reporter constructs in order to isolate pure populations of cells expressing a given gene/construct.

### a. Fluorescence

Fluorescent parameter measurement permits investigation of cell structures and functions based upon direct staining, reactions with fluorochrome labeled probes (*e.g.*, antibodies), or expression of fluorescent proteins. Fluorescence signals can be measured as single or multiple parameters corresponding to different laser excitation and fluorescence emission wavelengths. When different fluorochromes are used simultaneously, signal spillover can occur between fluorescence channels. This is corrected through compensation. Certain combinations of fluorochromes cannot be used simultaneously; those of skill in the art can identify such combinations.

### b. Immunofluorescence

Immunofluorescence involves the staining of cells with antibodies conjugated to fluorescent dyes such as FITC (fluorescein), PE (phycoerythrin), APC (allophycocyanin), and PE-based tandem conjugates (R670, CyChrome and others.). Cell surface antigens are the usual targets of this assay, but antibodies can be directed at antigens or cytokines in the cytoplasm as well.

DNA staining is used primarily for cell cycle profiling, or as one method for measuring apoptosis. Propidium iodide (P1), the most commonly used DNA stain, cannot enter live cells and can therefore be used for viability assays. For cell cycle or apoptosis assays using PI, cells must first be fixed in order for staining to take place (see protocol). The relative quantity of PI-DNA staining corresponds to the proportion of cells in GO/G1, S, and G2/M phases, with lesser amounts of staining indicating apoptotic/necrotic cells. PI staining can be performed simultaneously with certain fluorochromes, such as FITC and GFP, in assays to further characterize apoptosis or gene expression.

Gene Expression and Transfection can be measured indirectly by using a reporter gene in the construct. Green Fluorescent Protein-type constructs (EGFP, red and blue fluorescent proteins) and β-galactosidase, for example, can be used to quantify populations of those cells expressing the gene/construct. Mutants of GFP are now available that can be excited at common frequencies, but emit fluorescence at different wavelengths. This allows for measurement of co-transfection, as well as simultaneous detection of gene and antibody expression. Appropriate negative (background) controls for experiments involving GFP-type constructs should be included. Controls include, for example, the same cell type, using the gene insert minus the GFP-type construct.

### 3) Metabolic Studies and other studies

Annexin-V can be labeled with various fluorochromes in order to identify cells in early stages of apoptosis. CFSE binds to cell membranes and is equally distributed when cells divide. The number of divisions cells undergo in a period of time can then be counted. CFSE can be used in conjunction with certain fluorochromes for immunofluorescence. Calcium flux can be measured using Indo-1 markers. This can be combined with immunofluorescent staining. Intercellular conjugation assays can be performed using combinations of dyes such as calcein or hydroethidine.

### b) Synchronizing cell cycles

Once sorted or separated cells are obtained they can be cultured, and, can be synchronized or frozen into a particular metabolic state. This enhances the ability to identify phenotype-specific biomolecules. Such cells can be separated by the above methods, including by flow cytometry. Further, cells in the same cell cycle, same metabolic state or other synchronized state can be separated into groups using flow cytometry (see, Figure 19c).

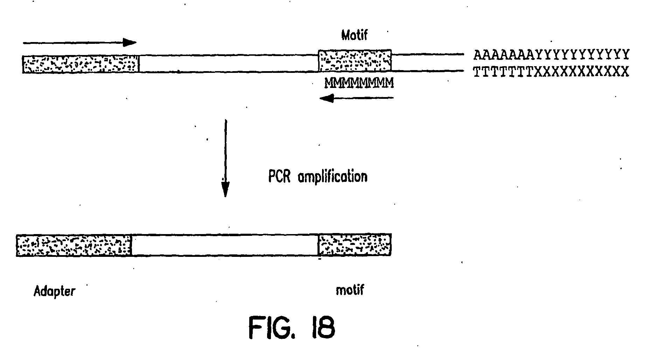

Cell cycles can be synchronized or frozen by a variety of methods, including but are not limited to, cell chelation of critical ions, such as by removal of magnesium, zinc, manganese, cobal and/or other ions that perform specific functions by EDTA or otherchelators (see, *e.g.*, EXAMPLES). Other methods include controlling various metabolic or biochemical pathways. Figure 18 depicts exemplary points of regulation of metabolic control mechanisms for cell synchronization. Examples of synchronizing or "freezing" Metabolic Control for synchronizing cells, include, but are not limited to, the following:
1) control of gene expression;
2) regulation of enzyme reactions;
3) negative control: Feedback inhibition or End product repression and enzyme induction are mechanisms of negative control that lead to a decrease in the transcription of proteins;
4) positive control: catabolite repression is considered a form of positive control because it affects an increase in transcription of proteins.
5) Control of individual proteins translation:
   a) oligonucleotides that hybridize to the 5' cap site have inhibit protein synthesis by inhibiting the initial interaction between the mRNA and the ribosome 40S sub-unit;
   b) oligonucleotides that hybridize to the 5' UTR up to, and including, the translation initiation codon inhibit the scanning of the 40S (or 305) subunit or assembly of the full ribosome (80S for eukaryotes or 70S for bacterial systems);
5) control of post translational modification:
6) control of allosteric enzymes, where the active site binds to the substrate of the enzyme and converts it to a product. The allosteric site is occupied by some small molecule that is not a substrate. If the protein is an enzyme, when the allosteric site is occupied, the enzyme is inactive, *i.e.*, the effector molecule decreases the activity of the enzyme. Some multicomponent allosteric enzymes have several sites occupied by various effector molecules that modulate enzyme activity over a range of conditions.

### 3. Analysis of low abundancy proteins

Important disease-associates markers and targets could be low abundancy proteins, that might not be detected by mass spectrometry. To ensure detection, a first capture compound display experiment can be performed. The resulting array of captured proteins is reacted with a non-selective dye, such as a fluorescent dye, that will light up or render visible more proteins on the array. The dye can provide ae semi-quantitative estimate of the amount of a protein. The of different proteins detected by the dye can be determined and then compared the number detected by mass spectrometric analysis. If there are more proteins detected using the dye, the experiments can be repeated using a higher starting number of cells so that low abundance proteins can be detected and identified by the mass spectrometric analysis.

For example, housekeeping proteins, such as actin and other such proteins, are present in high abundance and can mask low abundancy proteins. Capture compounds or other purification compound selected or designed to capture or remove the high abundancy proteins or biomolecules from a mixture before using a collection to asssess the components of the mixtuer. Once the high abundancy proteins are removed, low abundancy proteins have an effectivly higher concentration and can be detected. These methods, thus, have two steps: a first step to capture high abundancy components of biomolecule mixtures, such as the actins. For example, a cell lysate can be contacted with capture molecules that include a reactivity group such as biotin or other general reactivity function linked to a sorting group to remove such high abundancy proteins, and then use a suitable collection of capture compounds to identify lower abundancy compounds remaining in the lysate.

Also, as discussed above, capture compounds can be designed, such as by appropriate selection of W, to interact intact with intact organelles before disrupting them in cells that have been gently lysed or otherwise treated to permit access to organelles and internal membraes. Then the captured organelles can be disrupted, such as on which can inlcude an artificial membrane, such as lipid bilayer or micelle coating, to capture the organelle proteins and other biomolecules in an environment that retains their three-dimensional sturcture. These captured proteins can be analyzed. This permits the capture compounds to interact with the captured proteins and other biomolecules in thier native tertiary structure.

### 4. Monitoring protein conformation as an indicator of disease

The arrays and/or loci thereof can be used to detect or distinguish specific conformers of proteins. Hence, for example, if a particular conformation of a protein is associated with a disease (or healthy state) the arrays or member loci thereof can detect one conformer or distinguish conformers based upon a patter of binding to the capture compounds in a collection. Thus, the arrays and/or members thereof cna be used to detect conformationally altered protein diseases (or diseases of protein aggregation), where a-diseases-associated protein or polypeptide has a disease-associated conformation. The methods and arrays provided herein permit detection of a conformer associated with a disease to be detected. These diseases include, but are not limited to, amyloid diseases and neurodegenerative diseases. Other diseases and associated proteins that exhibit two or more different conformations in which at least one conformation is with disease, include those set forth in the following Table:

| **Disease** | **Insoluble protein** |
|---|---|
| Alzheimer's Disease (AD) | APP, Aβ, α1-antichymotrypsin, tau, non-Aβ component, presenellin 1, presenellin 2, apoE |
| Prion diseases, including but are not limited to, Creutzfeldt-Jakob disease, scrapie, bovine spongiform encephalopathy | PrP^{sc} |
| amyotrophic lateral sclerosis (ALS) | superoxide dismutase (SOD) and neurofilament |
| Pick's Disease | Pick body |
| Parkinson's disease | α-synuclein in Lewy bodies |
| Frontotemporal dementia | tau in fibrils |
| Diabetes Type II | amylin |
| Multiple myeloma | IgGL-chain |
| Plasma cell dyscrasias | |
| Familial amyloidotic polynueuropathy | Transthyretin |
| Medullary carcinoma of thyroid | Procalcitonin |
| Chronic renal failure | β₂-microgobulin |
| Congestive heart failure | Atrial natriuretic factor |
| Senile Cardiac and systemic amyloidosis | transthyretin |
| Chronic inflammation | Serum Amyloid A |
| Atherosclerosis | ApoAI |
| Familial amyloidosis | Gelsolin |
| Huntington's disease | Huntington |

The arrays can be contacted with a mixture of the conformers and the members that bind or retain each form can be identified, and a pattern thus associated with each conformer. Alternatively, those that bind to only one conformer, such as the conformer associated with disease can be identified, and sub-collections of one or more of such arrays can be used as a diagnostic reagent for the disease.

### 5. Small molecule identification and biomolecule-biomolecule interaction investigation

Biomolecules, such as proteins, are sorted using a covalent or noncovalent interaction with immobilized capture compounds. Arrays of bound to biomolecules, such as from cell lysates, then can be used to screen libraries or other mixtures of drug candidates or to further screen mixtuers of biomolecules to see what binds to the bound biomolecules. The capture biomolecule-biomolecule complexes or biomolecule-drug candidate complexes can be analyzed to identify biochemical pathways and also to identify targets with the candidate drug.

For example, protein-protein or protein-biomolecule interactions are exposed to test compounds, typically small molecules, including small organic molecules, peptides, peptide mimetics, antisense molecules or dsRNA, antibodies, fragments of antibodies, recombinant and sythetic antibodies and fragments thereof and other such compounds that can serve as drug candidates or lead compounds. Bound small molecules are identified by mass spectrometry or other analytical methods.

### F. Systems

In further embodiments, the compounds and the methods described herein are designed to be placed into an integrated system that standardizes and automates the following process steps:
• Isolation of biomolecules from a biological source, including isolation of the proteins from cell lysates (lysis, enzymatic digestion, precipitation, washing)
• Optionally, removal of low molecular weight materials
• Optionally, aliquoting the biomolecule mixture, such as a protein mixture
• Reaction of the biomolecule mixture, such as a protein mixture, with compounds of different chemical reactivity (X) and sequence diversity (B) provided herein; this step can be • performed in parallel using aliquots of the biomolecule mixture
• Optionally, removal of excess compound
• Hybridization of the compound-biomolecule conjugate, such as a compound-protein conjugate to single stranded oligonucleotides or oligonucleotide analogs that are complementary to the Q moiety of the compound; the single stranded oligonucleotides or oligonucleotide analogs are optionally presented in an array format and are optionally immobilized on an insoluble support
• Optionally, subsequent chemical or enzymatic treatment of the protein array
• Analysis of the biomolecule array, including, but not limited to, the steps of (i) deposition of matrix, and (ii) spot-by-spot MALDI-TOF mass spectrometry using an array mass spectrometer (with or without internal, *e.g.*, on-chip molecular weight standard for calibration and quantitation).

The system includes the collections provided herein, optionally arrays of such collections, software for control of the processes of sample preparationa and instrumental analyis and for analysis of the resulting data, and instrumentation, such as a mass spectrometer, for analysis of the biolmolecules. The system include other devices, such as a liquid chromatographic devices so that a protein mixture is at least partially separated. The eluent is collected in a continuous series of aliquots into, *e.g.*, microtiter plates, and each aliquot reacted with a capture compound provided.

In multiplex reactions, aliquots in each well can simultaneously react with one or more of the capture compounds provided herein that, for example each differ in X (*i.e.,* amino, thiol, lectin specific functionality) with each having a specific and differentiating selectivity moiety Y and in the Q group. Chromatography can be done in aqueous or in organic medium. The resulting reaction mixtures are pooled and analyzed directly. Alternatively, subsequent secondary reactions or molecular interaction studies are performed prior to analysis, including mass spectrometric analysis.

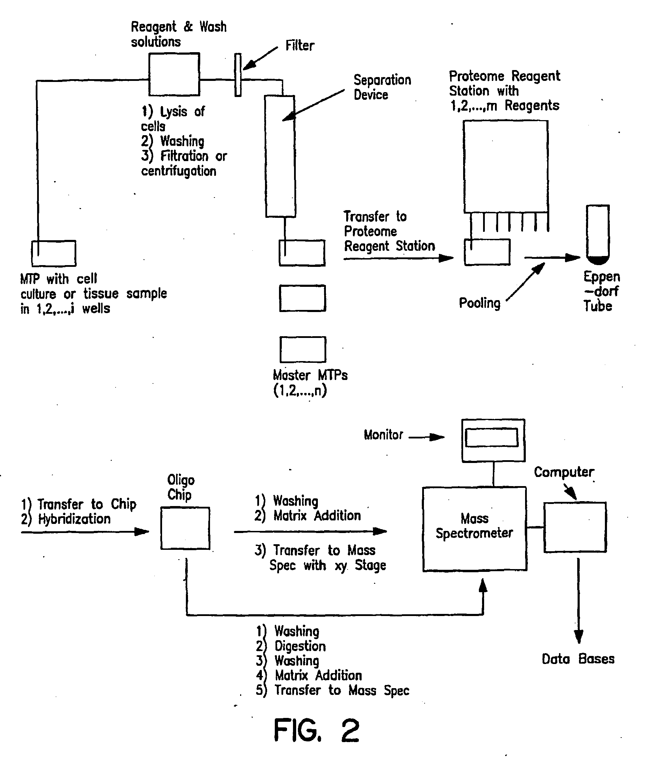

The systems provided herein can contains an assembly line, such as pipetting robots on xy stages and reagent supply/washing modules are linked with a central separation device and a terminal mass spectrometer for analysis and data interpretation. The systems can be programmed to perform process steps including (see, *e.g.*, FIG. 2), for example:
1) Cell cultures (or tissue samples) are provided in microtiter plates (MTPs) with 1, 2...i wells. To each well, solutions are added for lysis of cells, thereby liberating the proteins. In some embodiments, appropriate washing steps are included, as well as addition of enzymes to digest nucleic acids and other non-protein components. In further embodiments, instead of regular MTPs, MTPs with filter plates in the bottom of wells are used. Cell debris is removed either by filtration or centrifugation. A conditioning solution for the appropriate separation process is added and the material from each well separately loaded onto the separation device.
2) Separation utilizes different separation principles such as charge, molecular sizing, adsorption, ion-exchange, and molecular exclusion principles. Depending on the sample size, suitable appropriate dimensions are utilized, much as microbore high performance liquid chromatography (HPLC). In certain embodiments, a continuous flow process is used and the effluent is continuously aliquotted into MTP 1,2...n.
3) Reaction with Proteome Reagents. Each MTP in turn is transferred to a Proteome Reagent Station harboring 1, 2... m reagents differing only in the oligonucleotide sequence part *(i.e.,* Q) or/and in the chemical nature of the functionality reacting with the proteins *(i.e.,* X). If there are more than one MTP coming from one tissue sample then reagent 1 is added to the same well of the respective MTPs 1, 2...n, *i.e.,* in well A1, reagent 2 in well A2, etc. In embodiments where the MTPs have 96 wells (i = 1-96), 96 different Proteome Reagents *(i.e.,* 96 different compounds provided herien, m = 1-96) are supplied through 96 different nozzles from the Proteome Reagent Station to prevent cross-contamination.
4) Pooling: Excess Proteome Reagent is deactivated, aliquots from each well belonging to one and the same tissue samples are pooled, and the remaining material is stored at conditions that preserve the structure (and if necessary conformation) of the proteins intact, thereby serving as master MTPs for subsequent experiments.
5) Excess Proteome Reagent is removed in the pooled sample using, *e.g.*, the biotin/streptavidin system with magnetic beads, then the supernatant is concentrated and conditioned for hybridization.
6) Transfer to an Oligonucleotide Chip. After a washing step to remove non-hybridized and other low molecular weight material, a matrix is added. Alternatively, before matrix addition, a digestion with, *e.g.*, trypsin or/and chymotrypsin is performed. After washing out the enzyme and the digestion products, the matrix is added.
7) Transfer of chip to mass spectrometer. In one embodiment, MALDI-TOF mass spectrometry is performed. Other mass spectrometric configurations suitable for protein analysis also can be applied. The mass spectrometer has a xy stage and thereby rasters over each position on the spot for analysis. The Proteome Reagent can be designed so that most of the reagent part (including the part hybridizing with the oligonucleotide chip array) is cleaved either before or during mass spectrometry and therefore will be detected in the low molecular weight area of the spectrum and therefore well be well separated from the peptide (in case of enzymatic digestion) or protein molecular weight signals in the mass spectrum.
8) Finally, the molecular weight signals can be processed for noise reduction, background subtraction and other such processing steps. The data obtained can be archived and interpreted. The molecular weight values of the proteins (or the peptides obtained after enzymatic digestion) are associated with the human DNA sequence information and the derived protein sequence information from the protein coding regions. An interaction with available databases will reveal whether the proteins and their functions are already known. If the function is unknown, the protein can be expressed from the known DNA sequence in sufficient scale using standard methods to elucidate its function and subsequent location in a biochemical pathway, where it plays its metabolic role in a healthy individual or in the disease pathway for an individual with disease.

Since the master plates containing aliquots from the different proteins within a given tissue sample have been stored and are available, ubsequent experiments then can be performed in a now preselected way, *e.g.*, the proteins are displayed on the chip surface for protein-protein (biomolecule) interaction studies for target validation or/and to study the interaction with combinatorial libraries of small molecules for drug candidate selection.

### G. Bioinformatics

The raw data generated from the mass spectrometry analysis of the compound-protein species is processed by background subtraction, noise reduction, molecular weight calibration and peak refinement (*e.g.*, peak integration). The molecular weight values of the cleaved proteins or the digestion products are interpreted and compared with existing protein data bases to determine whether the protein in question is known, and if so, what modifications are present (glycosylated or not glycosylated, phosphorylated or not phosphorylated, etc.). The different sets of experiments belonging to one set of compounds are composed, compared and interpreted. For example, one set of experiments uses a set of compounds with one X moiety and different Q moieties. This set of experiments provides data for a portion of the proteome, since not all proteins in the proteome will react with a given X moiety. Superposition of the data from this set of experiments with data from other sets of experiments with different X moieties provides data for the complete proteome.

Sets of experiments comparing tissues of healthy and disease individuals or from different physiological or developmental stages (*e.g.*, tumor progression, dependence of drug treatments to monitor result of therapy, immune response to virus or bacteria infection) or different tissues areas (*e.g.*, of a tumor) are investigated, and the final data archived.

The following examples are included for illustrative purposes only and are not intended to limit the scope of the invention.

### EXAMPLE 1

### Examples for N¹ₘ-B_{¡}-N²ₙ

### a. N¹ and N² as identical tetramers, B as a trimer

N¹ = N², m = n = 4, i = 3, B = 64 sequence permutations

| |
|---|
| GTGC ATG GTGC |
| AAG |
| ACG |
| AGG |
| TTG |
| CTG |
| GTG |
| ... |
| ... |
| ... |
| GGG |

### b. N¹ and N² as non-identical tetramers, B as a tetramer

N¹ ≠ N², m = n = 4, i = 4, B = 256 sequence permutations

| |
|---|
| GTCC ATCG CTAC |
| AACG |
| ACCG |
| AGCG |
| .... |
| .... |
| ... |
| GGGG |

### c. N¹ as a heptamer, N² as an octamer, B as an octamer

N¹ ≠ N², m = 7, n, = 8, i = 8, B = 65,536 sequence permutations.

GCTGCCC | ATTCGTAC | GCCTGCCC
N1 | B | N2

### EXAMPLE 2

### Separation of proteins on a DNA array

N¹ₘ-B_{¡}-N²ₙ-(S¹)ₜ-M(R¹⁵)ₐ-(S²)_{b}-L-X-Protein where B is a trimer;
m = n = 4, i = 3, t = b = 1; underlined sequences are N¹ and N²

CTGC ATG GTGC - S1 - M(R15)a - S2 - L - X - Protein 1
—CACG TAC CACG

CTGC AAG GTGC - S1 - M(R15)a - S2 - L - X - Protein 2
—CACG TTC CACG

CTGC ACG GTGC - S1 - M(R15)a - S2 - L - X - Protein 3
---CACG TGC CACG
...
...
...
CTGC GGG GTGC - S1 - M(R15)a - S2 - L - X - Protein 64
—CACG CCC CACG

### EXAMPLE 3

### Synthesis of Glass-based Arrays:

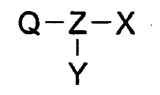

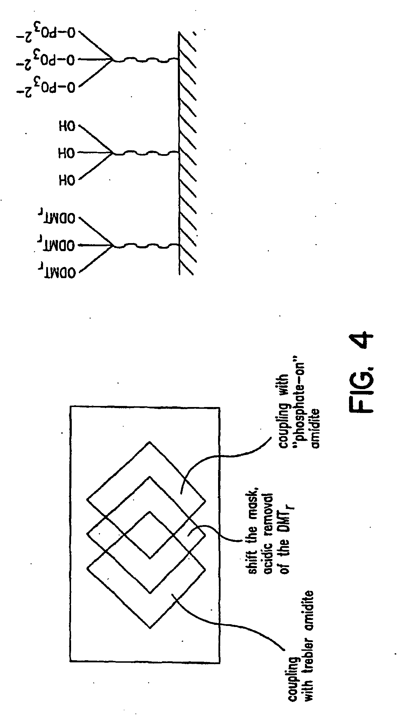

Glass slides wee silanized according to standard protocols using trimethoxyaminopropylsilane. The surface loading was boosted by first activating the slides with phanylenediisothiocyanate followed by treatment with 4th generation PAMAM (polyamidoamine) dedrimer (64 amino groups)(see, *e.g.*, http://www.dendritech.com/pamam.html). The slides were then coupled to an 18-atom amphiphilic linker using a phosphoramidite chemistry. The derivatized slides were then subjected to a masking synthesis method to produce distinct patches using trebler phosphoramidites (see, Figure 4). Alternatively, the phosphoramidites are applied to create a continuous gradient in each dimension of the glass slide. Features that may be varied using this masking or gradient synthesis include, but are not limited to, hydrophobicity/hydrophilicity and positive/negative charge.

### EXAMPLE 4

### 1. Preparation of protein mixtures from cells or via protein translation of a cDNA library prepared cell or tissues

The protein mixtures can be selectively divided on the physical or biochemical separation techniques

### 1. Preparation of limited complexity protein pools using cell culture or tissue

Proteins can be isolated from cell culture or tissues according to methods well known to those of skill in the art. The isolated proteins are purified using methods well known to those of skill in the art (*e.g.*, TPAE, differential protein precipitation (precipitation by salts, pH, and ionic polymers), differential protein crystallization bulk fractionation, electrophoresis (PAGE, isoelectric focusing, capillary), and chromatography (immunoaffinity, HPLC, LC)). Individual column fractions containing protein mixtures of limited complexity are collected for use as antigen.

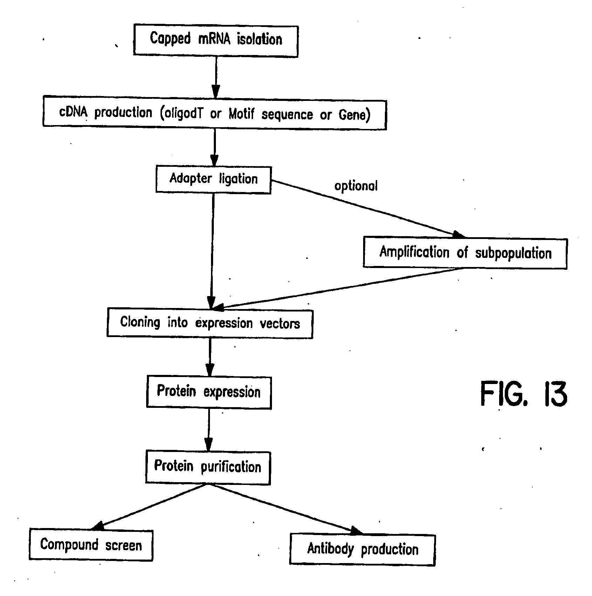

### 2. Preparation of limited complexity protein pools using cDNA expression libraries with (Figure 13)

### a. RNA Isolation

### i. Isolation of Total RNA

Cultured cells or tissues are homogenized in a denaturing solution containing 4 M guanidine thiocyanate. The homogenate is mixed sequentially with 2 M sodium acetate (pH 4), phenol, and finally chloroform/isoamyl alcohol or bromochloropropane. The resulting mixture is centrifuged, yielding an upper aqueous phase containing total RNA. Following isopropanol precipitation, the RNA pellet is dissolved in denaturing solution (containing 4 M guanidine thiocyanate), precipitated with isopropanol, and washed with 75% ethanol.

### ii. Isolation of Cytoplasmic RNA

Cells are washed with ice-cold phosphate-buffered saline and kept on ice for all subsequent manipulations. The pellet of harvested cells is resuspended in a lysis buffer containing the nonionic detergent Nonidet P-40. Lysis of the plasma membranes occurs almost immediately. The intact nuclei are removed by a brief micro centrifuge spin, and sodium dodecyl sulfate is added to the cytoplasmic supernatant to denature protein. Protein is digested with protease and removed by extractions with phenol/chloroform and chloroform. The cytoplasmic RNA is recovered by ethanol precipitation.

### b. mRNA purification

Messenger RNA is purified from total or cytoplasmic RNA preparation using standard procedures. Poly(A)⁺ RNA can be separated from total RNA by oligo (dT) binding to the Poly(A) tail of the mRNA. Total RNA is denatured to expose the Poly(A) (polyadenylated) tails. Poly(A)-containing RNA is then bound to magnetic beads coated with oligo(dT) and spirited from the total or cytoplasmic RNA through magnetic forces. The mRNA population can be further enriched for the presence of full-length molecules through the selection of a 5'-cap containing mRNA species.

### c. cDNA synthesis

Different types of primers can be used to synthesis full length or 5'-end containing cDNA libraries from the isolated mRNA.

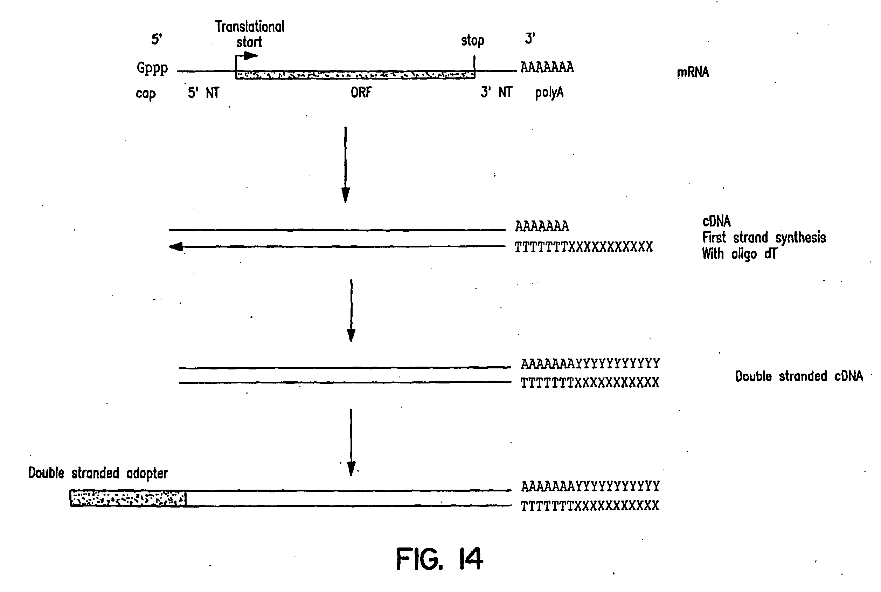

### i. Oligo (dT) primer, that will generate cDNAs for all mRNA species (Figure 14)

An example of the production of an adapted oligo dT primed cDNA library is provided in Figure 14.

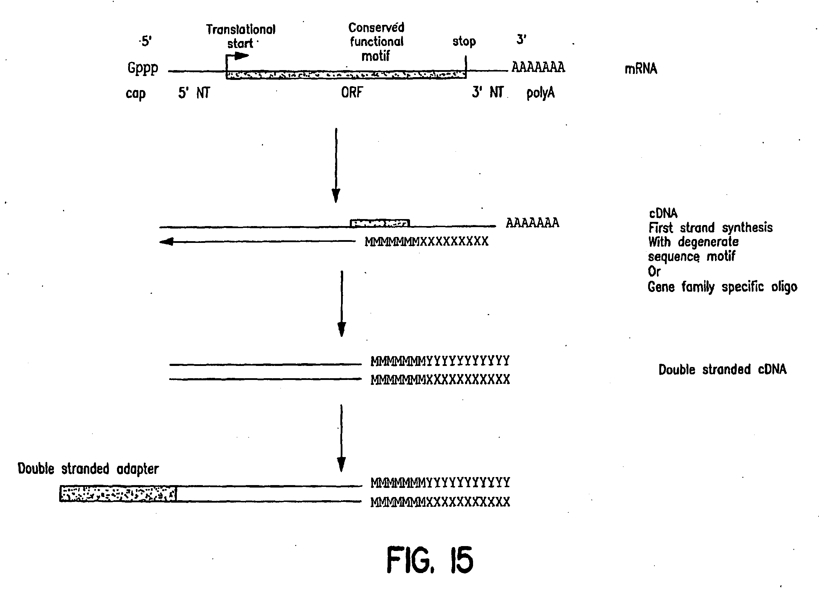

### ii. Functional protein motif specific degenerated oligonucleotides these primers will generate a limited number of genes belonging to the same protein family or of functionally related proteins (Figure 15)

An example of the production of an adapted sequence motif specific cDNA library is provided in Figure 15.

### iii. Gene specific oligonucleotide will produce cDNA for only one particular mRNA species (Figure 16)

The oligonucleotides used for the cDNA production can contain additional sequences, 1) protein tag specific sequences for easier purification of the recombinant proteins (6x HIS Figure 7), 2) restriction enzyme sites, 3) modified 5'-end for cDNA purification or DNA construction purpose (Figure 17).

The conversion of mRNA into double-stranded cDNA for insertion into a vector is carried out in two parts. First, intact mRNA hybridized to an oligonucleotide primer, is copied by reverse transcriptase and the products isolated by phenol extraction and ethanol precipitation. The RNA in the RNA-DNA hybrid is removed with RNase H as *E. coli* DNA polymerase I fills in the gaps. The second-strand fragments thus produced are ligated by *E. coli* DNA ligase. Second-strand synthesis is completed, residual RNA degraded, and cDNA made blunt with RNase H, RNase A, T4 DNA polymerase, and *E. coli* DNA ligase.

### d. Adapter ligation

Adapter molecules can be ligated to both ends of the blunt ended double stranded cDNA or to only one end of the cDNA. Site directed adapter ligation could be acheved through the use of 5' modified oligonucleotides (for example biotinylated, aminated) during cDNA synthesis that prevents adapter ligation to the 3' end of the cDNA. The resulting cDNA molecules contain a 5'-end cDNA library comprised of the 5' non-translated region, the translational start codon AUG coding for a methionine, followed by the coding region of the gene or genes. The cDNA molecules are flanked by known DNA sequence on their 5'- and 3'-end (Figures 14, 15 and 16).

### e. cDNA amplification

PCR Primers to the known 5'- and 3'-end sequences or known internal sequences can be synthesized and used for the amplification of either the complete library or specific subpopulations of cDNA using extended 5'- or 3'- amplification primer in combination with the primer located on the opposite site of the cDNA molecules (Figure 18).

### f. Primer design for the amplification of gene subpopulations

The sub-population primers contain two portions (Figure 19). The 5'-part of the primer is complementary to the sequence of a known sequence, extending with its 3'-end into the unknown cDNA sequence. Since each nucleotide in the cDNA part of the library can have an adenosine, cytidine, guanosine or thymidine residue, 4 different nucleotides possibilities exist for each nucleotide position. Four different amplification primers can be synthesized, each containing the same known sequence and extending by one nucleotide into the cDNA area of the library. The 4 primers only differ at their most 3'-nucleotide, being either A, C, G or T. If we suppose that each nucleotide (A, C, G, T) are equally represented in a stretch of DNA, each one of the 4 amplification primers will amplify one quarter of the total genes represented in the cDNA library. Extending the amplification primer sequence further and increasing the number of amplification primers, the complexity of the amplification products can be further reduced. Extending the sequence by 2 nucleotides requires the synthesis of 16 different primers decreasing the complexity by 16 fold, 3 nucleotides require 64 different primers and nucleotide extension requires n⁴ different primers.

### g. PCR amplification

PCR amplification entails mixing template DNA, two appropriate oligonucleotide primers (5'- and 3'-end primers located in the known added sequences directed in complementary orientation), *Tag* or other thermostable DNA polymerases, deoxyribonucleoside triphosphates (dNTPs), and a buffer. The PCR products are analyzed after cycling on DNA gels or through analysis on an ABI 377 using the genescan analysis software. These analysis methods allow the determination of the complexity of the amplified cDNA pool.

### h. Production of a protein expression library

Each amplified cDNA library sub-population is cloned 5' to 3' in a bacterial (*E. coli, etc.*) or eukaryotic (Baculovirus, yeast, mammalian) protein expression system. The gene s introduced with its own translational initiation signal and a 6xHis tag in all 3 frames. For example: The cDNA is restricted with two different, rare cutting restriction enzymes (5'-end BgIII and 3'-end Not I) and cloned in the 5' to 3' orientation in the Baculovirus transfer vector pVL1 393 under the direct control of the polyhedra promoter.

### i. Protein expression

Linearized Baculovirus DNA and recombinant transfer-vector DNA are being cotransfected into susceptible SF9 insect cells with calcium phosphate. For cotransfection, 10 ug of purified plasmid DNA will be prepared. An initial recombinant Baculovirus stock will be prepared and Sf9 cells are being infected for recombinant protein production.

### j. Protein purification

The expressed recombinant proteins contain an affinity tag (example is a 6xHis tag). They are being purification on Ni-NTA agarose. Approximately 1 to 2 mg of 6xHis recombinant fusion protein is routinely obtained per liter of insect cell culture.

### k. Purification Tag removal

If the expression vector or the amplification primer was constructed with a proteolytic cleavage site for thrombin, the purification tag can be removed from the recombinant proteins after the protein affinity purification step.

### II. Antibody generation by immunization of different animals with individual protein mixtures

### 3. Preparation of Antibody protein capture reagents

A purified protein preparation translated from a pool of cDNAs is injected intramuscularly, intradermally, or subcutaneously in the presence of adjuvant into an animal of the chosen species (rabbit). Booster immunizations are started 4 to 8 weeks after the priming immunization and continued at 2- to 3-week intervals. The polyclonal antiserum is being purified using standards known to those skilled in the art.

The purified antibody batches can be used directly as protein capture reagents without modification. In this case the antibody batches from different animals have to be kept separate (each batch is one capture reagent).

### III. Antibody proteins are isolated and conjugated with nucleic acid sequences which correspond to the original antigen preparation resulting in the antibody capture reagents

Generation of bi-functional capture/sorting molecules for sorting of the complex protein mixture on a solid phase.

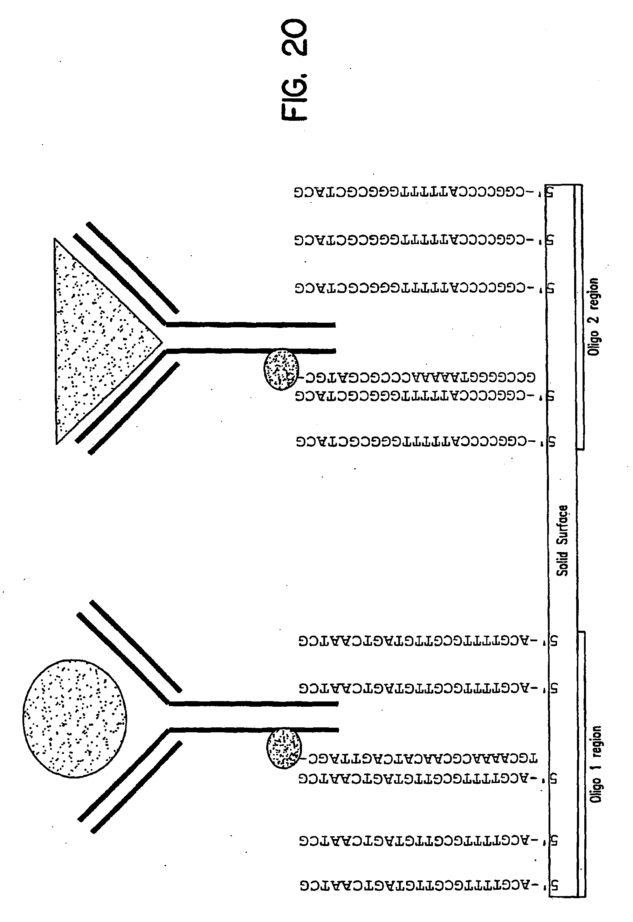

The glycosylated C_{H}² domain of the polyclonal antibodies are conjugation to 5' modified oligonucleotides using standard conjugation methods. The resulting molecule has one protein capture moiety (antibody) and one nucleic acid moiety (oligonucleotide) (Figure 20).

The antibody batches after immunization of an animal with a reduced complexity protein pool are conjugated with the one oligonucleotide sequence. Antibodies produced from multiple immunization events with different protein pools are conjugated to an oligonucleotide with a different sequence (Figure 20).

### 4. Capture of target proteins using reactivity functionality and sorting by oligonucleotide hybridization

Two different methods have been developed for making oligonucleotides bound to a solid support: they can be synthesised in situ, or presynthesised and attached to the support. In either case, it is possible to use the support-bound oligonucleotides in a hybridisation reaction with oligonucleotides in the liquid phase to form duplexes; the excess of oligonucleotide in solution can then be washed away.

The support may take the form of particles, for example, glass spheres, or magnetic beads. In this case the reactions could be carried out in tubes, or in the wells of a microtitre plate. Methods for both synthesising oligonucleotides and for attaching presynthesised oligonucleotides to these materials are known (see, *e.g.,* Stahl et al. (1988) Nucleic Acids Research 16(7):3025-3039).

### a. Preparation of amine-functionalized solid support

Oligonucleotides of a defined sequence is synthesized on amine-functionalized a glass support. An amine function was attached discrete locations on the glass slide using solution of 700µl of H₂N(CH₂)₃ Si(OCH₂CH₃)₃ in 10 ml of 95% ethanol at room temperature for 3 hours. The treated support is washed once with methanol and then once with ethyl ether. The support was dried at room temperature and then baked at 110 °C for 15 hours. It was then washed with water, methanol and water, and then dried.

The glass slide was reacted for 30 minutes at room temperature with 250 mg (1 millimole) of phthallic anhydride in the presence of 2 ml of anhydrous pyridine and 61 mg of 4-dimethyl amino pyridine.

The product was rinsed with methylene dichloride, ethyl alcohol and ether, and then dried. The products on the slide were reacted with 330 mg of dicyclohexylcarbodiimide (DCC) for 30 minutes at room temperature. The solution was decanted and replaced with a solution of 117 mg of 6-amino-1-hexanol in 2 ml of methylene dichloride and then left at room temperature for approximately 8 hours.

### b. Oligonucleotide synthesis on a solid support

The amine-functionalized solid support was prepared for oligonucleotide synthesis by treatment with 400 mg of succinic anhydride and 244 mg of 4-dimethyl aminopyride in 3 ml of anhydrous pyridine for 18 hours at room temperature. The solid support treated with 2 ml of DMF containing 3 millimoles (330 mg) of DCC and 3 millimoles (420 mg) of p-nitrophenol at room temperature overnight. The slide was washed with DMF, CH₃CN, CH₂Cl₂ and ethyl ether. A solution of 2 millimoles (234 mg) of H₂N(CH₂)₆OH in 2 ml of DMF was reacted with slide overnight.
The product of this reaction was a support,
-O(CH₂)₃NHCO(CH₂)₂CONH(CH₂)₆CH₂OH. The slide was washed washed with DMF, CH₃CN, methanol and ethyl ether.

The functionalized ester resulting from the preparation of the glass support was used for the synthesis of a oligonucleotide sequence. Each nucleoside residue was added as a phosphoramidite according to the procedure of Caruthers et al. (U.S. Patent No. 4,415,732).

### 5. Protein analysis of the captured proteins and complex protein sample comparison

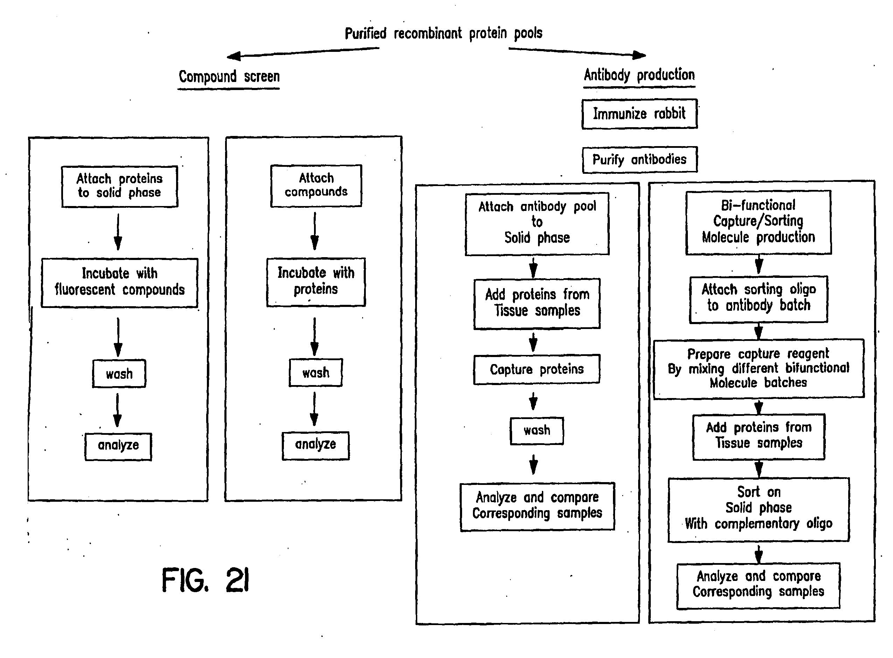

The purified antibody batches can be either 1) directly attached to a solid surface, and incubated with protein samples, 2) incubated with the samples and subsequently bound to a solid support without using the bi-functional capture molecule, 3) the bi-functional capture molecule can be used to capture its corresponding protein in a sample and subsequently sort the captured proteins through specific nucleotide hybridization (Figure 21).

### IV. Antisense oliogonucleotide capture reagents are immobilized in discrete and known locations on a solid surface to create an antibody capture array

### 6. Preparation of capture array surface

5'-aminated oligonucleotides are synthesized using phosphoramidate chemistry and attached to N-oxysussinimide esters. The attached oligonucleotide sequences are complementary to the sorting oligonucleotides of the bi-functional antibody molecules (Figure 20). Proteins are captured through nucleic acid hybridization of their sorting oligonucleotide to the complementary sequence attached to the solid surface oligonucleotide.

### V. The antibody capture reagents are added to the total protein mixture (reactivity step). The reaction mixture is then added to the solid surface array under conditions which allow oligonucleotide hybridization (sorting step).

### 7. Bi-functional reagent/protein capture and sorting

The bi-functional antibodies are being incubated with the protein sample under conditions that allow the antibodies to bind to their corresponding antigen. The bi-functional antibody molecule with the captured protein is added to the oligonucleotide prepared capture array. Under standard DNA annealing conditions which do not denature the antigen-antibody binding the bi-functional antibody will hybridize with its nucleic acid moiety to the complementary oligonucleotide.

### VI. The capture protein is identified using MALDI mass spectrometry

### 8. Analysis of the capture proteins

The attached proteins will be analyzed using standard protein analysis methods like Mass Spectrometry.

Since modifications will be apparent to those of skill in this art, it is intended that this invention be limited only by the scope of the appended claims.

## Claims

1. A gradient array for capturing biomolecules, comprising:
a 2-dimensional array of capture compounds that comprise moieties X and Y ; and
a surface containing loci for presenting the capture compounds that contain moieties X and Y, wherein:
the surface comprises a solid support with the compounds thereon on loci arranged in a two-dimensional array;
the surface comprises at least 10 different capture compounds, where different compounds are located at each locus;
the X moieties are each independently selected to bind covalently to amino acid side chains of proteins;
each moiety Y modulates one or more of the affinity, steric properties and electronic properties of X thereby increasing selectivity of the binding by X such that X binds to fewer proteins when the selectivity moiety Y is present than in its absence;
along each row of loci (X-axis) in the array a selected property of X at each locus selected from among hydrophobicity, lipophilicity, charge, size or specificity of the capture compound at each locus is altered in a predetermined manner along the row to provide a gradient of the property;
along each column of loci (Y-axis) in the array, a property of Y at each locus selected from among hydrophobicity, charge, size, specificity is altered in a predetermined manner along the Y-axis to provide a gradient of the property;
for each array, the property on the X-axis that is altered is not the same as the property on the Y-axis that is altered, whereby the array thereby presents a two dimensional gradient of two or more of the properties and presents loci with different affinities for biomolecules.

2. The gradient array of claim 1, wherein the compounds along the X-axis comprise an azobenzene group and a gradient of hydrophilicity is created by increased light exposure.

3. The gradient array of claim 1, wherein the compounds along Y-axis comprise an azobenzene group and a gradient of hydrophilicity is created by increased light exposure.

4. The gradient array of claim 1, wherein the compounds along the X-axis comprise a charged group and a gradient of charge is created by increased exposure to current.

5. The gradient array of claim 1, wherein the compounds along the Y-axis comprise a charged group and a gradient of charge is created by increased exposure to current.

6. The gradient array of claim 1, wherein the property of the compounds that is varied along the X-axis is specificity for a NH₂, SH, SS or OH group.

7. The gradient array of claim 1, wherein the property of the compounds that is varied along the Y-axis is specificity for NH₂, SH; SS or OH group.

8. The gradient array of claim 1, wherein an X is an α-halo ether, an α-halo carbonyl group, maleimido, a metal complex, an expoxide, an isothiocyanate, or an antibody against phosphorylated or glycosylated peptides/proteins.

9. The gradient array of claim 1, wherein X is -C(=O)O-Ph-pNO₂, -C(=O)O-C₆F₅, -C(=O)-O-(N-succinimidyl), -OCH₂-I, -OCH₂-Br, -OCH₂-Cl, -C(O)CH₂I, -C(O)CH₂Br or -C(O)CH₂Cl.

10. The gradient array of claim 1, wherein loci or X or Y moieties at each loci comprise a mass modifying tag.

11. A method of analyzing biomolecules comprising,
a) contacting a composition comprising a biomolecule with an array of any of claims 1-10 to form biomolecule complexes with X and/or Y moieties at loci on the array ; and
b) identifying or detecting bound biomolecules.

## Patentansprüche

1. Gradientenanordnung zum Einfangen von Biomolekülen, umfassend:
eine zweidimensionale Anordnung von Fängerverbindungen, welche die Reste X und Y umfassen;
eine Oberfläche, die Positionen zur Präsentation der Fängerverbindungen enthält, welche die Reste X und Y enthalten, worin:
die Oberfläche einen festen Träger mit den Verbindungen darauf an Positionen umfasst, die in einer zweidimensionalen Anordnung angeordnet sind;
die Oberfläche mindestens 10 unterschiedliche Fängerverbindungen umfasst, wobei an jeder Position unterschiedliche Verbindungen positioniert sind;
die X-Reste jeweils unabhängig ausgewählt sind, um kovalent an Aminosäureseitenketten der Proteine zu binden;
jeder Rest Y eine oder mehrere der Eigenschaften Affinität, sterische Eigenschaften und elektronische Eigenschaften von X moduliert, wodurch die Selektivität der Bindung durch X erhöht ist, so dass X, wenn der Selektivitätsrest Y anwesend ist, an weniger Proteine bindet, als bei seiner Abwesenheit;
entlang einer jeden Reihe von Positionen (X-Achse) in der Anordnung eine ausgewählte Eigenschaft von X an jeder Position, ausgewählt unter anderem aus Hydrophobizität, Lipophilizität, Ladung, Größe oder Spezifität der Fängerverbindung an jeder Position, in einer vorbestimmten Art und Weise entlang der Reihe verändert ist, um einen Gradient der Eigenschaft bereitzustellen;
entlang einer jeden Spalte von Positionen (Y-Achse) in der Anordnung eine Eigenschaft von Y an jeder Position, ausgewählt unter anderem aus Hydrophobizität, Ladung, Größe und Spezifität, in einer vorbestimmten Art und Weise entlang der Y-Achse verändert ist, um einen Gradient der Eigenschaft bereitzustellen;
für jede Anordnung die Eigenschaft auf der X-Achse, die verändert ist, nicht die gleiche ist wie die auf der Y-Achse veränderte Eigenschaft, wobei die Anordnung **dadurch** einen zweidimensionalen Gradienten von zwei oder mehreren der Eigenschaften präsentiert und Positionen mit unterschiedlichen Affinitäten für Biomoleküle präsentiert.

2. Gradientenanordnung nach Anspruch 1, worin die Verbindungen entlang der X-Achse eine Azobenzol-Gruppe umfassen und ein Hydrophobizitätsgradient durch eine erhöhte Lichtexposition kreiert ist.

3. Gradientenanordnung nach Anspruch 1, worin die Verbindungen entlang der Y-Achse eine Azobenzol-Gruppe umfassen und ein Hydrophobizitätsgradient durch eine erhöhte Lichtexposition kreiert ist.

4. Gradientenanordnung nach Anspruch 1, worin die Verbindungen entlang der X-Achse eine geladene Gruppe umfassen und ein Ladungsgradient durch eine erhöhte Stromexposition kreiert ist.

5. Gradientenanordnung nach Anspruch 1, worin die Verbindungen entlang der Y-Achse eine geladene Gruppe umfassen und ein Ladungsgradient durch eine erhöhte Stromexposition kreiert ist.

6. Gradientenanordnung nach Anspruch 1, worin es sich bei der Eigenschaft der Verbindungen, die entlang der X-Achse verändert ist, um eine Spezifität für eine NH₂-, SH-, SS- oder OH-Gruppe handelt.

7. Gradientenanordnung nach Anspruch 1, worin es sich bei der Eigenschaft der Verbindungen, die entlang der Y-Achse verändert ist, um eine Spezifität für eine NH₂-, SH-, SS- oder OH-Gruppe handelt.

8. Gradientenanordnung nach Anspruch 1, worin ein X ein α-Haloether, eine α-Halo-Carbonylgruppe, ein Maleimid, ein Metallkomplex, ein Epoxid, ein Isothiocyanat oder ein Antikörper gegen phosphorylierte oder glykosylierte Peptide/Proteine ist.

9. Gradientenanordnung nach Anspruch 1, worin X -C(=O)O-Ph-pNO₂, -C(=O)O-C₆F₅, -C(=O)-O-(N-Succinimidyl), -OCH₂-I, -OCH₂-Br, -OCH₂-Cl, -C(O)CH₂l, -C(O)CH₂Br oder -C(O)CH₂Cl ist.

10. Gradientenanordnung nach Anspruch 1, worin Positionen oder X- oder Y-Reste an jeder der Positionen eine Massen-modifizierende Markierung umfassen.

11. Verfahren zur Analyse von Biomolekülen, umfassend
a) In-Kontakt-bringen einer Zusammensetzung umfassend ein Biomolekül mit einer Anordnung nach einem der Ansprüche 1-10, um Biomolekülkomplexe mit X- und/oder Y-Resten an den Positionen auf der Anordnung zu bilden; und
b) Identifizieren oder Detektieren der gebundenen Biomoleküle.

## Revendications

1. Réseau à gradient pour la capture de biomolécules, comprenant :
un réseau bidimensionnel de composés de capture comprenant des groupements X et Y ; et
une surface contenant des locus pour la présentation des composés de capture qui contiennent des groupements X et Y, où :
la surface comprend un support solide avec les composés sur ce support sur des locus disposés en un réseau bidimensionnel ;
la surface comprend au moins 10 composés de capture différents, des composés différents étant situés à chaque locus ;
les groupements X sont choisis chacun indépendamment pour la liaison covalente aux chaînes latérales d'aminoacides des protéines ;
chaque groupement Y module une ou plusieurs des propriétés consistant en l'affinité, des propriétés stériques et des propriétés électroniques de X, en augmentant ainsi la sélectivité de la liaison par X de telle sorte que X se lie à un nombre réduit de protéines lorsque le groupement de sélectivité Y est présent, par rapport à son absence ;
le long de chaque rangée de locus (axe des X) dans le réseau, une propriété choisie de X à chaque locus choisie entre le pouvoir hydrophobe, le pouvoir lipophile, la charge, les dimensions ou la spécificité du composé de capture à chaque locus, est modifiée d'une manière prédéterminée le long de la rangée pour fournir un gradient de la propriété ; le long de chaque colonne de locus (axe des Y) dans le réseau, une propriété de Y à chaque locus choisi entre le pouvoir hydrophobicité, la charge ou les dimensions, et la spécificité, est modifiée d'une manière prédéterminée le long de l'axe des Y pour fournir un gradient de la propriété ;
pour chaque réseau, la propriété sur l'axe des X qui est modifiée n'est pas identique à la propriété sur l'axe des Y qui est modifiée, ce qui fait que le réseau présente ainsi un gradient bidimensionnel de deux ou plus de deux des propriétés et présente des locus ayant des affinités différentes pour des biomolécules.

2. Réseau à gradient suivant la revendication 1, dans lequel les composés le long de l'axe des X comprennent un groupe azobenzène et un gradient de pouvoir hydrophile est engendré par une exposition accrue à la lumière.

3. Réseau à gradient suivant la revendication 1, dans lequel les composés le long de l'axe des Y comprennent un group azobenzène et un gradient de pouvoir hydrophile est engendré par une exposition accrue à la lumière.

4. Réseau à gradient suivant la revendication 1, dans lequel les composés le long de l'axe des X comprennent un groupe chargé et un gradient de charge est engendré par une exposition accrue à un courant.

5. Réseau à gradient suivant la revendication 1, dans lequel les composés le long de l'axe des Y comprennent un groupe chargé et un gradient de charge est engendré par une exposition accrue à un courant.

6. Réseau à gradient suivant la revendication 1, dans lequel la propriété des composés qui est modifiée le long de l'axe des X est la spécificité pour un groupe NH₂, SH, SS ou OH.

7. Réseau à gradient suivant la revendication 1, dans lequel la propriété des composés qui est modifiée le long de l'axe des Y est la spécificité pour un groupe NH₂, SH, SS ou OH.

8. Réseau à gradient suivant la revendication 1, dans lequel un groupe X est un groupe a-halogéno-éther, un groupe α-halogénocarbonyle, un groupe maléimido, un complexe métallique, un époxyde, un isothiocyanate, ou un anticorps contre des peptides/protéines phosphorylées ou glycosylées.

9. Réseau à gradient suivant la revendication 1, dans lequel X représente un groupe -C (=O) O-Ph-ₚNO₂, -C(=O)O-C₆F₅, -C (=O) -O- (N-succinimidyle) , -OCH₂-I, -OCH₂-Br, -OCH₂-Cl, -C (O) CH₂I, -C(O)CH₂Br ou -C (O) CH₂Cl.

10. Réseau à gradient suivant la revendication 1, dans lequel les locus ou les groupements X ou Y à chaque locus comprennent un marqueur modificateur de masse.

11. Méthode pour analyser des biomolécules, comprenant
a) la mise en contact d'une composition comprenant une biomolécule avec un réseau de l'une quelconque des revendications 1 à 10 pour former des complexes de biomolécules avec les groupements X et/ou Y à des locus sur le réseau ; et
b) à identifier ou détecter les biomolécules liées.
